# EUROPEAN PATENT SPECIFICATION

(11) **EP 1 999 143 B1**
(45) Date of publication and mention of the grant of the patent: **13.07.2011**
(21) Application number: 07753276.0
(22) Date of filing: 16.03.2007
(51) Int. Cl.: C07K 14/43, C07K 14/47

(54) **NEUROMEDIN U RECEPTOR AGONISTS AND USES THEREOF**
NEUROMEDIN-U-REZEPTORAGONISTEN UND IHRE VERWENDUNG
AGONISTES DU RÉCEPTEUR DE NEUROMÉDINE U ET LEURS UTILISATIONS

(30) Priority: 20.03.2006 US 783933 P
(43) Date of publication of application: 10.12.2008
(73) Proprietor: Merck Sharp & Dohme Corp., Rahway, NJ 07065 (US); Istituto di Ricerche di Biologia Molecolare P. Angeletti, 00040 Pomezia (IT)
(72) Inventor: MARSH, Donald J., Rahway, New Jersey 07065-0907 (US); PESSI, Antonello, I-00040 Pomezia (IT); BEDNAREK, Maria A., Rahway, New Jersey 07065-0907 (US); BIANCHI, Elisabetta, I-00040 Pomezia (IT); INGALLINELLA, Paolo, I-00040 Pomezia (IT); PEIER, Andrea M., Rahway, New Jersey 07065-0907 (US)
(74) Representative: Buchan, Gavin MacNicol
(86) International application number: PCT/US2007/006635
(87) International publication number: WO 2007/109135

(56) References cited:
- EP-A1- 1 237 001
- BRIGHTON P J ET AL: "Neuromedin U and Its Receptors: Structure, Function, and Physiological Roles" PHARMACOLOGICAL REVIEWS, WILLIAMS AND WILKINS INC., BALTIMORE, MD,, US, vol. 56, no. 2, 2004, pages 231-248, XP003002000 ISSN: 0031-6997
- CLARK R ET AL: "Long-acting growth hormones produced by conjugation with polyethylene glycol" JOURNAL OF BIOLOGICAL CHEMISTRY, AMERICAN SOCIETY OF BIOLOCHEMICAL BIOLOGISTS, BIRMINGHAM,, US, vol. 271, no. 36, 6 September 1996 (1996-09-06), pages 21969-21977, XP002216386 ISSN: 0021-9258
- DOGGRELL SHEILA A: "Neuromedin U. A new target in obesity" EXPERT OPINION ON THERAPEUTIC TARGETS, ASHLEY PUBLICATIONS, LONDON, GB, vol. 9, no. 4, August 2005 (2005-08), pages 875-877, XP009088829 ISSN: 1472-8222
- MALENDOWICZ L K ET AL: "Effects of neuromedin U (NMU)-8 on the rat hypothalamo-pituitary-adr enal axis: Evidence of a direct effect of NMU-8 on the adrenal gland" NEUROPEPTIDES, vol. 26, no. 1, 1994, pages 47-53, XP002455631 ISSN: 0143-4179
- HANADA REIKO ET AL: "Neuromedin U has a novel anorexigenic effect independent of the leptin signaling pathway" NATURE MEDICINE, NATURE PUBLISHING GROUP, NEW YORK, NY, US, vol. 10, no. 10, October 2004 (2004-10), pages 1067-1073, XP009088830 ISSN: 1078-8956
- NANDHA K A ET AL: "NEUROMEDIN U - AN OVERVIEW" BIOMEDICAL RESEARCH, BIOMEDICAL RESEARCH PRESS INC., SAPPORO, JP, vol. 14, no. SUPPL 3, 1993, pages 71-76, XP001052975 ISSN: 0388-6107
- PARDRIDGE W M ET AL: "COMBINED USE OF CARBOXYL-DIRECTED PROTEIN PEGYLATION AND VECTOR-MEDIATED BLOOD-BRAIN BARRIER DRUG DELIVERY SYSTEM OPTIMIZES BRAIN UPTAKE OF BRAIN-DERIVED NEUROTROPHIC FACTOR FOLLOWING INTRAVENOUS ADMINISTRATION" PHARMACEUTICAL RESEARCH, NEW YORK, NY, US, vol. 15, no. 4, April 1998 (1998-04), pages 576-582, XP001107216 ISSN: 0724-8741
- DEGUCHI Y ET AL: "RETENTION OF BIOLOGIC ACTIVITY OF HUMAN EPIDERMAL GROWTH FACTOR FOLLOWING CONJUGATION TO A BLOOD-BRAIN BARRIER DRUG DELIVERY VECTOR VIA AN EXTENDED POLY(ETHYLENE GLYCOL) LINKER" BIOCONJUGATE CHEMISTRY, ACS, WASHINGTON, DC, US, vol. 10, no. 1, January 1990 (1990-01), pages 32-37, XP001164141 ISSN: 1043-1802
- TAMAI IKUMI ET AL: "Structure-internalization relationship for adsorptive-mediated endocytosis of basic peptides at the blood-brain barrier" JOURNAL OF PHARMACOLOGY AND EXPERIMENTAL THERAPEUTICS, vol. 280, no. 1, 1997, pages 410-415, XP002455632 ISSN: 0022-3565

## Description

### BACKGROUND OF THE INVENTION

### (1) Field of the Invention

The present invention relates to neuromedin U receptor agonists for use in the treatment of metabolic disorders such as obesity.

### (2) Description of Related Art

Neuromedin U (NMU) was originally isolated from porcine spinal cord based upon its ability to contract rat uterine smooth muscle and has since been implicated in a variety of other physiological processes, including strews, nociception, inflammation, cardiovascular function and energy homeostasis. Characterization of NMU has identified three peptides with similar bioactivity, full length NMU, (a 25-mer (NMU-25)) in humans, pigs, and dogs, a 23-mer (NMU-23) in rats and mice, and an 8-mer (NMU-8). NMU-8 is derived from cleavage of full-length NMU and shares an identical C-terminus with the full-length precursor. NMU-8 is highly conserved among vertebrates, containing seven C-terminal residues that are identical across all species that have been examined; these residues are critical for bioactivity(Brighton et al., Pharmacol. Rev. 56: 231-248 (2004)).

NMU's role in the regulation of energy homeostasis is supported by both pharmacologic and genetic data. Properties of NMU include inhibition of food intake and increase in energy expenditure seen when the substance is administered centrally (Howard et al., Nature 406: 70-74 (2000); Nakazato et al., Biochem. Biophys. Res. Comm. 277:191-194(2000); Ivanov et al., Endocrinol. 143: 3813-3821 (2002); and Wren et al., Endocrinol., 143: 4227-4234 (2002)). NMU-deficient mice develop obesity characterized by hyperphagia and reduced energy expenditure (Hanada et al., Nat. Med., 10: 1067-1073 (2004)), and transgenic mice overexpressing NMU are lean and hypophagic (Kowalski et al., J. Endocrinol.186: 151-164 (2005)). The internal energy status of an animal affects expression and release of NMU as well (Wren *et al*., *ibid*.).

Two high affinity NMU receptors, NMUR1 (Intl. Patent Appl. No. PCT/US99/15941) and NMUR2 (U. S. Patent No. 7163799), have been identified. NMUR1 is predominantly expressed in the periphery, whereas NMUR2 is primarily expressed in the brain. Pharmacologic experiments have served to better define NMU's short- and long-term effects on energy homeostasis and to identify which NMU preceptors) are involved in mediating these actions. It has been shown that acute administrations of NMU either centrally or peripherally reduce food intake in mice in a dose-dependent fashion. The anorectic actions of centrally administered NMU are absent in NMUR2-deficient (*Nmur2*^{*-*/*-*}) mice but are present in NMUR1 - deficient *(Nmur1*^{*-*/*-*}) mice. In contrast, the anorectic actions of peripherally administered NMU are absent in *Nmur1*^{*-*/}*⁻* mice and present in *Nmur2*^{*-*/}*⁻* mice. Additionally, acute peripheral administration of NMU dose-dependently increases core body temperature in mice, suggesting that NMU1 may also modulate energy expenditure. Chronic administration of NMU either centrally or peripherally reduces food intake, body weight and adiposity in mice, again in a dose-dependent fashion. In *Nmur2*^{*-*/}*⁻* transgenic mice, body weight, body composition, body temperature and food intake are largely unaffected by chronic central administration of rat NMU-23. In *Nmur1*^{*-*/}*⁻* transgenic mice, body weight, body composition and food intake are largely unaffected by chronic peripheral administration of rat NMU-23.

Because the sites of action for NMU1- vs. NMUR2-mediated efficacy differ and appear to be independent of one another, but have a role in obesity, it is suggested that both NMUR1- and NMUR2-selective agonists and NMUR1/2 non-selective agonists may be useful for the treatment of obesity. Therefore, there is a need for neuromedin U receptor agonists useful in the treatment of metabolic disorders.

### BRIEF SUMMARY OF THE INVENTION

The present disclosure provides neuromedin U receptor agonists. In one aspect, the neuromedin U receptor agonists are specific for one receptor subtype, in another aspect, the neuromedin U receptor agonists are capable of binding and stimulating both the NMUR1 or NMUR2 receptor. In further aspect, the neuromedin U receptor agonists have been derivatized to enable the neuromedin U receptor agonists to cross the blood-brain barrier and interact with NMU receptors in the brain. The neuromedin U receptor agonists can be used therapeutically and as research tools.

Therapeutic applications of the neuromedin U receptor agonists include administering the neuromedin U receptor agonists to an individual to treat a metabolic disorder afflicting the individual. Such disorders include, but are not limited to, obesity, metabolic syndrome or syndrome X, and type II diabetes. Complications of diabetes such as retinopathy may be positively affected thereby as well. Obesity is a comorbidity of and may well contribute to such disease states as diabetes, hypertension, dyslipidemias, cardiovascular disease, gallstones, osteoarthritis and certain forms of cancers. Administration of one or more of the neuromedin U receptor agonists disclosed herein to effect weight loss in an individual may also be useful in preventing such diseases and as part of therapy for any one of the above-recited conditions, as well as others. In other embodiments, there is provided a method for treating a metabolic disease in an individual comprising administering to the individual one or more of the neuromedin U receptor agonist s described above. The metabolic disease may be selected from the group consisting of diabetes, metabolic syndrome, hyperglycemia, and obesity and may be administered via a route peripheral to the brain, such as an oral, mucosal, buccal, sublingual, nasal, rectal, subcutaneous, transdermal, intravenous, intramuscular, or intraperitoneal route. Finally, the neuromedin U receptor agonists can be administered to an individual to effect a reduction in food intake by the individual, to effect a reduction in weight gain in the individual, to prevent weight gain in the individual, to effect weight loss in the individual, and/or to prevent weight regain in the individual.

Accordingly, the present disclosure provides an isolated neuromedin U receptor agonist, which has the formula (I)

Z¹-peptide-Z²

wherein the peptide has the amino acid sequence X¹-X²-X³-X⁴-X⁵-X⁶-X⁷-X⁸-X⁹-X¹⁰-X¹¹-X¹²-X¹³-X¹⁴-X¹⁵-X¹⁶-X¹⁷-X¹⁸-X¹⁹-X²⁰-X²¹-X²²-X²³-X²⁴-X²⁵ (SEQ 3D NO:27), wherein amino acids 1 to 17 can be any amino acid or absent; wherein amino acid XIS is absent, Y, W, F, a des-amino acid or an acyl group; amino acid X¹⁹ is A, W, Y, F or an aliphatic amino acid; amino acid X²⁰ is absent, L, G, sarcosine (Sar), D-Leu, NMe-Leu, D-Ala or A; amino acid X²¹ is F, NMe-Phe, an aliphatic amino acid, an aromatic amino acid, A or W; X²² is R, K, A or L; amino acid X²³ is P, Sar, A or L; amino acid X²⁴ is R, Harg or K; and amino acid X²⁵ is N, any D- or L-amino acid, Nle or D-Nle, or A.; and Z¹ is an optionally present protecting group that, if present, is joined to the N-terminal amino group; and Z² is NH₂ or an optionally present protecting group that, if present, is joined to the C-terminal carboxy group, and pharmaceutically acceptable salts thereof.

In particular aspects of the neuromedin U receptor agonist, the peptide has the amino acid sequence X¹-X²-X³-X⁴-X⁵-X⁶-X⁷-X⁸-X⁹-X¹⁰-X¹¹-X¹²-X¹³-X¹⁴-X¹⁵-X¹⁶-X¹⁷-X¹⁸-F-LF-R-P-R-N (SEQ ID NO:1), wherein amino acids 1 to 17 can be any amino acid or absent, which in particular aspects has an amino acid sequence selected from the group consisting of SEQ ID NO:2, SEQ ID NO:3, SEQ ID NO:4, SEQ ID NO:5, and SEQ NO:6. In currently preferred aspects, the peptide has an amino acid sequence shown in SEQ ID NO:2.

In another aspect of the neuromedin U receptor agonist, the peptide comprises the amino acid sequence F-R-V-D-E-E-F-Q-S-P-F-A-S-Q-S-R-G-X¹⁸-X¹⁹-X²⁰-X²¹-X²²-X²³-X²⁴-X²⁵ (SEQ ID NO:7) wherein amino acid X¹⁸ is absent, Y, W, F, a des-amino acid or an acyl group; amino acid X¹⁹ is A, W, Y, F or an aliphatic amino acid; amino acid X²⁰ is absent, G, sarcosine (Sar), D-Leu, NMe-Leu, D-Ala or A; amino acid X²¹ is NMe, an aliphatic amino acid, an aromatic amino acid, A or W; amino acid X²² is K, A or L; amino acid X²³ is Sar, A or L; amino acid X²⁴ is Harg or K; and amino acid X²⁵ is any D- or L-amino acid, NIe or D-Nle, or A, which in particular aspects has the amino acid sequence selected from the group consisting of SEQ ID NO:14, SEQ ID NO:15, SEQ ID NO:22, SEQ ID NO:23, SEQ NO:24, and SEQ ID NO:25.

In a further still aspect of the neuromedin U receptor agonist, the peptide comprises the amino acid sequence X1-X2-X3-X4-X5-X6-X7-X8 (SEQ ID NO:8) wherein amino acid X¹ is absent, Y, W, F, a des-amino acid or an acyl group; amino acid X² is A, W, Y, F or an aliphatic amino acid; amino acid X³ is absent, G, sarcosine(Sar), D-Leu, NMe-Leu, D-Ala or A; amino acid X⁴ is NMe-Phe, an aliphatic amino acid, an aromatic amino acid, A or W; amino acid X⁵ is K, A or L; amino acid X⁶ is Sar, A or L; amino acid X7 is Harg or K; and amino acid X⁸ is any D- or L-amino acid, Nle or D-Nle, or A, which in particular aspects has the amino acid sequence selected from the group consisting of SEQ ID NO:9, SEQ ID NO:10, SEQ ID NO:11, SEQ ID NO:12, SEQ ID NO:13, SEQ ID NO:16, SEQ ID NO:17, SEQ ID NO:18, SEQ ID NO:19, SEQ ID NO:20, and SEQ ID NO:21.

In further aspects of the above neuromedin U receptor agonists, the N-tenninal amino acid is covalently joined to one or more molecules selected from the group consisting of PEG, cholesterol, N-ethylmaleimidyl, and palmitoyl. In further still aspects of the neuromedin U receptor agonist, the peptide further includes a cysteine residue at the N-terminus of the peptide to which is optionally present a protecting group that, if present, is joined to the N-terminal amino group of the cysteine residue. In particular aspects of the neuromedin U receptor agonist, the thiol group of the cysteine residue at the N-terminus is covalently joined to one or more molecules selected from the group consisting of PEG, cholesterol, N-ethylmaleimidyl, and palmitoyl. In a currently preferred embodiment, the neuromedin U receptor agonists has the amino acid of SEQ ID NO:2, which further includes a cysteine residue at the N-terminus of the peptide to which is present a protecting group joined to the N-terminal amino group of the cysteine residue and a PEG molecule joined to the thiol group.

The neuromedin U receptor agonist can further include a linker group having a distal end and a proximal end. The linker group is covalently joined at its distal end to the N-terminus of the peptide, and is covalently linked at the proximal end to the carboxyl terminus of a cysteine residue, onto which is optionally present a protecting group that, if present, is joined to the N-terminal amino group of the cysteine residue. In particular aspects, wherein the thiol group of the cysteine residue is covalently joined to one or more molecules selected from the group consisting of PEG, cholesterol, N-ethylmaleimidyl, and palmitoyl.

The present disclosure further provides for the use of any one or more of the embodiments and aspects of the neuromedin U receptor agonist in the manufacture of a medicament for treatment of a metabolic disorder. Disorders include, but are not limited to, obesity, metabolic syndrome or syndrome X, and type II diabetes. Complications of diabetes such as retinopathy may be positively affected thereby as well. Obesity is a comorbidity of and may well contribute to such disease states as diabetes, hypertension, dyslipidemias, cardiovascular disease, gallstones, osteoarthritis and certain forms of cancers. Thus, the present, disclosure provides a pharmaceutical composition comprising one or more of any of the above neuromedin U receptor agonists and a pharmaceutically acceptable carrier.

The present disclosure further provides a method for producing a neuromedin U receptor agonist that can cross the blood-brain barrier comprising covalently joining to the peptide one or more PEG molecules wherein the one or more PEG molecules render the peptide capable of crossing the blood-brain barrier. Therefore, in particular aspects, the neuromedin U receptor agonist has the formula (I)

Z¹-peptide-Z²

wherein the peptide has the amino acid sequence X¹-X²-X³-X⁴-X⁵-X⁶-X⁷-X⁸-X⁹-X¹⁰-X¹¹-X¹²-X¹³-X¹⁴-X¹⁵-X¹⁶-X¹⁷-X¹⁸-X¹⁹-X²⁰-X²¹-X²²-X²³-X²⁴-X²⁵ (SEQ ID NO:27), wherein amino acids 1 to 17 can be any amino acid or absent; wherein amino acid X¹⁸ is absent, Y, W, F, a des-amino acid or an acyl group; amino acid X¹⁹ is A, W, Y, F or an aliphatic amino acid; amino acid X²⁰ is absent, L, G, sarcosine (Sar), D-Leu, NMe-Leu, D-Ala or A; amino acid X²¹ is F, NMe-Phe, an aliphatic amino acid, an aromatic amino acid, A or W; X²² is R, K, A or L; amino acid X²³ is P, Sar, A or L; amino acid X²⁴ is R, Harg or K; and amino acid X²⁵ is N, any D- or L-amino acid, Nle or D-Nle, A.; and Z¹ is an optionally present protecting group that, if present, is joined to the N-terminal amino group; and Z² is NH₂ or an optionally present protecting group that, if present, is joined to the C-terminal carboxy group, and pharmaceutically acceptable salts thereof.

Further provided is a method for producing a neuromedin U receptor agonist comprising all or a portion of the NMU-25 peptide that is specific for a neuromedin U receptor subtype comprising modifying one or more of the seven amino acids at the C-terminus of the peptide to an amino acid or amino acid analog that is not native to the human NMU-25 peptide.

Therefore, in one aspect, the peptide comprises the amino acid sequence F-R-V-D-E-E-F-Q-S-P-F-A-S-Q-S-R-G-X¹⁸-X¹⁹-X²⁰-X²¹-X²²-X²³-X²⁴-X²⁵(SEQ ID) NO:7) wherein amino acid X¹⁸ is absent, Y, W, F, a des-amino acid or an acyl group; amino acid X¹⁹ is A, W, Y, F or an aliphatic amino acid; amino acid X²⁰ is absent, G, sarcosine (Sar), D-Leu, NMe-Leu, D-Ala or A; amino acid X²¹ is NMe-Phe, an aliphatic amino acid, an aromatic amino acid, A or W; amino acid X²² is K, A or L; amino acid X²³ is Sar, A or L; amino acid X²⁴ is Harg or K; and amino acid X²⁵ is any D- or L-amino acid, Nle or D-Nle, or A, which in particular aspects has the amino acid sequence selected from the group consisting of SEQ ID NO:14, SEQ ID NO:15, SEQ ID NO:22, SEQ ID NO:23, SEQ ID NO:24, and SEQ ID NO:25.

In another aspect of the neuromedin U receptor agonist, the peptide comprises the amino acid sequence X¹-X²-X³-X⁴-X⁴-X⁶-X⁷-X⁸ (SEQ ID NO:8) wherein amino acid X¹ is absent, Y, W, F, a des-amino acid or an acyl group; amino acid X² is A, W, Y, F or an aliphatic amino acid; amino acid X³ is absent, G, sarcosine (Sar), D-Leu, NMe-Leu, D-Ala or A; amino acid X⁴ is NMe-Phe, an aliphatic amino acid, an aromatic amino acid, A or W; amino acid X⁵ is K, A or L; amino acid X⁶ is Sar, A or L; amino acid X⁷ is Harg or K; and amino acid X⁸ is any D- or L-amino acid, Nle or D-Nle, or A, which in particular aspects has the amino acid sequence selected from the group consisting of SEQ ID NO:9, SEQ ID NO: 10, SEQ ID NO:11, SEQ ID NO:12, SEQ ID NO:13, SEQ ID NO:16, SEQ ID NO:17, SEQ ID NO:18, SEQ ID NO:19, SEQ ID NO:20, and SEQ ID NO:21.

Further provided is a method for treating a metabolic disorder in an individual comprising administering to the individual a therapeutically effective amount of a neuromedin U receptor agonist that has the formula (I)

Z¹-peptide-Z²

wherein the peptide has the amino acid sequence X¹-X²-X³-X⁴-X⁵-X⁶-X⁷-X⁸-X⁹-X¹⁰-X¹¹X¹²-X¹³-X¹⁴-X¹⁵-X¹⁶-X¹⁷-X¹⁸-X¹⁹-X²⁰-X²¹-X²²-X²³-X²⁴-X²⁵ (SEQ ID NO:27), wherein amino acids 1 to 17 can be any amino acid or absent; wherein amino acid X¹⁸ is absent, Y, W, F, a des-amino acid or an acyl group; amino acid X¹⁹ is A, W, Y, F or an aliphatic amino acid; amino acid X²⁰ is absent, L, G, sarcosine (Sar), D-Leu, NMe-Leu, D-Ala or A; amino acid X²¹ is F, NMe-Phe, an aliphatic amino acid, an aromatic amino acid, A or W; X²² is R, K, A or L; amino acid X²³ is P, Sar, A or L; amino acid X²⁴ is R, Harg or K; and amino acid X²⁵ is N, any D- or L-amino acid, Nle or D-Nle, A.; and Z¹ is an optionally present protecting group that, if present, is joined to the N-terminal amino group; and Z² is NH₂ or an optionally present protecting group that, if present, is joined to the C-terminal carboxy group, to treat the metabolic disorder.

The method is particularly useful for treating a metabolic disorder selected from the group consisting of obesity, metabolic syndrome or syndrome X, type II diabetes, complications of diabetes, hypertension, dyslipidemias, cardiovascular disease, gallstones, osteoarthritis, and certain forms of cancers.

In particular aspects of the method, the peptide has the amino acid sequence X¹-X²-X³-X⁴-X⁵-X⁶-X⁷-X⁸-X⁹-X¹⁰-X¹¹-X¹²-X¹³-X¹⁴-X¹⁵-X¹⁶-X¹⁷-X¹⁸-F-L-R-P-R-N (SEQ ID NO:1), wherein amino acids 1 to 17 can be any amino acid or absent, which in particular aspects has an amino acid sequence selected from the group consisting of SEQ ID NO:2, SEQ II) NO:3, SEQ ID NO:4, SEQ ID NO:5, and SEQ ID NO:6. In currently preferred aspects, the peptide has an amino acid sequence shown in SEQ ID NO:2.

In another aspect of the method, the peptide comprises the amino acid sequence F-R-V-D-E-E-F-Q-S-P-F-A-S-Q-S-R-G-X¹⁸-X¹⁹-X²⁰-X²¹-X²²⁻X²³-X²⁴-X²⁵ (SEQ ID NO:7) wherein amino acid X¹⁸ is absent, Y, W, F, a des-amino acid or an acyl group; amino acid X¹⁹ is A, W, Y, F or an aliphatic amino acid; amino acid X²⁰ is absent, G, sarcosine (Sar), D-Leu, NMe-Leu, D-Ala or A; amino acid X²¹ is NMe-Phe, an aliphatic amino acid, an aromatic amino acid, A or W; amino acid X²² is K, A or L; amino acid X²³ is Sar, A or L; amino acid X³⁴ is Harg or K; and amino acid X²⁵ is any D- or L-amino acid, Nle or D-Nle, or A, which in particular aspects has the amino acid sequence selected from the group consisting of SEQ ID NO:14, SEQ ID NO:15, SEQ ID NO:22, SEQ ID NO:23, SEQ ID NO:24, and SEQ ID NO:25.

In a further still aspect of the method, the peptide comprises the amino acid sequence X¹-X²-X³-X⁴-X⁵-X⁶-X⁷-X⁸ (SEQ ID NO:8) wherein amino acid X¹ is absent, Y, W, F, a des-amino acid or an acyl group; amino acid X² is A, W, Y, F or an aliphatic amino acid; amino acid X³ is absent, G, sarcosine (Sar), D-Leu, NMe-Leu, D-Ala or A; amino acid X⁴ is NMe-Phe, an aliphatic amino acid, an aromatic amino acid, A or W; amino acid X⁵ is K, A or L; amino acid X⁶ is Sar, A or L; amino acid X⁷ is Harg or K; and amino acid X⁸ is any D- or L-amino acid, Nle or D-Nle, or A, which in particular aspects has the amino acid sequence selected from the group consisting of SEQ ID NO:9, SEQ ID NO:10, SEQ ID NO:11, SEQ ID NO:12, SEQ ID NO:13, SEQ ID NO:16, SEQ ID NO:17, SEQ ID NO:18, SEQ ID NO:19, SEQ ID NO:20, and SEQ ID NO:21.

In further aspects of the above method, the N-terminal amino acid is covalently joined to one or more molecules selected from the group consisting of PEG, cholesterol, N-ethylmaleimidyl, and palmitoyl. In further still aspects of the neuromedin U receptor agonist, the peptide further includes a cysteine residue at the N-terminus of the peptide to which is optionally present a protecting group that, if present, is joined to the N-terminal amino group of the cysteine residue. In particular aspects of the method, the thiol group of the cysteine residue at the N-terminus is covalently joined to one or more molecules selected from the group consisting of PEG, cholesterol, N-ethylmaleimidyl, and palmitoyl. In a currently preferred embodiment, the neuromedin U receptor agonists has the amino acid of SEQ ID NO:2, which further includes a cysteine residue at the N-terminus of the peptide to which is present a protecting group joined to the N-terminal amino group of the cysteine residue and a PEG molecule joined to the thiol group.

The neuromedin U receptor agonist can further include a linker group having a distal end and a proximal end is covalently joined at its distal end to the N-terminus of the peptide and the proximal end of the linker group is covalently linked to the carboxyl terminus of a cysteine residue to which is optionally present a protecting group that, if present, is joined to the N-terminal amino group of the cysteine residue. In particular aspects, wherein the thiol group of the cysteine residue is covalently joined to one or more molecules selected from the group consisting of PEG, cholesterol, N-ethylmaleimidyl, and palmitoyl. In a currently preferred embodiment of the method, neuromedin U receptor agonist has the formula Ac-C₂-peptide-CONH₂ wherein Ac is an acetyl group, C2 is Cys(PEG)₂40kDa and the peptide has the amino acid sequence shown in SEQ ID NO:2.

### BRIEF DESCRIPTION OF THE DRAWINGS

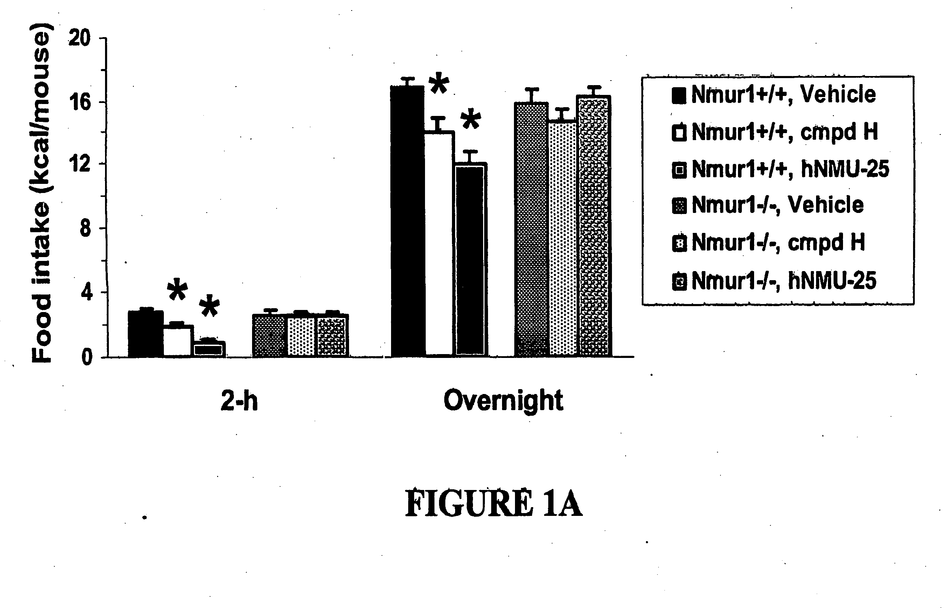

Figure 1A demonstrates that peripheral administration of agonist H reduces food intake and these anorectic actions are mediated by NMUR1. Diet-induced obese *Nmur1*+/+ and *Nmur1*-/- mice were dosed ip with either Vehicle (water), compound H at 3.25 mmoles/kg, or hNMU-25 at 3.25 mmoles/kg ~30 min. prior to the onset of the dark phase and food intake was measured about 2 and 18 hours (Overnight) later, respectively. *, P < 0.05 vs. Vehicle, n =11-12 per treatment group.

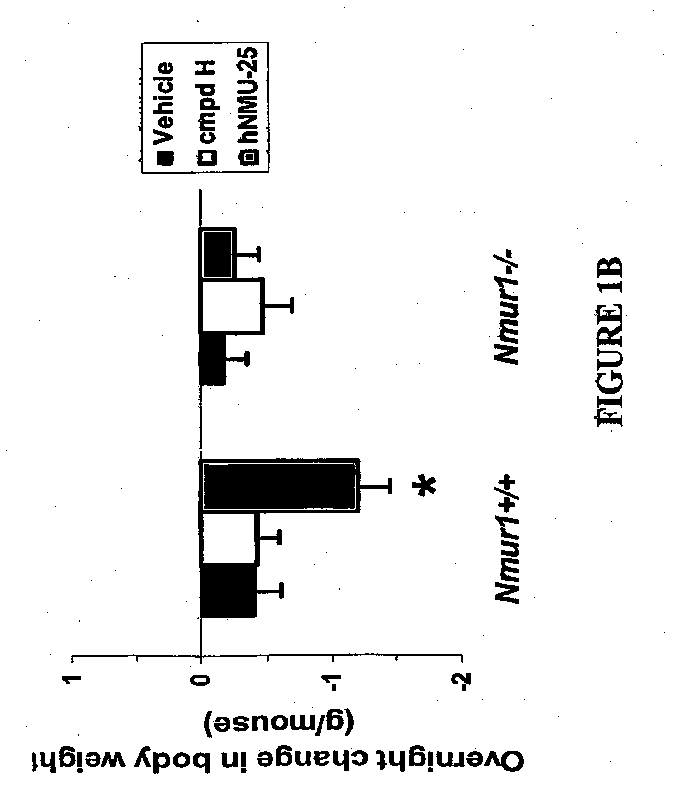

Figure 1B shows the overnight change in body weight of the mice in Figure 1A.
Figure 2A shows that acute peripheral administration of the NMUR1-selective agonist H significantly reduced food intake in wild-type mice, but not in *Nmur1* knockout mice, demonstrating that NMUR1 is required for the anorectic actions of agonist H. Additionally, these data demonstrate that NMUR1-selective agonism is sufficient to recapitulate the anorectic actions of the pan NMU1/2 agonist NMU.
Figure 2B shows that acute peripheral administration of the NMUR1-selective agonist NMU13 also significantly reduced food intake in wild-type mice, but not in Nmur1 knockout mice, demonstrating that NMU1 is required for the anorectic actions of this agonist as well. Additionally, these data demonstrate that NMUR1-selective agonism is sufficient to recapitulate the anorectic actions of the pan NMUR1/2 agonist NMU.
Figure 3A shows that acute subcutaneous administration of PEGylated NMU reduces food intake for three days post-dose. Consistent with the in vitro and in vivo metabolic profile of the PEGylated analogs, NMU1 exhibits greater efficacy at reducing overnight food intake when compared to hNMU-25 and reductions in food intake are observed for three days post-dose. Significant reductions in body weight were also observed (Figure 3B).
Figure 3B shows the change in body weight of the mice in Figure 3A.
Figure 4A shows that NMU12 is also an effective anorectic peptide. Similar to NMU1, a significant reduction in food intake and body weight (Figure 4B) was observed for three days after a single subcutaneous administration of the agonists.
Figure 4B shows the change in body weight in the mice of Figure 4A.
Figure 5A shows that the anorectic effects of NMU12 are mediated by the NMU1 and NMU2 receptors. Acute administration of NMU12 was highly efficacious in wild-type animals but no effect was observed in the NMUR1/NMUR2 double knockout animals.
Figure 5B shows the daily change in body weight of the mice in Figure 5A.
Figure 6A shows the anorectic effects of PEGylated NMU12 are mediated by both the NMUR1 and NMUR2 receptors. Reductions in food intake and body weight (Figure 6B) were observed for two days post-dose in the NMUR1 knockout animals. However, only overnight effects were observed in the NMUR2 knockout animals. This is in contrast to the anorectic effects of hNMU-25, which is mediated solely by NMU1, demonstrating that hNMU-25 and NMU12 have distinct mechanisms of action.
Figure 6B shows the change in body weight of the mice in Figure 6A over four days.
Figure 7A shows that chronic administration of NMU12 can reduce food intake and body weight. NMU12 was dosed every day (QD), every other day (Q2D) or every three days (Q3D). The Figure shows the cumulative change in body weight for nine days after the beginning of treatment. The last dose was administered on day four of the study and measurements were taken to day nine.
Figure 7B shows that the cumulative food intake of the mice in Figure 7A was significantly reduced in all dosing paradigms for NMU12. Food intake was reduced 12-27% at these doses relative to the vehicle treated group.
Figure 7C shows that the cumulative change in body weight of the mice in Figure 7A ranged from a loss of 3.3% to as much as a 7.3% relative to the vehicle control group.
Figure 8 shows a comparison of the *in vitro* stability of hNMU-25 and PEGylated agonist NMU1 and NMU12 in plasma with spike-in experiments. PEGylation provided greater stability in human plasma. The half-life of hNMU-25 in human plasma was less than 16 hours whereas the PEGylated agonists exhibited a half-life greater than 3 days in human plasma incubated at 37°C.
Figure 9 shows a comparison of the pharmacokinetic properties of hNMU-25 and PEGylated agonist NMU12 in mice. PEGylation provided greater metabolic stability in vivo. Animals were dosed subcutaneously with 10 mg/kg of hNMU-25 or NMU12. Plasma was collected at various time points post dose and measured in the Bioassay. The dashed line indicates the limits of detection for the assay (LOD).

### DETAILED DESCRIPTION OF THE INVENTION

The present disclosure provides neuromedin U receptor agonist. The neuromedin U receptor agonist described herein act at NMU receptors, bind the NMU receptors, and stimulate NMU receptor activity. In one aspect, the neuromedin U receptor agonists are specific for one receptor subtype, wherein such specificity is defined as having an IC₅₀ values that is less than about 200 nM in the corresponding NMU1 or NMUR2 receptor binding assay. Selectivity is based upon functional activity or EC₅₀ ratios at human NMuR2/human-NMUR1 for NMUR1-selective peptides and at human NNRM1/human NMUR2 for NMUR2-selective peptides. Further, the selective neuromedin U receptor agonists described herein range from about 21 to 909-fold selective for NMUR1 and from about 2 to 200-fold selective for NMUR2. In another aspect, the neuromedin U receptor agonists are capable of binding and stimulating both the NMUR1 and NMUR2 receptors and have been derivatized to enable the neuromedin U receptor agonists to cross the blood-brain barrier and interact with NMU receptors in the brain. The neuromedin U receptor agonists can be used therapeutically and as research tools.

One or more of the neuromedin U receptor agonists can be administered to an individual to treat a metabolic disorder afflicting the individual. Such disorders include, but are not limited to, obesity, metabolic syndrome or syndrome X, and type II diabetes. Complications of diabetes such as retinopathy may be positively affected thereby as well. Obesity is a comorbidity of and may well contribute to such disease states as diabetes, hypertension, dyslipidemias, cardiovascular disease, gallstones, osteoarthritis and certain forms of cancers. Administration of one or more of the neuromedin U receptor agonists disclosed herein to effect weight loss in an individual may also be useful in preventing such diseases and as part of therapy for any one of the above-recited conditions, as well as others. In other embodiments, there is provided a method for treating a metabolic disease in an individual comprising administering to the individual a one or more of the neuromedin U receptor agonists described above. The metabolic disease may be selected from the group consisting of diabetes, metabolic syndrome, hyperglycemia, and obesity and may be administered via a route peripheral to the brain, such as an oral, mucosal, buccal, sublingual, nasal, rectal, subcutaneous, transdermal, intravenous, intramuscular, or intraperitoneal route. In particular embodiments, the neuromedin U receptor agonists can be used to treat multiple disorders in an individual. As will be apparent to one of ordinary skill in the art in view of the disclosure herein, the neuromedin U receptor agonists can be administered to an individual to effect a reduction in food intake by the individual, to effect a reduction in weight gain in the individual, to prevent weight gain in the individual, to effect weight loss in the individual, and/or to prevent weight regain in the individual.

Research tool uses may involve the use of a neuromedin U receptor agonist and the presence of an NMU receptor or fragment thereof. Examples of research tool uses include screening for compounds active at NMU receptors, determining the presence of NMU receptors in a sample or preparation, and examining the role or effects of NMU. Additionally, the neuromedin U receptor agonists can be used to screen for NMU binding compounds (agonists or antagonists) by using a neuromedin U receptor agonist in a competition experiment with test compounds.

The neuromedin U receptor agonists comprise the general formula (I)

Z¹-peptide-Z²

wherein the peptide has the amino acid sequence X¹-X²-X³-X⁴-X⁴-X⁵-X⁷-X⁸-X⁹-X¹⁰-X^{1 1}-X¹²-X¹³-X¹⁴-X¹⁵-X¹⁶-X¹⁷⁻X¹⁸-X¹⁹-X²⁰-X²¹-X²²-X²³-X²⁴-X²⁵ (SEQ ID NO:27) wherein amino acids 1 to 17 can be any amino acid or absent, wherein amino acid X¹⁸ is absent, Y, W, F, a des-amino acid or an acyl group; amino acid X¹⁹ is A, W, Y, F or an aliphatic amino acid; amino acid X²⁰ is absent, L, G, sarcosine (Sar), D-Leu, NMe-Leu, D-Ala or A; amino acid X²¹ is F, NMe-Phe, an aliphatic amino acid, an aromatic amino acid, A or W; X²² is R, K, A or L; amino acid X²³ is P, Sar, A or L; amino acid X²⁴ is R, Harg or K; and amino acid X²⁵ is N, any D- or L-amino acid, Nle or D-Nle, A; and Z¹ is and optionally present protecting group that, if present, is joined to the N-terminal amino group; and Z² is NH₂ or an optionally present protecting group that, if present, is joined to the C-terminal carboxy group, and pharmaceutically acceptable salts thereof.

In particular embodiments, the peptide comprises the amino acid sequence X¹-X²-X³-X⁴-X⁵-X⁶-X⁷-X⁸-X⁹-X¹⁰-X¹¹-X¹²-X¹³-X¹⁴-X¹⁶-X¹⁶-X¹⁷-X¹⁸-F-L-R-P-R-N (SEQ ID NO:1) wherein amino acids 1 to 17 can be any amino acid or absent. The amino acid sequences of particular neuromedin U receptor agonists having the above amino acid sequence are shown in Table 1.

In further embodiments, the peptide comprises the amino acid sequence F-R-V-D-E-E-F-Q-S-P-F-A-S-Q-S-R-G-X¹⁸-X¹⁹-X²⁰-X²¹-X²²-X²³-X²⁴-X²⁵ (SEQ ID NO:7) wherein amino acid X¹⁸ is absent, Y, W, F, a des-amino acid or an acyl group; amino acid X¹⁹ is A, W, Y, F or an aliphatic amino acid; amino acid X²⁰ is absent, G, sarcosine (Sar), D-Leu, NMe-Leu, D-Ala or A; amino acid X²¹ is NMe-Phe, an aliphatic amino acid, an aromatic amino acid, A or W; amino acid X²² is K, A or L; amino acid X²³ is Sar, A or L; amino acid X²⁴ is Harg or K; and amino acid X²⁵ is any D- or L-amino acid, Nle or D-Nle, or A. Examples of peptides having the above amino acid sequence are shown in Table 2.

In yet another embodiment, the peptide comprises the amino acid sequence X¹-X²-X³-X⁴-X⁵-X⁶-X⁷-X⁸ (SEQ ID NO:8) wherein amino acid X¹ is absent, Y, W, F, a des-amino acid or an acyl group; amino acid X² is A, W, Y, F or an aliphatic amino acid; amino acid X3 is absent, G, sarcosine (Sar), D-Leu, NMe-Leu, D-Ala or A; amino acid X⁴ is NMe-Phe, an aliphatic amino acid, an aromatic amino acid, A or W; amino acid X⁵ is K, A or L; amino acid X⁶ is Sar, A or L; amino acid X⁷ is Harg or K; and amino acid X⁸ is any D- or L-amino acid, Nle or D-Nle, or A. Examples of peptides having the above amino acid sequence are shown in Table 2.

In particular aspects, the neuromedin U receptor agonist optionally includes a protecting group covalently joined to the N-terminal amino group. A protecting group-covalently joined to the N-terminal amino group of the neuromedin U receptor agonists reduces the reactivity of the amino terminus under in vivo conditions. Amino protecting groups include -C₁-₁₀ alkyl, -C₁₋₁₀ substituted alkyl, -C₂₋₁₀ alkenyl, -C₂₋₁₀ substituted alkenyl, aryl, -C₁₋₆ alkyl aryl, -C(O)-(CH₂)₁₋₆-COOH, -C(O)-C₁₋₆ alkyl, -C(O)-aryl, -C(O)-O-C₁₋₆ alkyl, or -C(O)-O-aryl. In particular embodiments, the amino terminus protecting group is selected from the group consisting of acetyl, propyl, succinyl, benzyl, benzyloxycarbonyl, and t-butyloxycarbonyl. Deamination of the N-terminal amino acid is another modification that is contemplated for reducing the reactivity of the amino terminus under in vivo conditions.

Chemically modified compositions of the neuromedin U receptor agonists wherein the neuromedin U receptor agonist derivatives are linked to a polymer are also included . The polymer selected is usually modified to have a single reactive group, such as an active ester for acylation or an aldehyde for alkylation, so that the degree of polymerization may be controlled as provided for in the present methods. Included within the scope of polymers is a mixture of polymers. Preferably, for therapeutic use of the end-product preparation, the polymer will be pharmaceutically acceptable.

The polymer or mixture thereof may be selected from the group consisting of; for example, polyethylene glycol (PEG), monomethoxy polyethylene glycol, dextran, cellulose, or other carbohydrate based polymers, poly-(N-vinyl pyrrolidone) polyethylene glycol, propylene glycol homopolymers, a polypropylene oxide/ethylene oxide co-polymer, polyoxyethylated polyols (for example, glycerol), and polyvinyl alcohol.

In further still embodiments, the neuromedin U receptor agonists are modified by PEGylation, cholesteroylation, or palmitoylation. The modification can be to any amino acid residue in the neuromedin U receptor agonist, however, in currently preferred embodiments, the modification is to the N-terminal amino acid of the neuromedin U receptor agonist, either directly to the N-terminal amino acid or by way coupling to the thiol group of a cysteine residue added to the N-terminus or a linker added to the N-terminus such as Ttds. In further embodiments, the N-terminus of the neuromedin U receptor agonist comprises a cysteine residue to which a protecting group is coupled to the N-terminal amino group of the cysteine residue and the cysteine thiolate group is derivatized with N-ethylmaleimide, PEG group, cholesterol group, or palmitoyl group. In further still embodiments, an acetylated cysteine residue is added to the N-terminus of the neuromedin U receptor agonists, and the thiol group of the cysteine is derivatized with N-ethylmaleimide, PEG group, cholesterol group, or palmitoyl group.

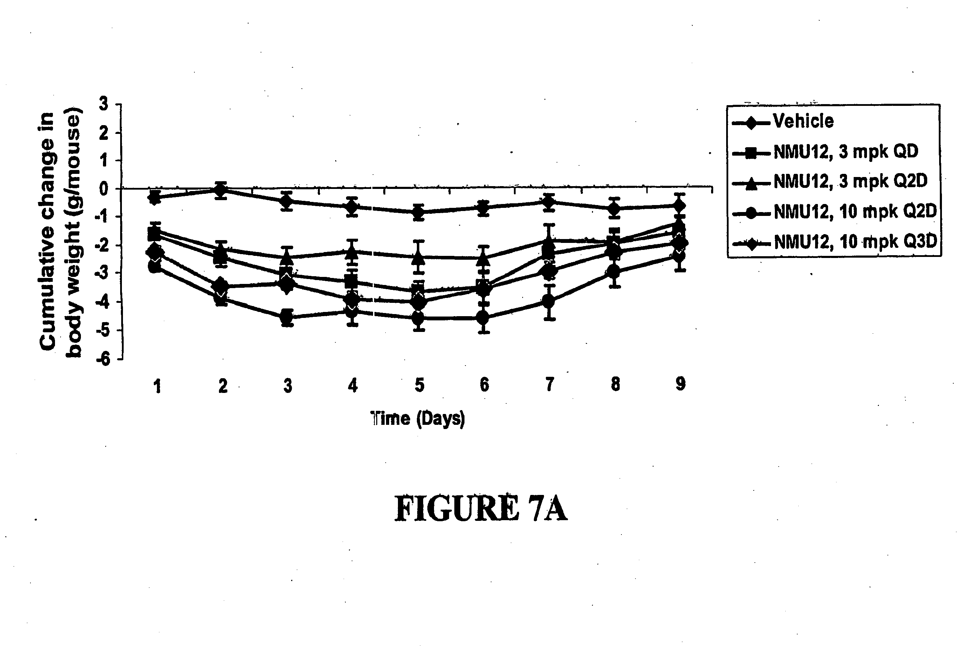

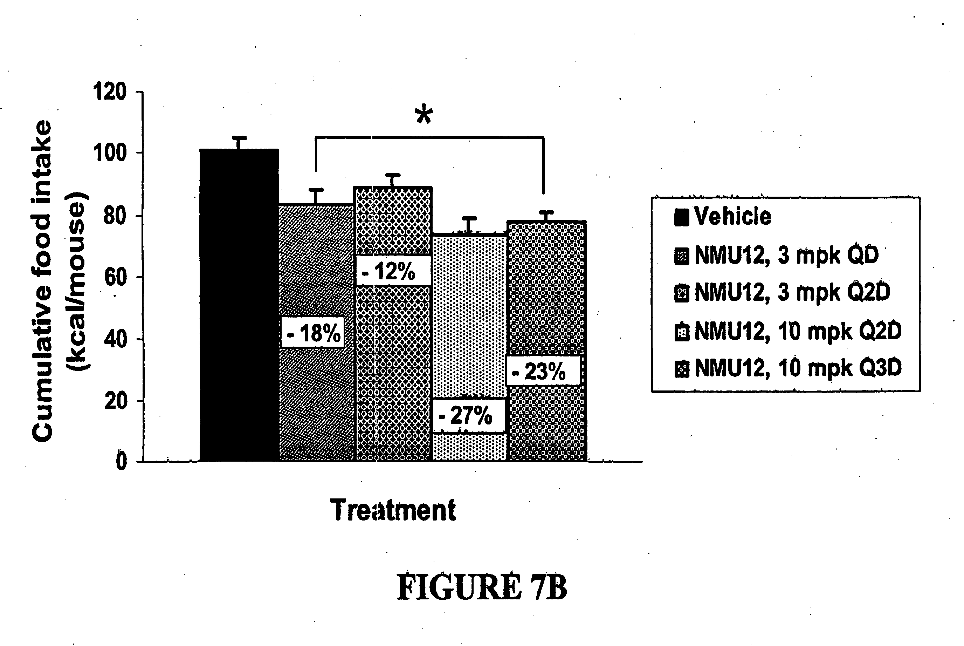

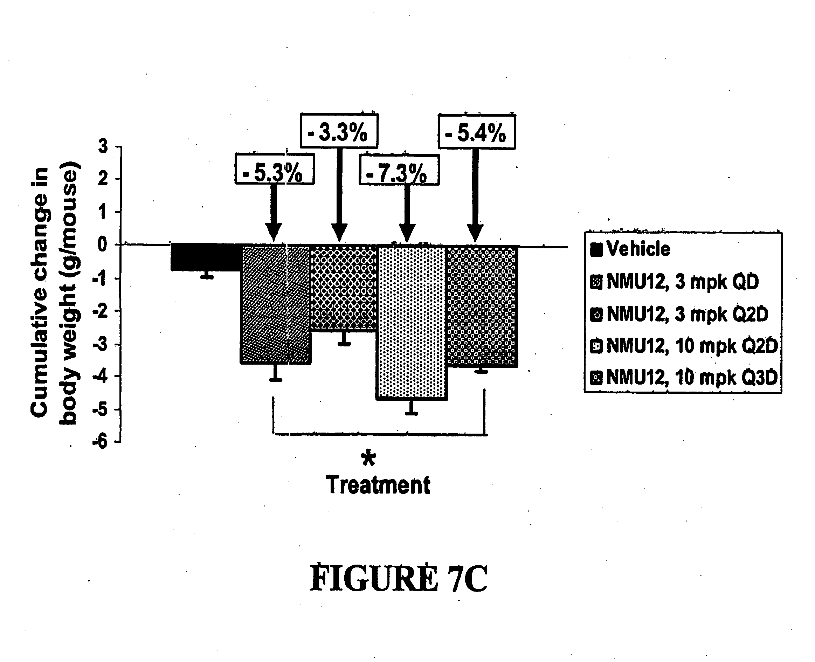

It is well known that the properties of certain proteins can be modulated by attachment of polyethylene glycol (PEG) polymers, which increases the hydrodynamic volume of the protein and thereby slows its clearance by kidney filtration. (*See, f*or example, Clark et al., J. Biol. Chem. 271: 21969-21977 (1996)). Therefore, it is envisioned that the core peptide residues can be PEGylated to provide enhanced therapeutic benefits such as, for example, increased efficacy by extending half-life in vivo. The inventors have discovered that including a PEG group at the N-terminus of the neuromedin U receptor agonist, not only extends the serum half-life of the PEGylated neuromedin U receptor agonist compared to the native NMU-25 peptide but also enables particular neuromedin U receptor agonists such as NMU12 to cross the blood-brain barrier and interact with the NMUR2 receptors in the brain. In general, after intravenous administration, native NMU-25 is cleared rapidly from the systemic circulation. As shown in Figure 7, at 37 °C, the half-life of intact human NMU-25 (NMU1) is significantly increased by covalent attachment of a polyethylene glycol molecule to the amino group at the N-terminal phenylalanine residue of the molecule or to the thiol group of a cysteine residue covalently joined by an amide bond to the N-terminal phenylalanine residue of NMU-25 (NMU12). Thus, PEGylating the neuromedin U receptor agonists will improve the pharmacokinetics and pharmacodynamics of the neuromedin U receptor agonists.

Peptide PEGylation methods are well known in the literature and described in the following references : Lu et al., Int. J. Pept. Protein Res.43: 127-38 (1994); Lu et al., Pept. Res. 6: 140-6 (1993); Felix et al., Int. J. Pept. Protein Res. 46: 253-64 (1995); Gaertner et al., Bioconjug. Chem. 7: 38-44 (1996); Tsutsumi et al., Thromb. Haemost. 77: 168-73 (1997); Francis et al., Int. J. Hematol. 68: 1-18 (1998); Roberts et al., J. Pharm. Sci. 87: 1440-45 (1998); and Tan et al., Protein Expr. Purif. 12: 45-52 (1998). Polyethylene glycol or PEG is meant to encompass any of the forms of PEG that have been used to derivatize other proteins, including, but not limited to, mono-(C₁₋₁₀) alkoxy or aryloxy-polyethylene glycol. Suitable PEG moieties include, for example, 40 kDa methoxy poly(ethylene glycol) propionaldehyde (Dow, Midland, Michigan); 60 kDa methoxy poly(ethylene glycol) propionaldehyde (Dow, Midland, Michigan); 40kDa methoxy poly(ethylene glycol) maleimido-propionamide (Dow, Midland, Michigan); 31 kDa alpha-methyl-w-(3-oxopropoxy), polyoxyethylene (NOF Corporation, Tokyo); mPEG₂-NHS-40k (Nektar); mPEG₂-MAL-40k (Nektar), SUNBRIGHT GL2-400MA ((PEG)₂40kDa) (NOF Corporation, Tokyo), SUNBRIGHT ME-200MA (PEG20kDa) (NOF Corporation, Tokyo), The PEG groups are generally attached to the neuromedin U receptor agonists via acylation or reductive alkylation through a reactive group on the PEG moiety (for example, an aldehyde, amino, thiol, or ester group) to a reactive group on the neuromedin U receptor agonist (for example, an aldehyde, amino, thiol, or ester group).

The PEG molecule(s) may be covalently attached to any Lys, Cys, or K(CO(CH₂)₂SH) residues at any position in the neuromedin U receptor agonist. The neuromedin U receptor agonists described herein can be PEGylated directly to any amino acid at the N-terminus by way of the N-terminal amino group. A "linker arm" may be added to the neuromedin U receptor agonist to facilitate PEGylation. PEGylation at the thiol side-chain of cysteine has been widely reported-(*See*, e.g., Caliceti & Veronese, Adv. Drug Deliv. Rev. 55: 1261-77 (2003)). If there is no cysteine residue in the peptide, a cysteine residue can be introduced through substitution or by adding a cysteine to the N-terminal amino acid. Those neuromedin U receptor agonists, which have been PEGylated, have been PEGylated through the side chains of a cysteine residue added to the N-terminal amino acid. Alternatively, the PEG molecule(s) may be covalently attached to an amide group in the C-terminus of the neuromedin U receptor agonist. In general, there is at least one PEG molecule covalently attached to the Neuromedin U receptor agonist. In particular aspects, the PEG molecule is branched while in other aspects, the PEG molecule may be linear. In particular aspects, the PEG molecule is between 1 kDa and 100 kDa in molecular weight. In further aspects, the PEG molecule is selected from 10, 20, 30, 40, 50 and 60 kDa. In further still aspects, it is selected from 20, 40, or 60 kDa. Where there are two PEG molecules covalently attached to the neuromedin U receptor agonist of the present invention, each is 1 to 40 kDa and in particular aspects, they have molecular weights of 20 and 20 kDa, 10 and 30 kDa, 30 and 30 kDa, 20 and 40 kDa, or 40 and 40 kDa. In particular aspects, the neuromedin U receptor agonists contain mPEG-cysteine. The mPEG in mPEG-cysteine can have various molecular weights. The range of the molecular weight is preferably 5 kDa to 200 kDa, more preferably 5 kDa to 100 kDa, and further preferably 20 kDa to 60 kDA. The mPEG can be linear or branched.

Currently, it is preferable that the neuromedin U receptor agonists are PEGylated through the side chains of a cysteine added to the N-terminal amino acid. Currently, the agonists preferably contain mPEG-cysteine. The mPEG in mPEG-cysteine can have various molecular weights. The range of the molecular weight is preferably 5kDa to 200kDa, more preferably 5kDa to 100kDa, and further preferably 20kDa to 60kDA. The mPEG can be linear or branched.

A useful strategy for the PEGylation of synthetic neuromedin U receptor agonists, consists of combining, through forming a conjugate linkage in solution, a peptide, and a PEG moiety, each bearing a special functionality that is mutually reactive toward the other. The neuromedin U receptor agonists can be easily prepared with conventional solid phase synthesis. The neuromedin U receptor agonist is "preactivated" with an appropriate functional group at a specific site. The precursors are purified and fully characterized prior to reacting with the PEG moiety. Conjugation of the peptide with PEG usually takes plate in aqueous phase and can be easily monitored by reverse phase analytical HPLC. The PEGylated neuromedin U receptor agonist can be easily purified by cation exchange chromatography or preparative HPLC and characterized by analytical HPLC, amino acid analysis and laser desorption mass spectrometry.

The neuromedin U receptor agonist can comprise other non-sequence modifications, for example, glycosylation, lipidation, acetylation, phosphorylation, carboxylation, methylation, or any other manipulation or modification, such as conjugation with a labeling component. While, in particular aspects, the neuromedin U receptor agonist herein utilize naturally-occurring amino acids or D isoforms of naturally occurring amino acids, substitution with non-naturally occurring amino acids (for example., methionine sulfoxide, methionine methylsulfonium, norleucine, epsilon-aminocaproic acid, 4-aminobutanoic acid, tetrahydroisoquinoline-3-carboxylic acid, 8-aminocaprylic acid, 4 aminobutyric acid, Lys(N(epsilon)-trifluoroacetyl) or synthetic analogs, for example, o-aminoisobutyric acid, p or y-amino acids, and cyclic analogs. In further still aspects, the neuromedin U receptor agonists comprise a fusion protein that having a first moiety, which is a neuromedin U receptor agonist, and a second moiety, which is a heterologous peptide.

The neuromedin U receptor agonist may be modified by a variety of chemical techniques to produce derivatives having essentially the same activity as the unmodified neuromedin U receptor agonist and/or having other desirable properties. A protecting group covalently joined to the C-terminal carboxy group reduces the reactivity of the carboxy terminus under *in vivo* conditions. For example, carboxylic acid groups of the peptide, whether carboxyl-terminal or side chain, may be provided in the form of a salt of a pharmacologically-acceptable cation or esterified to form a C₁₋₆ ester, or converted to an amide of formula NRR₂ wherein R and R₂ are each independently H or C₁₋₆ alkyl, or combined to form a heterocyclic ring, such as a 5-or 6-membered ring. The carboxy terminus protecting group is preferably attached to the α-carbonyl group of the last amino acid. Carboxy terminus protecting groups include, but are not limited to, amide, methylamide, and ethylamide. Amino groups of the peptide, whether N-terminal or side chain, may be in the form of a pharmacologically-acceptable acid addition salt, such as the HCl, HBr, acetic, benzoic, toluene sulfonic, maleic, tartaric, and other organic salts, or may be modified to C₁₋₆ alkyl or dialkyl amino or further converted to an amide.

Hydroxyl groups of the neuromedin U receptor agonist side chain maybe converted to C₁₋₆ alkoxy or to a C₁₋₆ ester using well-recognized techniques. Phenyl and phenolic rings of the peptide side chain may be substituted with one or more halogen atoms, such as fluorine, chlorine, bromine or iodine, or with C₁₋₆ alkyl, C₁₋₆ alltoxy, carboxylic acids and esters thereof, or amides of such carboxylic acids. Methylene groups of the neuromedin U receptor agonist side chains can be extended to homologous C₂₋₄ alkylenes. Thiols can be protected with any one of a number of well-recognized protecting groups, such as acetamide groups. Those skilled in the art will also recognize methods for introducing cyclic structures into the peptides of this invention to select and provide conformational constraints to the structure that result in enhanced stability. For example, a carboxyl-terminal or amino-terminal cysteine residue can be added to the peptide, so that when oxidized the peptide will contain a disulfide bond, thereby generating a cyclic peptide. Other peptide cyclizing methods include the formation of thioethers and carboxyl-and amino-terminal amides and esters.

Polysaccharide polymers are another type of water soluble polymer that may be used for protein modification. Dextrans are polysaccharide polymers comprised of individual subunits of glucose predominantly linked by α1-6 linkages. The dextran itself is available in many molecular weight ranges, and is readily available in molecular weights from about 1 kDa to about 70 kDa. Dextran is a suitable water soluble polymer for use as a vehicle by itself or in combination with another vehicle (*See,* for example, WO 96/11953 and WO 96/05309). The use of dextran conjugated to therapeutic or diagnostic immunoglobulins has been reported; see, for example, European Patent Publication No. 0 315 456. Dextran of about 1 kDa to about 20 kDa is preferred when dextran is used as a vehicle in accordance with the present invention.

As described above, the presence of a "linker" group is optional. When present, its chemical structure is not critical, since it serves primarily as a spacer. However, in certain embodiments, the linker may itself provide improved properties to the compositions of the present invention. The linker is preferably made up of amino acids linked together by peptide bonds. Thus, in particular embodiments, the linker is made up of from 1, to 20 amino acids linked by peptide bonds, wherein the amino acids are selected from the 20 naturally occurring amino acids. Some of these amino acids may be glycosylated, as is well understood by those in the art. In a more preferred embodiment, the 1 to 20 amino acids are selected from glycine, alanine, proline, asparagine, glutamine, and lysine. Even more preferably, a linker is made up of a majority of amino acids that are sterically unhindered, such as glycine and alanine. Thus, preferred linkers are polyglycines (particularly (Gly)4, (Gly)5), poly(Gly-Ala), and polyalanines. Other specific examples of linkers are (Gly)3Lys(Gly)4; (Gly)3AsnGlySer(Gly)2; (Gly)3Cys(Gly)4; and GlyProAsnGlyGly.

Non-peptide linkers can also be used. For example, alkyl linkers such as-NH-(CH₂)ₛ-C(O)-, wherein s = 2-20 could be used. These alkyl linkers may further be substituted by any non-sterically hindering group such as lower alkyl (for example, C₁₋₆) lower acyl, halogen (for example, Cl, Br), CN, NH₂, phenyl, and the like. An exemplary non-peptide linker is a PEG linker, wherein n is such that the linker has a molecular weight of 100 to 5000 kD, preferably 100 to 500 kD. The peptide linkers may be altered to form derivatives in the same manner as described above. Other linkers include Ttds (1-amino-4,7,10-trioxa-13-tridecanamine succinimic acid).

The present invention includes diastereomers as well as their racemic and resolved enantiomerically pure forms. The neuromedin U receptor agonists can contain D-amino acids, L-amino acids, or a combination thereof. In general, the amino acids are in the L-form with particular amino acids in D-form. As is known in the art, individual amino acids can be represented as follows: A=Ala=Alanine; C=Cys=Cysteine; D=Asp=Aspartic Acid; E=Glu=Glutamic Acid; F=Phe=Phenylalanine; G=Gly=Glycine; H=His=Histidine; I=Ile=Isoleucine; K=Lys=Lysine; L=Leu=Leucine; M=Met=Methionine; N=Asn=Asparagine; P=Pro=Proline; Q=Gln=Glutamine; R=Arg=Arginine; S=Ser=Serine; T=Thr=Threonine; V=Val=Valine; W=Trp=Tryptophan; and Y=Tyr=Tyrosine.

Examples of neuromedin U receptor agonists comprising the amino acid sequence X¹-X²-X³-X⁴-X⁵-X⁶-X⁷-X⁸-X⁹-X¹⁰-X¹¹-X¹²-X¹³-X¹⁴-X¹⁵-X¹⁶-X¹⁷-X¹⁸-F-L-F-R-P-R-N (SEQ ID NO:1) wherein amino acids 1 to 17 can be any amino acid or absent are shown in Table 1 . The neuromedin U receptor agonists are protected at the C-terminus with an amino group and at the N-terminus with an acetyl group (except for neuromedin U receptor agonist NMU1). With the exception ofneuromedin U receptor agonist NMU2, the neuromedin U receptor agonists further include a cysteine residue at the N-terminus to which the acetyl group is covalently linked to the amino group of the cysteine residue. As shown in the table, for many of the neuromedin U receptor agonists, the thiol group of the cysteine residue is reacted with a second group. For example, for neuromedin U receptor agonists in the table shown with a C₁ at the N-terminus, the neuromedin U receptor agonist has an N-acetylated cysteine residue at the N-terminus of the neuromedin U receptor agonist linked by way of its thiol group to N-ethylmaleimidyl; for neuromedin U receptor agonists in the table shown with a C₂ at the N-terminus, the neuromedin U receptor agonist has an N-acetylated cysteine residue at the N-terminus of the neuromedin U receptor agonist linked by way of its thiol group to a (PEG)₂40kDa; for neuromedin U receptor agonists shown with a C₄ at the N-terminus of the neuromedin U receptor agonist, the neuromedin U receptor agonist has an N-acetylated-cysteine residue at the N-terminus of the neuromedin U receptor agonist linked by way of its thiol group to a (PEG)20kDa; for neuromedin U receptor agonists shown with a-C₅ at the N-terminus of the neuromedin U receptor agonist, the neuromedin U receptor agonist has an N-acetylated cysteine residue at the N-terminus of the neuromedin U receptor agonist linked by way of its thiol group to a (PEG)₂20kDa; for neuromedin U receptor agonists shown with a C₆ at the N-terminus of the neuromedin U receptor agonist, the neuromedin U receptor agonist has an N-acetylated cysteine residue at the N-terminus of the neuromedin U receptor agonist linked by way of its thiol group to a (PEG)40kDa; for neuromedin U receptor agonists shown with a C₃ at the N-terminus of the neuromedin receptor agonist, the neuromedin U receptor agonist has an N-acetylated cysteine residue at the N-terminus of the neuromedin U receptor agonist linked by way of its thiol group to Cholesterol.

| Table 1 | | |
|---|---|---|
| SEQ ID NO. | Peptides | Neuromedin U Receptor Agonist Sequences |
| 2 | NMU | FRVDEEFQSPFASQSRGYFLFRPRN-CONH₂ |
| 2 | NMU1 | **P**₁-FRVDEEFQSPFASQSRGYFLFRPRN-CONH₂ |
| 2 | NMU2 | **Ac**-FRVDEEFQSPFASQSRGYFLFRPRN-CONH₂ |
| 4 | NMU3 | GYFLFRPRN-CONH₂ |
| 28 | NMU4 | FRVDEEFQSPFASQSRPYFLFRPRN-CONH₂ |
| 28 | NMU5 | **Ac**-FRVDEEFQSPFASQSR**P**YFLFRPRN-CONH₂ |
| 4 | Pre-1 | **Ac-C**GYFLFRPRN-CONH₂ |
| 4 | NMU6 : | **Ac-C**₁GYFLFRPRN-CONH₂ |
| 4 | NMU7 | **Ac-C**₂GYFLFRPRN-CONH₂ |
| 4 | NMU11 | **Ac-C**₃GYFLFRPRN-CONH₂ |
| 2 | Pre-2 | **Ac-C**FRVDEEFQSPFASQSRGYFLFRPRN-CONH₂ |
| 2 | NMU8 | **Ac-C**₁FRVDEEFQSPFASQSRGYFLFRPR-CONH₂ |
| 2 | NMU9 | **Ac-C**₂FRVDEEFQSPFASQSRGYFLFRPRN-CONH₂ |
| 2 | NMU12 | **Ac-C**₂FRVDEEFQSPFASQSRGYFLFRPRN-CONH₂ |
| 2 | NMU10 | **Ac-C**₃FRVDEEFQSPFASQSRGYFLFRPRN-CONH₂ |
| 2 | NMU21 | **Ac-C**₄FRVDEEFQSPFASQSRGYFLFRPRN-CONH₂ |
| 2 | NMU26 | **Ac-C**₅FRVDEEFQSPFASQSRGYFLFRPRN-CONH₂ |
| 25 | Pre-3 | **Ac-C**FRVDEEFQSPFASQSRGYFaWRPRN-CONH₂ |
| 25 | NMU13 | **Ac-C**₁FRVDEEFQSPFASQSRGYFaWRPRN-CONH₂ |
| 25 | NMU14 | **Ac-C**₂FRVDEEFQSPFASQSRGYFaWRPRN-CONH₂ |
| 5 | Pre-4 | **Ac-C**-Ttds-FLFRPRN-CONH₂ |
| 5 | NMU15 | **Ac-C**₁-Ttds-FLFRPRN-CONH₂ |
| 5 | NMU16 | **Ac-C**₂-Ttds-FLFRPRN-CONH₂ |
| 3 | Pre-5 | **Ac-C**ASQSRGYFLFRPRN CONH₂ |
| 3 | NMU17 | **Ac-C**₁ASQSRGYFLFRPRN-CONH₂ |
| 3 | NMU18 | **Ac-C**₂ASQSRGYFLFRPRN-CONH₂ |
| 6 | Pre-6 | **Ac-C**FQSPFASQSGYFLFRPRN-CONH₂ |
| 6 | NMU19 | **Ac-C**₁FQSPFASQSRGYFLFRPRN-CONH₂ |
| 6 | NMU20 | **Ac-C**₂FQSPFASQSRGYFLFRPRN-CONH₂ |
| 2 | Pre-7 | **Ac-C**-**C**-FRVDEEFQSPFASQSRGYFLFRPRN-CONH₂ |
| 2 | NMU22 | **Ac-C**₁-**C**₁-FRVDEEFQSPFASQSRGYFLFRPRN-CONH₂ |
| 2 | NMU23 | **Ac-C**₄-**C**₄-FRVDEEPQSPFASQSRGYFLFRPRN-CONH₂ |
| 2 | Pre-8 | **Pam-C**FRVDEEFQSPFASQSRGYFLFRPRN-CONH₂ |
| 2 | NMU24 | **Pam-C**₁FRVDEEFQSPFASQSRGYFLFRPRN-CONH₂ |
| 2 | NMU25 | **Pam-C**₂FRVDEEFQSPFASQSRQYFLFRPRN-CONH₂ |
| 2 | NMU27 | **Ac-C**₆FRVDEEFQSPFASQSRGYFLFRPRN-CONH₂ |
| 4 | Pre-9 | **Ac-C**-TtdS-GYFLFRPRN-CONH₂ |
| 4 | NMU28 | **Ac-C**-Ttds-GYFLFRPRN-CONH₂ |
| 4 | NMU29 | **Ac-C**₂-Ttds-GYFLFRPRN-CONH₂ |
| 28 | NMU30 | **Ac-C**₁FRVDEEFQSPFASQSRPYFLFRPRN-CONH₂ |
| 28 | NMU31 | **Ac-C**₂FRVDEEFQSPFASQSRPYFLFRPRN-CONH₂ |
| **C** = cysteine; **P₁** = (PEG)₂40kDa; **C₁** = Cys(N-ethylmaleimidyl), **C₂** =Cys(PEG)₂40kDa, **C₄** = Cys(PEG)20kDa, **C₅** = Cys(PEG)₂20kDa, **C₆** =Cys(PEG)40kDa each corresponding to a cysteine residue PEGylated via the side-chain thiol with a branched PEG [(PEG)₂] or a linear PEG of the indicated MW; **C₃** = Cys(Cholesteroyl), corresponding to a cysteine residue linked to cholesterol via the side-chain thiol; **Ttds**, 1-amino-4,7,10-trioxa-13-tridecanamine succinimic acid; a, D-Alanine; **A**c = acetyl; **Pam** = palmitoyl | | |

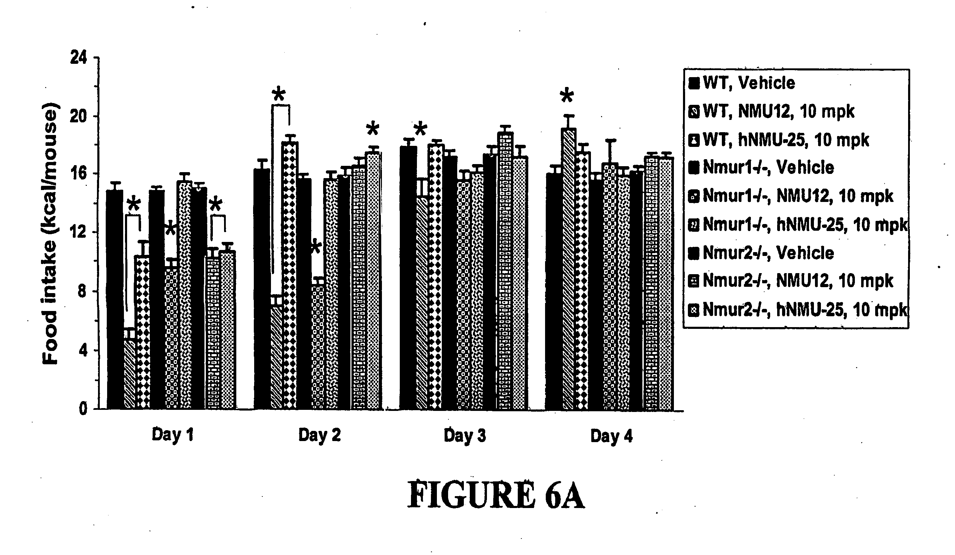

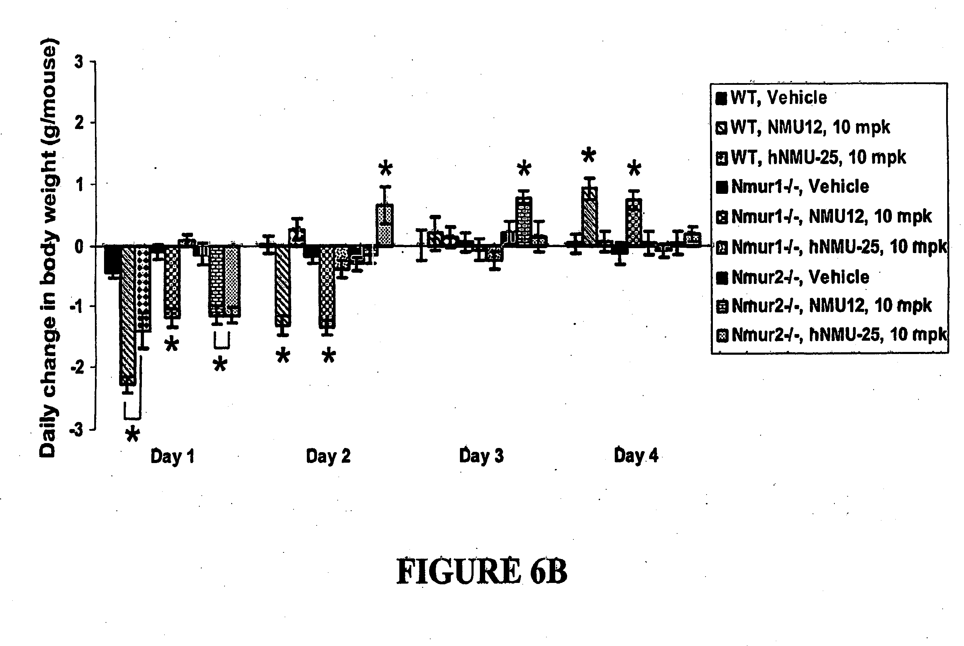

The neuromedin U receptor agonists shown in Table 1, with the exception of those neuromedin U receptor agonists comprising the amino acid sequence of SEQ ID NO:25, are bispecific in that they can bind and activate either NMUR1 or NMUR2 receptors. The neuromedin U receptor agonists comprising SEQ ID NO:25 have been found to be NMUR1 specific. PEGylation of the neuromedin U receptor agonists shown in Table 1 appear to extend the serum half-life of the neuromedin U receptor agonists and significantly, render particular neuromedin U receptor agonists such as NMU12 to be capable of crossing the blood-brain barrier. For example, as shown in Example 4 and Figures 6A and 6B, NMU12 was shown to be able to reduce food intake and reduce weight gain in *Nmur1* knockout mice. The results indicate that PBGylated peptide NMU12 administered peripherally was able to cross the blood-brain barrier. PEGylation appeared to extend the serum half-life of NMU1, NMU9, and NMU20 by three days and NMU11 and NMU18 by two days. NMU9 and NMU12 differ by the source of(PEG)₂40kDa covalently joined to the thiol group of the N-terminal cysteine residue.

Examples of neuromedin U receptor agonists comprising the amino acid sequence F-R-V-D-E-E-F-Q-S-P-F-A-S-Q-S-R-G-X¹⁸-X¹⁹-X²⁰-X²¹-x²²-X²³-X²⁴-X²⁵ (SEQ ID NO:7) or X¹-X²-X³-X⁴-X⁵-X⁶-X⁷-X⁸ (SEQ ID NO:8) wherein amino acid X¹⁸ or X¹ is absent, Y, W, F, a des-amino acid or an acyl group; amino acid X¹⁹ or X² is A, W, Y, F or an aliphatic amino acid; amino acid X²⁰ or X³ is absent, G, sarcosine (Sar), D-Leu, NMe-Leu, D-Ala or A; amino acid X²¹ or X⁴ is NMe-Phe, an aliphatic amino acid, an aromatic amino acid, A or W; amino acid X²² or X⁵ is K, A or L; amino acid X²³ or X⁶ is Sar, A or L; amino acid X²⁴ or X⁷ is Harg or K; and amino acid X²⁵ or X⁸ is any D- or L-amino acid, Nle or D-Nle, or A. Examples ofpeptides having the above amino acid sequence are shown in Table 2. In general, the peptides comprising SEQ ID NO:7 or SEQ ID NO:8 are specific for NMUR1 receptor; however, as shown in the Example, neuromedin U receptor agonists N, O, and P are bispecific.

| Table 2 | | |
|---|---|---|
| SEQ ID NO. | Peptides | Neuromedin U Receptor Agonist Sequences |
| 9 | A | YFWRPRN-CONH₂ |
| 10 | B | YF-(D-L)-WRPRN-CONH₂ |
| 11 | C | YFGWRPRN-CONH₂ |
| 12 | D | YF-(D-A)-WRPRN-CONH₂ |
| 26 | E | Ac-F-(D-L)-WRPRN-CONH₂ |
| 13 | F | Ac-FFRPRN-CONH₂ |
| 14 | G | RVDEEFQSPFASQSRGYFWRPRN-CONH₂ |
| 15 | H | FRVDEEFQSPFASQSRGYF-(D-L)-WRPRN-CONH₂ |
| 16 | I | FWLFRP-(Harg)-N-CONH₂ |
| 17 | J | FWLFRA-(Harg)-N-CONH₂ |
| 18 | K | WFLFRAR-(D-Nle)-CONH₂ |
| 19 | L | FWLFRARN-CONH₂ |
| 20 | M | WALFRARN-CONH₂ |
| 21 | N | FALFRPRN-CONH₂ |
| 22 | O | FRVDEEFQSPFASQSRGFWLFRP-(Harg)-N-CONH₂ |
| 23 | P | FRVDEEFQSPFASQSRGFWLFRA-(Harg)-N-CONH₂ |
| 24 | Q | FRVDEEFQSPFASQSRGFWLFRPR-(D-Nle)-CONH₂ |

Further provided are pharmaceutical compositions comprising a therapeutically effective amount of one or more of the neuromedin U receptor agonists disclosed herein for the treatment of a metabolic disorder in an individual. Such disorders include, but are not limited to, obesity, metabolic syndrome or syndrome X, type II diabetes, complications of diabetes such as retinopathy, hypertension, dyslipidemias, cardiovascular disease gallstones, osteoarthritis, and certain forms of cancers. The obesity-related disorders herein are associated with, caused by, or result from obesity.

"Obesity" is a condition in which there is an excess of body fat. The operational definition of obesity is based on the Body Mass Index (BMI), calculated as body weight per height in meters squared (kg/m2). "Obesity" refers to a condition whereby an otherwise healthy subject has a Body Mass Index (BMI) greater than or equal to 30 kg/m2, or a condition whereby a subject with at least one co-morbidity has a BMI greater than or equal to 27 kg/m2. An "obese subject" is an otherwise healthy subject with a Body Mass Index (BMI) greater than or equal to 30 kg/m2 or a subject with at least one co-morbidity with a BMI greater than or equal to 27 kg/m2. A "subject at risk for obesity" is an otherwise healthy subject with a BMI of 25 kg/m2 to less than 30 kg/m2 or a subject with at least one co-morbidity with a BMI of 25 kg/m2 to less than 27 kg/m2.

The increased risks associated with obesity occur at a lower Body Mass Index (BMI) in Asians. In Asian countries, including Japan, "obesity" refers to a condition whereby a subject with at least one obesity-induced or obesity-related co-morbidity that requires weight reduction or that would be improved by weight reduction, has a BMI greater than or equal to 25 kg/m2. In Asian countries, including Japan, an "obese subject" refers to a subject with at least one obesity-induced or obesity-related co-morbidity that requires weight reduction or that would be improved by weight reduction, with a BMI greater than or equal to 25 kg/m2. In Asian countries, a "subject at risk of obesity" is a subject with a BMI of greater than 23 kg/m2 to less than 25 kg/m2.

As used herein, the term "obesity" is meant to encompass all of the above definitions of obesity.

Obesity-induced or obesity-related co-morbidities include, but are not limited to, diabetes, non-insulin dependent diabetes mellitus - type 2, impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility. In particular, co-morbidities include: hypertension, hyperlipidemia, dyslipidemia, glucose intolerance, cardiovascular disease, sleep apnea, diabetes mellitus, and other obesity-related conditions.

"Treatment" (of obesity and obesity-related disorders) refers to the administration of the compounds of the present invention to reduce or maintain the body weight of an obese subject. One outcome of treatment may be reducing the body weight of an obese subject relative to that subject's body weight immediately before the administration of the compounds of the present invention. Another outcome of treatment may be preventing body weight regain of body weight previously lost as a result of diet, exercise, or pharmacotherapy. Another outcome of treatment may be decreasing the occurrence of and/or the severity of obesity-related diseases. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

"Prevention" (of obesity and obesity-related disorders) refers to the administration of the compounds of the present invention to reduce or maintain the body weight of a subject at risk of obesity. One outcome of prevention may be reducing the body weight of a subject at risk of obesity relative to that subject's body weight immediately before the administration of the compounds of the present invention. Another outcome of prevention may be preventing body weight regain of body weight previously lost as a result of diet, exorcise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence, progression or severity of obesity-related disorders, such as, but not limited to, arteriosclerosis, Type II diabetes, polycystic ovarian disease, cardiovascular diseases, osteoarthritis, dermatological disorders, hypertension, insulin resistance, hypercholesterolemia, hypertriglyceridemia, and cholelithiasis.

The obesity-related disorders herein are associated with, caused by, or result from obesity. Examples of obesity-related disorders include overeating and bulimia, hypertension, diabetes, elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovarian disease, craniopharyngioma, the Prader-Willi Syndrome, Frohlich's syndrome, GH-deficient subjects, normal variant short stature, Turner's syndrome, and other pathological conditions showing reduced metabolic activity or a decrease in resting energy expenditure as a percentage of total fat-free mass, e.g, children with acute lymphoblastic leukemia. Further examples of obesity-related disorders are metabolic syndrome, also known as syndrome X, insulin resistance syndrome, sexual and reproductive dysfunction, such as infertility, hypogonadism in males and hirsutism in females, gastrointestinal motility disorders, such as obesity-related gastro-esophageal reflux, respiratory disorders, such as obesity-hypoventilation syndrome (Pickwickian syndrome), cardiovascular disorders, inflammation, such as systemic inflammation of the vasculature, arteriosclerosis, hypercholesterolemia, hyperuricaemia, lower back pain, gallbladder disease, gout, and kidney cancer. The compounds of the present invention are also useful for reducing the risk of secondary outcomes of obesity, such as reducing the risk of left ventricular hypertrophy.

The term "diabetes," as used herein, includes both insulin-dependent diabetes mellitus (IDDM, also known as type I diabetes) and non-insulin-dependent diabetes mellitus (NIDDM, also known as Type II diabetes). Type I diabetes, or insulin-dependent diabetes, is the result of an absolute deficiency of insulin, the hormone which regulates glucose utilization. Type II diabetes, or insulin-independent diabetes (i.e., non-insulin-dependent diabetes mellitus), often occurs in the face of normal, or even elevated levels of insulin and appears to be the result of the inability of tissues to respond appropriately to insulin. Most of the Type II diabetics are also obese. The compounds of the present invention are useful for treating both Type I and Type II diabetes. The compounds are especially effective for treating Type II diabetes. The compounds of the present invention are also useful for treating and/or preventing gestational diabetes mellitus.

The neuromedin U receptor agonists disclosed herein may be used in a pharmaceutical composition when combined with a pharmaceutically acceptable carrier. Such compositions comprise a therapeutically-effective amount of the neuromedin U receptor agonist and a pharmaceutically acceptable carrier. Such acomposition may also be comprised of (in addition to neuromedin U receptor agonist and a carrier) diluents, fillers, salts, buffers, stabilizers, solubilizers, and other materials well known in the art. Compositions comprising the neuromedin U receptor agonists can be administered, if desired, in the form of salts provided the salts are pharmaceutically acceptable. Salts may be prepared using standard procedures known to those skilled in the art of synthetic organic chemistry.

The term "individual" is meant to include humans and companion or domesticated animals such as dogs, cats, horses, and the like. Therefore, the compositions comprising formula I are also useful for treating or preventing obesity and obesity-related disorders in cats and dogs. As such, the term "mammal" includes companion animals such as cats and dogs.

The term "pharmaceutically acceptable salts" refers to salts prepared from pharmaceutically acceptable non-toxic bases or acids including inorganic or organic bases and inorganic or organic acids. Salts derived from inorganic bases include aluminium, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. The term "pharmaceutically acceptable salt" further includes all acceptable salts such as acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate (embonate), estolate, palmitate, esylate, pantothenate, fumarate, phosphate/diphosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, iodide, tosylate, isothionate, triethiodide, lactate, panoate, valerate, and the like which can be used as a dosage form for modifying the solubility or hydrolysis characteristics or can be used in sustained release or pro-drug formulation. It will be understood that, as used herein, references to the neuromedin U receptor agonists of the general formula (I) are meant to also include the pharmaceutically acceptable salts.

As utilized herein, the term "pharmaceutically acceptable" means a non-toxic material that does not interfere with the effectiveness of the biological activity of the active ingredient(s), approved by a regulatory agency of the Federal or a state government or listed in the U.S. Pharmacopoeia or other generally recognized pharmacopoeia for use in animals and, more particularly, in humans. The term "carrier" refers to a diluent, adjuvant, excipient, or vehicle with which the therapeutic is administered and includes, but is not limited to such sterile liquids as water and oils. The characteristics of the carrier will depend on the route of administration. The neuromedin U receptor agonist may be in multimers (for example, heterodimers or homodimers) or complexes with itself or other peptides. As a result, pharmaceutical compositions may comprise one ore more neuromedin U receptor agonists in such multimeric or complexed form.

As used herein, the term "therapeutically effective amount" means the total amount of each active component of the pharmaceutical composition or method that is sufficient to show a meaningful patient benefit, i.e., treatment, healing, prevention or amelioration of the relevant medical condition, or an increase in rate of treatment, healing, prevention or amelioration of such conditions. When applied to an individual active ingredient, administered alone, the term refers to that ingredient alone. When applied to a combination, the term refers to combined amounts of the active ingredients that result in the therapeutic effect, whether administered in combination, serially, or simultaneously.

The pharmacological composition can comprise one or more neuromedin U receptor agonists; one or more neuromedin U receptor agonists and one or more other agents for treating a metabolic disorder; or the pharmacological composition comprising the one or more neuromedin U receptor agonists can be used concurrently with a pharmacological composition comprising an agent for treating a metabolic disorder. Such disorders include, but are not limited to, obesity, metabolic syndrome or syndrome X, type II diabetes, complications of diabetes, hypertension, dyslipidemias, cardiovascular disease, gallstones, osteoarthritis, and certain forms of cancers.

When the pharmacological composition comprises another agent for treating a metabolic disorder or the treatment includes a second pharmacological composition comprising an agent for treating a metabolic disorder, the agent includes, but are not limited to, cannabinoid (CB1) receptor antagonists, glucagon like peptide 1 (GLP-1) receptor agonists, lipase inhibitors, leptin, tetrahydrolipstatin, 2-4-dinitrophenol, acarbose, sibutramine, phentamine, fat absorption blockers, simvastatin, mevastatin, ezetimibe, atorvastatin, sitagliptin, metformin, orlistat, Qnexa, topiramate, naltrexone, bupriopion, phentermine, losartan, losartan with hydrochlorothiazide, and the like.

Suitable agents of use in combination with a compound of the present invention, include, but are not limited to:
(a) anti-diabetic agents such as (1) PPAR-γ agonists such as glitazones (e.g. ciglitazone; darglitazone; englitazone; isaglitazone (MCC-555); pioglitazone (ACTOS); rosiglitazone (AVANDIA); troglitazone; rivoglitazone, BRL49653; CLX-0921; 5-BTZD, GW-0207, LG-100641, R483, and LY-300512, and the like and compounds disclosed in WO97/10813, 97/27857, 97/28115, 97/28137, 97/27847, 03/000685, and 03/027112 and SPPARMS (selective PPAR gamma modulators) such as T131 (Amgen), FK614 (Fujisawa), netoglitazone, and metaglidasen; (2) biguanides such as buformin; metformin; and phenformin, and the like; (3) protein tyrosine phosphatase-1B (PTP-1B) inhibitors such as ISIS 113715, A-401674, A-364504, IDD-3, IDD 2846, KP-40046, KR61639, MC52445, MC52453, C7, OC-060062, OC-86839, OC29796, TTP-277BC1, and those agents disclosed in WO 04/041799, 04/050646, 02/26707, 02/26743, 04/092146, 03/048140, 04/089918, 03/002569, 04/065387, 04/127570, and US 2004/167183; (4) sulfonylureas such as acetohexamide; chlorpropamide; diabinese; glibenclamide; glipizide; glyburide; glimepiride; gliclazide; glipentide; gliquidone; glisolamide; tolazamide; and tolbutamide, and the like; (5) meglitinides such as repaglinide, metiglinide (GLUFAST) and nateglinide, and the like; (6) alpha glucoside hydrolase inhibitors such as acarbose; adiposine; camiglibose; emiglitate; miglitol; voglibose; pradimicin-Q; salbostatin; CKD-711; MDL-25,637; MDL-73,945; and MOR 14, and the like; (7) alpha-amylase inhibitors such as tendamistat, trestatin, and Al-3688, and the like; (8) insulin secreatagogues such as linogliride nateglinide, mitiglinide (GLUFAST), ID1101 A-4166, and the like; (9) fatty acid oxidation inhibitors, such as clomoxir, and etomoxir, and the like; (10) A2 antagonists, such as midaglizole; isaglidole; deriglidole; idazoxan; earoxan; and fluparoxan, and the like; (11) insulin or insulin mimetics, such as biota, LP-100, novarapid, insulin detemir, insulin lispro, insulin glargine, insulin zinc suspension (lente and ultralente); Lys-Pro insulin, GLP-1 (17-36), GLP-1 (73-7) (insulintropin); GLP-1 (7-36)-NH2) exenatide/Exendin-4, Exenatide LAR, Linaglutide, AVE0010, CJC 1131, BIM51077, CS 872, THO318, BAY-6943-26, GP010, ALBUGON (GLP-1 fused to albumin), HGX-007(Epac agonist), S-23521, and compounds disclosed in WO 04/022004, WO 04/37859, and the like; (12) non-thiazolidinediones such as JT-501, and farglitazar (GW-2570/GI-262579), and the like; (13) PPARα/γdual agonists such as AVE 0847, CLX-0940, GW-1536, GW1929, GW-2433, KRP-297, L-796449, LBM 642, LR-90, LY510919, MK-0767, ONO 5129; SB 219994, TAK-559, Task-654, 677954 (GlaxoSmithkline), E-3030 (Eisai), LY510929 (Lilly), AK109 (Asahi), DRF2655 (Dr. Reddy), DRF8351 (Dr. Reddy), MC3002 (Maxocore), TY51501 (ToaEiyo), naveglitazar, muraglitizar, peliglitazar, tesaglitazar (GALIDA), reglitazar (JTT-501), chiglitazar, and those disclosed in WO 99/16758, WO 99/19313, WO 99/20614, WO 99/38850, WO 00/23415, WO 00/23417, WO 00/23445, WO 00/50414, WO 01/00579, WO 01/79150, WO 02/062799, WO 03/033481, WO 03/033450, WO 03/033453; and (14) other insulin sensitizing drugs; (15) VPAC2 receptor agonists; (16) GLK modulators, such as PSN105, RO 281675, RO 274375 and those disclosed in WO 03/015774, WO 03/000262, WO 03/055482, WO 04/046139, WO 04/045614, WO 04/063179, WO 04/063194, WO 04/050645, and the like; (17) retinoid modulators such as those disclosed in WO 03/000249; (18) GSK 3beta/GSK 3 inhibitors such as 4-[2-(2-bromophenyl)-4-(4-fluorophenyl-1H-imidazol-5-yl]pyridine, CT21022, CT20026, CT-98023, SB-216763, SB410111, SB-675236, CP-70949, XD4241 and those compounds disclosed in WO 03/037869, 03/03877, 03/037891, 03/024447, 05/000192, 05/019218 and the like; (19) glycogen phosphorylase (HGLPa) inhibitors, such as AVE 5688, PSN 357, GPi-879, those disclosed in WO 03/037864, WO 03/091213, WO 04/092158, WO 05/013975, WO 05/013981, US 2004/0220229, and JP 2004-196702, and the like; (20) ATP consumption promotors such as those disclosed in WO 03/007990; (21) fixed combinations of PPARγ agonists and metformin such as AVANDAMET; (22) PPAR pan agonists such as GSK 677954; (23) GPR40 (G-protein coupled receptor 40) also called SNORF 55 such as BG 700, and those disclosed in WO 04/041266, 04/022551, 03/099793; (24) GPR119 (also called RUP3; SNORF 25) such as RUP3, HGPRBMY26, PFI 007, SNORF 25; (25) adenosine receptor 2B antagonists such as ATL-618, ATI-802, E3080, and the like; (26) carnitine palmitoyl transferase inhibitors such as ST 1327, and ST 1326, and the like; (27) Fructose 1,6-bisphospohatase inhibitors such as CS-917, MB7803, and the like; (28) glucagon antagonists such as AT77077, BAY 694326, GW 4123X, NN2501, and those disclosed in WO 03/064404, WO 05/00781, US 2004/0209928, US 2004/029943, and the like; (30) glucose-6-phosphase inhibitors; (31) phosphoenolpyruvate carboxykinase (PEPCK) inhibitors; (32) pyruvate dehydrogenase kinase (PDK) activators; (33) RXR agonists such as Mac1036, CS00018, JNJ 10166806, and those disclosed in WO 04/089916, US 6759546, and the like; (34) SGLT inhibitors such as AVE 2268, KGT 1251, T1095/RWJ 394718; (35) BLX-1002;
(b) lipid lowering agents such as (1) bile acid sequestrants such as, cholestyramine, colesevelem, colestipol, dialkylaminoalkyl derivatives of a cross-linked dextran; Colestid®; LoCholest®; and Questran®, and the like; (2) HMG-CoA reductase inhibitors such as atorvastatin, itavastatin, pitavastatin, fluvastatin, lovastatin, pravastatin, rivastatin, rosuvastatin, simvastatin, rosuvastatin (ZD-4522), and the like, particularly simvastatin; (3) HMG-CoA synthase inhibitors; (4) cholesterol absorption inhibitors such as FMVP4 (Forbes Medi-Tech), KT6-971 (Kotobuki Pharmaceutical), FM-VA12 (Forbes Medi-Tech), FM-VP-24 (Forbes Medi-Tech), stanol esters, beta-sitosterol, sterol glycosides such as tiqueside; and azetidinones such as ezetimibe, and those disclosed in WO 04/005247 and the like; (5) acyl coenzyme A -cholesterol acyl transferase (ACAT) inhibitors such as avasimibe, eflucimibe, pactimibe (KY505), SMP 797 (Sumitomo), SM32504 (Sumitomo), and those disclosed in WO 03/091216, and the like; (6) CETP inhibitors such as JTT 705 (Japan Tobacco), torcetrapib, CP 532,632, BAY63-2149 (Bayer), SC 591, SC 795, and the like; (7) squalene synthetase inhibitors; (8) anti-oxidants such as probucol, and the like; (9) PPARα agonists such as beclofibrate, benzafibrate, ciprofibrate, clofibrate, etofibrate, fenofibrate, gemcabene, and gemfibrozil, GW 7647, BM 170744 (kowa), LY518674 (Lilly), GW590735 (GlaxoSmithkline), KRP-101 (Kyorin), DRF10945 (Dr. Reddy), NS-220/R1593 (Nippon Shinyaku/Roche, ST1929 (Sigma Tau) MC3001/MC3004 (MaxoCore Pharmaceuticals, gemcabene calcium, other fibric acid derivatives, such as Atromid®, Lopid®, and Tricor®, and those disclosed in US 6,548,538, and the like; (10) FXR receptor modulators such as GW 4064 (GlaxoSmithkline), SR 103912, QRX401, LN-6691 (Lion Bioscience), and those disclosed in WO 02/064125, WO 04/045511, and the like; (11) LXR receptor modulators such as GW 3965 (GlaxoSmithkline), T9013137, and XTCO179628 (X-Ceptor Therapeutics/Sanyo), and those disclosed in WO-03/031408, WO 03/063796, WO 04/072041, and the like; (12) lipoprotein synthesis inhibitors such as niacin; (13) renin angiotensin system inhibitors; (14) PPAR δ partial agonists, such as those disclosed in' WO 03/024395; (15) bile acid reabsorption inhibitors, such as BARI 1453, SC435, PHA384640, S8921, AZD7706, and the like; and bile acid sequesterants such as colesevelam (WELCHOL/ CHOLESTAGEL), (16) PPARγ agonists such as GW 501516 (Ligand, GSK), GW 590735, GW-0742 (GlaxoSmithkline), T659 (Amgen/Tularik), LY934 (Lilly), NNC610050 (Novo Nordisk) and those disclosed in WO97/28149, WO 01/79197, WO 02/14291, WO 02/46154, WO 02/46176, WO 02/076957, WO 03/016291, WO 03/033493, WO 03/035603, WO 03/072100, WO 03/097607, WO 04/005253, WO 04/007439, and JP10237049, and the like; (17) triglyceride synthesis inhibitors; (18) microsomal triglyceride transport (MTTP) inhibitors, such as implitapide, LAB687, JTT130 (Japan Tobacco), CP346086, and those disclosed in WO 03/072532, and the like; (19) transcription modulators; (20) squalene epoxidase inhibitors; (21) low density lipoprotein (LDL) receptor inducers; (22) platelet aggregation inhibitors; (23) 5-LO or FLAP inhibitors; and (24) niacin receptor agonists including HM74A receptor agonists; (25) PPAR modulators such as those disclosed in WO 01/25181, WO 01/79150, WO 02/79162, WO 02/081428, WO 03/016265, WO 03/033453; (26) niacin-bound chromium, as disclosed in WO 03/039535; (27) substituted acid derivatives disclosed in WO 03/040114; (28) infused HDL such as LUV/ETC-588 (Pfizer), APO-A1 Milano/ETC216 (Pfizer), ETC-642 (Pfizer), ISIS301012, D4F (Bruin Pharma), synthetic trimeric ApoA1, Bioral Apo A1 targeted to foam cells, and the like; (29) IBAT inhibitors such as BARI143/HMR145A/ HMR1453 (Sanofi-Aventis, PHA384640E (Pfizer), S8921 (Shionogi) AZD7806 (AstrZeneca), AK105 (Asah Kasei), and the like; (30) Lp-PLA2 inhibitors such as SB480848 (GlaxoSmitbkline), 659032 (GlaxoSmithkline), 677116 (GlaxoSmithkline), and the like; (31) other agents which affect lipic composition including ETC1001/ESP31015 (Pfizer), ESP-55016 (Pfizer), AGI1067 (AtheroGenics), AC3056 (Amylin), AZD4619 (AstrZeneca); and
(c) anti-hypertensive agents such as (1) diuretics, such as thiazides, including chlorthalidone, chlorthiazide, dichlorophenamide, hydroflumethiazide, indapamide, and hydrochlorothiazide; loop diuretics, such as bumetanide, ethacrynic acid, furosemide, and torsemide; potassium sparing agents, such as amiloride, and triamterene; and aldosterone antagonists, such as spironolactone, epirenone, and the like; (2) beta-adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol, and timolol, and the like; (3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, and verapamil, and the like; (4) angiotensin converting enzyme (ACE) inhibitors such as benazepril; captopril; cilazapril; delapril; enalapril; fosinopril; imidapril; losinopril; moexipril; quinapril; quinaprilat; ramipril; perindopril; perindropril; quanipril; spirapril; tenocapril; trandolapril, and zofenopril, and the like; (5) neutral endopeptidase inhibitors such as omapatrilat, cadoxatril and ecadotril, fosidotril, sampatrilat, AVE7688, ER4030, and the like; (6) endothelin antagonists such as tezosentan, A308165, and YM62899, and the like; (7) vasodilators such as hydralazine, clonidine, minoxidil, and nicotinyl alcohol, and the like; (8) angiotensin II receptor antagonists such as candesartan, eprosartan, irbesartan, losartan, pratosartan, tasosartan, telmisartan, valsartan, and EXP-3137, FI6828K, and RNH6270, and the like; (9) α/β adrenergic blockers as nipradilol, arotinolol and amosulalol, and the like; (10) alpha 1 blockers, such as terazosin, urapidil, prazosin, bunazosin, trimazosin, doxazosin, naftopidil, indoramin, WHIP 164, and XEN010, and the like; (11) alpha 2 agonists such as lofexidine, tiamenidine, moxonidine, rilmenidine and guanobenz, and the like; (12) aldosterone inhibitors, and the like; (13) angiopoietin-2-binding agents such as those disclosed in WO 03/030833; and
(d) anti-obesity agents, such as (1) 5HT (serotonin) transporter inhibitors, such as paroxetine, fluoxetine, fenfluramine, fluvoxamine, sertraline, and imipramine, and those disclosed in WO 03/00663, as well as serotonin/noradrenaline re uptake inhibitors such as sibutramine (MERIDIA/REDUCTIL) and dopamine uptake inbibitor/Norepenephrine uptake inhibitors such as radafaxine hydrochloride, 353162 (GlaxoSmithkline), and the like; (2) NE (norepinephrine) transporter inhibitors, such as GW 320659, despiramine, talsupram, and nomifensine; (3) CB1 (cannabinoid-1 receptor) antagonist/inverse agonists, such as rimonabant (ACCOMPLIA Sanofi Synthelabo), SR-147778 (Sanofi Synthelabo), AVE1625 (Sanofi-Aventis), BAY 65-2520 (Bayer), SLV 319 (Solvay), SLV326 (Solvay), CP945698 (Pfizer), E-6776 (Esteve), 01691 (Organix), ORG14481 (Organon), VER24343 (Vernalis), NESS0327 (Univ of Sassari/Univ of Cagliari), and those disclosed in US Patent Nos. 4,973,587, 5,013,837, 5,081,122, 5,112,820, 5,292,736, 5,532,237, 5,624,941, 6,028,084, and 6,509367; and WO 96/33159, WO97/29079, WO98/31227, WO 98/33765, WO98/37061, WO98/41519, WO98/4363 WO98/43636, WO99/02499, WO00/10967, WO00/10968, WO 01/09120, WO 01/58869, WO 01/64632, WO 01/64633, WO 01/64634, WO 01/70700, WO 01/96330, WO 02/076949, WO 03/006007, WO 03/007887, WO 03/020217, WO 03/026647, WO 03/026648, WO 03/027069, WO 03/027076, WO 03/027114, WO 03/037332, WO 03/040107, WO 04/096763, WO 04/111039, WO 04/111033, WO 04/111034, WO 04/111038, WO 04/013120, WO 05/000301, WO 05/016286, WO 05/066126 and EP-658546 and the like; (4) ghrelin agonists/antagonists, such as BVT81-97 (BioVitrum), RC1291 (Rejuvenon), SRD-04677 (Sumitomo), unacylated ghrelin (TheraTechnologies), and those disclosed in WO 01/87335, WO 02/08250, WO 05/012331, and the like; (5) H3 (histamine H3) antagonist/inverse agonists, such as thioperamide, 3-(1H-imidazol-4-yl)propyl N-(4-pentenyl)carbamate), clobenpropit, iodophenpropit, imoproxifan, GT2394 (Gliatech), and A331440, and those disclosed in WO 02/15945; and O-[3-(1H-imidazol-4-yl)propanol]carbamates (Kiec-Kononowicz, K.,et al., Pharmazie; 55:349-55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm.(Weinheim) 334:45-32 (2001)), substituted N-phenylcarbamates (Reidemeister, S. et al., Pharmazie, 55:83-6 (2000)), and proxifan derivatives (Sasse, A. et al., J. Med. Chem.. 43:3335-43 (2000)) and histamine H3 receptor modulators such as those disclosed in WO 03/024928 and WO 03/024929; (6) melanin-concentrating hormone 1 receptor (MCH1R) antagonists, such as T-226296 (Takeda), T71 (Takeda/Amgen), AMGN-608450, AMGN-503796 (Amgen), 856464 (GlaxoSmithkline), A224940 (Abbott), A798 (Abbott), ATC0175/AR224349 (Arena Pharmaceuticals), GW803430 (GlaxoSmithkine), NBI-1A (Neurocrine Biosciences), NGX-1 (Neurogen), SNP-7941 (Synaptic), SNA-P9847 (Synaptic), T-226293 (Schering Plough), TPI-1361-17 (Saitama Medical School/University of california Irvine), and those disclosed WO 01/21169, WO 01/82925, WO 01/87834, WO 02/051809, WO 02/06245, WO 02/076929, WO 02/076947, WO 02/04433, WO 02/51809, WO 02/083134, WO 02/094799, WO 03/004027, WO 03/13574, WO 03/15769, WO 03/028641, WO 03/035624, WO 03/033476, WO 03/033480, WO 04/004611, WO 04/004726, WO 04/011438, WO 04/028459, WO 04/034702, WO 04/039764, WO 04/052848, WO 04/087680; and Japanese Patent Application Nos. JP 13226269, JP 1437059, JP2004315511; and the like; (7) MCH2R (melanin concentrating hormone 2R) agonist/antagonists; (8) NPY1 (neuropeptide Y Y1) antagonists, such as BMS205749, BIBP3226, J-115814, BIBO 3304, LY-357897, CP-671906, and GI-264879A; and those disclosed in U.S. Patent No. 6,001,836; and WO 96/14307, WO 01/23387, WO 99/51600, WO 01/85690, WO 01/85098, WO 01/85173, and WO 01/89528; (9) NPY5 (neuropeptide Y Y5) antagonists, such as 152,804, S2367 (Shionogi), E-6999 (Esteve), GW-569180A, GW-594884A (GlaxoSmithkline), GW-387081X, GW-548118X; FR 236,208; FR226928, FR 240662, FR252384; 1229U91, GI-264879A, CGP71683A, C-75 (Fasgen) LY-377897, LY366377, PD-160170, SR-120562A, SR-120819A,S2367 (Shionogi), JCF-104, and H409/22; and those compounds disclosed in U.S. Patent Nos. 6,140,354, 6,191,160, 6,258,837, 6,313,298, 6,326,375, 6,329,395, 6,335,345, 6,337,332, 6,329,395, and 6,340,683 ; and EP-01010691, EP-01044970, and FR252384; and PCT Publication Nos. WO 97/19682, WO 97/20820, WO 97/20821, WO 97/20822, WO 97/20823, WO 98/27063, WO 00/107409, WO 00/185714, WO 00/185730, WO 00/64880, WO 00/68197, WO 00/69849, WO 01/09120, WO 01/14376, WO 01/85714, WO 01/85730, WO 01/07409, WO 01/02379, WO 01/02379, WO 01/23388, WO 01/23389, WO 01/44201, WO 01/62737, WO 01/62738, WO-01/09120, WO 02/20488, WO 02/22592, WO 02/48152, WO 02/49648, WO 02/051806, WO 02/094789, WO 03/009845, WO 03/014083, WO 03/022849, WO 03/028726, WO 05/014592, WO 05/01493; and Norman et al., J. Med. Chem. 43:4288-4312 (2000); (10) leptin, such as recombinant human leptin (PEG-OB, Hoffman La Roche) and recombinant methionyl human leptin (Amgen); (11) leptin derivatives, such as those disclosed in Patent Nos. 5,552,524; 5,552,523; 5,552,522; 5,521,283; and WO 96/23513; WO 96/23514; WO 96/23515; WO 96/23516; WO 96/23517; WO 96/23518; WO 96/23519; and WO 96/23520; (12) opioid antagonists, such as nalmefene (Revex ®), 3-methoxynaltrexone, naloxone, and naltrexone; and those disclosed in WO 00/21509; (13) orexin antagonists, such as SB-334867-A (GlaxoSmithkline); and those disclosed in WO 01/96302, 01/68609, 02/44172, 02/51232,02/51838, 02/089800, 02/090355, 03/023561, 03/032991, 03/03784730 04/004733, 04/026866, 04/041791, 04/085403, and the like; (14) BRS3 (bombesin receptor subtype 3) agonists; (15) CCK-A (cholecystokinin-A) agonists, such as AR-R 15849, GI 181771, JMV-180, A-71378, A-71623, PD170292, PD 149164, SR146131, SR125180, butabindide, and those disclosed in US 5,739,106; (16) CNTF (ciliary neurotrophic factors), such as GI-181771 (Glaxo-SmithKline); SR146131 (Sanofi Synthelabo); butabindide; and PD170,292, PD 149164 (Pfizer); (17) CNTF derivatives, such as axokine (Regeneron); and those disclosed in WO 94/09134, WO 98/22128, and WO 99/43813; (18) GHS (growth hormone secretagogue receptor) agonists, such as NN703, hexarelin, MK-0677, SM-130686, CP-424,391, L-692,429 and L-163,255, and those disclosed in U.S. Patent No. 6358951, U.S. Patent Application Nos. 2002/049196 and 2002/022637; and WO 01/56592, and WO 02/32888; (19) 5HT2c (serotonin receptor 2c) agonists, such as APD3546/AR10A (Arena Pharmaceuticals), ATH88651 (Athersys), ATH88740 (Athersys), BVT933 (Biovitrum/GSK), DPCA37215 (BMS), IK264; LY448100 (Lilly), PNU 22394; WAY 470 (Wyeth), WAY 629 (Wyeth), WAY161503 (Biovitrum), R-1065, VR1065 (Vernalis/Roche) YM 348; and those disclosed in U.S. Patent No. 3,914,250; and PCT Publications 01/616548, 02/36596,-02/48124, 02/10169, 02/44152; 02/51844, 02/40456, 02/40457, 03/057698, 05/000849, and the like; (20) Mc3r (melanocortin 3 receptor) agonists; (21) Mc4r (melanocortin 4 receptor) agonists, such as CHIR86036 (Chiron), CHIR915 (Chiron); ME-10142 (Melacure), ME-10145 (Melacure), HS-131 (Melacure), NBI72432 (Neurocrine Biosciences), NNC 70-619 (Novo Nordisk), TTP2435 (Transtech)and those disclosed in PCT Publications WO 99/64002, 00/74679, 01/991752, 01/0125192, 01/52880, 01/74844, 01/70708, 01/70337, 01/91752, 01/010842, 02/059095, 02/059107, 02/059108, 02/059117, 02/062766, 02/069095, 02/12166, 02/11715, 02/12178, 02/15909, 02/38544, 02/68387, 02/068388, 02/067869, 02/081430, 03/06604, 03/007949, 03/009847, 03/009850, 03/013509, 03/031410,03/094918, 04/028453, 04/048345, 04/050610, 04/075823, 04/083208, 04/089951, 05/000339, and EP 1460069, and US 2005049269, and JP2005042839, and the like; (22) monoamine reuptake inhibitors, such as sibutratmine (Meridia ®/Reductil®) and salts thereof, and those compounds disclosed in U.S. Patent Nos. 4,746,680, 4,806,570, and 5,436,272, and U.S. Patent Publication No. 2002/0006964, and WO 01/27068, and WO 01/62341; (23) serotonin reuptake inhibitors, such as dexfenfluramine, fluoxetrine, and those in U.S. Patent No. 6,365,633, and WO 01/27060, and WO 01/162341; (24) GLP-1 (glucagon-like peptide 1) agonists; (25) Topiramate (Topimax®); (26) phytopharm compound 57 (CP 644,673); (27) ACC2 (acetyl-CoA carboxylase-2) inhibitors; (28) β3 (beta adrenergic receptor 3) agonists, such as rafebergron/AD9677/TAK677 (Dainippon/ Takeda), CL-316,243, SB 418790, BRL-37344, L-796568, BMS-196085, BRL-35135A, CGP12177A, BTA-243, GRC1087 (Glenmark Pharmaceuticals) GW 427353 (solabegron hydrochloride), Trecadrine, Zeneca D7114, N-5984 (Nisshin Kyorin), LY-377604 (Lilly), KT07924 (Kissei), SR 59119A, and those disclosed in US Patent Nos. 5,705,515, US 5,451,677; and WO94/18161, WO95/29159, WO97/46556 W098/04526 WO98/32753, WO 01/74782, WO 02/32897, WO 03/014113, WO 03/016276, WO 03/016307, WO 03/024948, WO 03/024953, WO 03/037881, WO 04/108674, and the like; (29) DGAT1 (diacylglycerol acyltransferase 1) inhibitors; (30) DGAT2 (diacylglycerol acyltransferase 2)inhibitors; (31) FAS (fatty acid synthase) inhibitors, such as Cerulenin and C75; (32) PDE (phosphodiestetase) inhibitors, such as theophylline, pentoxifylline, zaprinast, sildenafil, amrinone, milrinone, cilostamide, rolipram, and cilomilast, as well as those described in WO 03/037432, WO 03/037899; (33) thyroid hormone β agonists, such as KB-2611 (KaroBioBMS), and those disclosed in WO 02/15845; and Japanese Patent Application No. JP 2000256190; (34) UCP-1 (uncoupling protein 1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), and retinoic acid; and those disclosed in WO 99/00123; (35) acyl-estrogens, such as oleoyl-estrone, disclosed in del Mar-Grasa, M. et al., Obesity Research, 9:202-9 (2001); (36) glucocorticoid receptor antagonists, such as CP472555 (Pfizer), KB 3305, and those disclosed in WO 04/000869, WO 04/075864, and the like; (37) 11β HSD-1 (11-beta hydroxy steroid dehydrogenase type 1) inhibitors, such as BVT 3498 (AMG 331), BVT 2733, 3-(1-adamantyl)-4-ethyl-5-(ethylthio)-4H-1,2,4-triazole, 3-(1-adamantyl)-5-(3,4,5-trimethoxyphenyl-4-methyl-4H-1,2,4-triazole, 3-adamantanyl-4,5,6,7;8,9,10,11,12,3a-decahydro-1,2,4-triazolo[4,3-a][11]annulene, and those compounds disclosed in WO 01/90091, 01/90090, 01/90092, 02/072084, 04/011410, 04/033427, 04/041264, 04/027047, 04/056744, 04/065351, 04/089415, 04/037251, and the like; (38) SCD-1 (stearoyl-CoA desaturase-1) inhibitors; (39) dipeptidyl peptidase IV (DPP-4) inhibitors, such as isoleucine thiazolidide, valine pyrrolidide, sitagliptin, saxagliptin, NVP-DPP728, LAF237 (vildagliptin), P93/01, TSL 225, TMC-2A/2B/2C, FE 999011, P9310/K364, VIP 0177, SDZ 274-444, GSK 823093, E 3024, SYR 322, TS021, SSR 162369, GRC 8200, K579, NN7201, CR 14023, PHX 1004, PHX 1149, PT-630, SK-0403; and the compounds disclosed in WO 02/083128, WO 02/052764, WO 02/14271, WO 03/000180, WO 03/000181, WO 03/000250, WO 03/002530, WO 03/002531, WO 03/002553, WO 03/002593, WO 03/004498, WO 03/004496, WO 03/005766, WO 03/017936, WO 03/024942, WO 03/024965, WO 03/033524, WO 03/055881, WO 03/057144, WO 03/037327, WO 04/041795, WO 04/071454, WO 04/0214870, WO 04/041273, WO 04/041820, WO 04/050658, WO 04/046106, WO 04/067509, WO 04/048532, WO 04/099185, WO 04/108730, WO 05/009956, WO 04/09806, WO 05/023762, US 2005/043292, and EP 1 258 476; (40) lipase inhibitors, such as tetrahydrolipstatin (orlistat/XENICAL), ATL962 (Alizyme/Takeda), GT389255 (Genzyme/Peptimmune)Triton WR1339, RHC80267, lipstatin, teasaponin, and diethylumbelliferyl phosphate, FL-386, WAY-121898, Bay-N-3176, valilactone, esteracin, ebelactone A, ebelactone B, and RHC 80267, and those disclosed in WO 01/77094, WO 04/111004, and U.S. Patent Nos. 4,598,089, 4,452,813, 5,512,565, 5,391,571, 5,602,151, 4,405,644, 4,189,438, and 4,242,453, and the like; (41) fatty acid transporter inhibitors; (42) dicarboxylate transporter inhibitors; (43) glucose transporter inhibitors; and (44) phosphate transporter inhibitors; (45) anorectic bicyclic compounds such as 1426 (Aventis) and 1954 (Aventis), and the compounds disclosed in WO 00/18749, WO 01/32638, WO 01/42746, WO 01/62747, and WO 03/015769; (46) peptide YY and PYY agonists such as PYY336 (Nastech/Merck), AC162352 (IC Innovations/Curis/Amylin), TM30335/TM30338 (7TM Pharma), PYY336 (Emisphere Tehcnologies), PEGylated peptide YY3-36, those disclosed in WO 03/026591, 04/089279, and the like; (47) lipid metabolism modulators such as maslinic acid, erythrodiol, ursolic acid uvaol, betulinic acid, betulin, and the like and compounds disclosed in WO 03/011267; (48) transcription factor modulators such as those disclosed in WO 03/026576; (49) Mc5r (melanocortin 5 receptor) modulators, such as those disclosed in WO 97/19952, WO 00/15826, WO 00/15790, US 20030092041, and the like; (50) Brain derived neutotropic factor (BDNF), (51) Mc1r (melanocortin 1 receptor modulators such as LK-184 (Proctor & Gamble), and the like; (52) 5HT6 antagonists such as BVT74316 (BioVitrum), BVT5182c (BioVitrum), E-6795 (Esteve), E-6814 (Esteve), SB399885 (GlaxoSmithkline), SB271046 (GlaxoSmithkline), RO-046790 (Roche), and the like; (53) fatty acid transport protein 4 (FATP4); (54) acetyl-CoA carboxylase (ACC) inhibitors such as CP640186, CP610431, CP640188 (Pfizer); (55) C-terminal growth hormone fragments such as AOD9604 (Monash Univ/Metabolic Pharmaceuticals), and the like; (56) oxyntomodulin; (57) (neuropeptide FF receptor antagonists such as those disclosed in WO 04/083218, and the like; (58) amylin agonists such as Symlin/pramlintide/AC137 (Amylin); (59) Hoodia and trichocaulon extracts; (60) BVT74713 and other gut lipid appetite suppressants; (61) dopamine agonists such as bupropion (WELLBUTRIN/GlaxoSmithkline); (62) zonisamide (ZONEGRAN/Dainippon/Elan), and the like.

Specific compounds that can be used in combination with the neuromedin U receptor agonists include specific CB1 antagonists/inverse agonists include those described in WO03/077847, including: *N*-[3-(4-chlorophenyl)-2(*S*)-phenyl-1(*S*)-methylpropyl]-2-(4-trifluoromethyl-2-pyrimidyloxy)-2-methylpropanamide, *N*-[3-(4-chlorophenyl)-2-(3-cyanophenyl)-1-methylpropyl]-2-(5-trifluoromethyl-2-pyridyloxy)-2-methylpropanamide, *N*-[3-(4-chlorophenyl)-2-(5-chloro-3-pyridyl)-1-methylpropyl]-2-(5-trifluoromethyl-2-pyridyloxy)-2-methylpropanamide, and pharmaceutically acceptable salts thereof; as well as those in WO05/000809, which includes the following: 3-{1-[bis(4-chlorophenyl)methyl]azetidin-3-ylidene}-3-(3,5-difluorophenyl)-2,2-dimethylpropanenitrile, 1-{1-[1-(4-chlorophenyl)pentyl]azetidin-3-yl}-1-(3,5-difluorophenyl)-2-methylpropan-2-ol. 3-((S)-(4-chlorophenyl) {3-[(1S)-1-(3,5-difluorophenyl)-2-hydroxy-2-methylpropyl]azetidin-1-yl}methyl)benzonitrile, 3-((S)-(4-chlorophenyl) {3-[(1S)-1-(3,5-difluorophenyl)-2-fluoro-2-methylpropyl]azetidin-1-yl}methyl)benzonitrile, 3-((4-chlorophenyl){3-[1-(3,5-difluorophenyl)-2,2-dimethylpropyl]azetidin-1-yl}methyl)benzonitrile, 3-((1S)-1-{1-[(S)-(3-cyanophenyl)(4-cyanophenyl)methyl]azetidin-3-yl}-2-fluoro-2-methylpropyl)-5-fiuorobenzonitrile, 3-[(S)-(4-chlorophenyl)(3-{(1S)-2-fluoro-1-[3-fluoro-5-(4H-1,2,4-triazol-4-yl)phenyl]-2-methylpropyl}azetidin-1-yl)methyl]benzonitrile, and 5-((4-chlorophenyl){3-[(1S)-1-(3,5-difluorophenyl)-2-fluoro-2-methylpropyl]azetidin-1-yl}methyl)thiophene-3-carbonitrile, and pharamecueitcally acceptable salts thereof; as well as: 3-[(*S*)-(4-chlorophenyl)(3-{(1*S*)-2-fluoro-1-[3-fluoro-5-(5-oxo-4,5-dihydro-1,3,4-oxadiazol-2-yl)phenyl]-2-methylpropyl} -azetidin-1-yl)methyl]benzonitrile, 3-[(*S*)-(4-chlorophenyl)(3-{(1*S*)-2-fluoro-1-13-fluoro-5-(1,3,4-oxadiazol-2-yl)phenyl]-2-methylpropyl}azetidin-1-yl)methyl]benzonitrile, 3-[(*S*)-(3-{(1*S*)-1-[3-(5-amino-1,3,4-oxadiazol-2-yl)-5-fluorophenyl]-2-fluoro-2-methylpropyl} azetidin-1-yl)(4-chlorophenyl)methyl]benzonitrile, 3-[(*S*)-(4-cyanophenyl)(3-{(1*S*)-2-fluoro-1-[3-fluoro-5-(5-oxo-4,5-dihydro-1,3,4-oxadiazol-2-yl)phenyl]-2-methylpropyl}azetidin-1-yl)methyl]benzonitrile, 3-[(5)-(3-{(1*S*)-1-[3-(5-amino-1,3,4-oxadiazol-2-yl)-5-fluorophenyl]-2-fluoro-2-methylpropyl} azetidin-1-yl)(4-cyanophenyl)methyl]benzonitrile, 3-[(*S*)-(4-cyanophenyl)(3-{(1*S*)-2-fluoro-1-[3-fluoro-5-(1,3,4-oxadiazol-2-yl)phenyl]-2-methylpropyl}azetidin-1-yl)methyl]benzonitrile, 3-[(*S*)-(4-chlorophenyl)(3-{(1*S*)-2-fluoro-1-[3-fluoro-5-(1,2,4-oxadiazol-3-yl)phenyl]-2-methylpropyl}azetidin-1-yl)methyl]benzonitrile, 3-[(1*S*)-1-(1-{(*S*)-(4-cyanophenyl)[3-(1,2,4-oxadiazol-3-yl)phenyl]-methyl}azetidin-3-yl)-2-fluoro-2-methylpropyl]-5-fluorobenzonitrile, 5-(3-{1-[1-(diphenylmethyl)azetidin-3-yl]-2-fluoro-2-methylpropyl}-5-fluorophenyl)-1*H*-tetrazole, 5-(3-{1-[1-(diphenylmethyl)azetidin-3-yl]-2-fluoro-2-methylpropyl}-5-fluorophenyl)-1-methyl-1*H*-tetrazole, 5-(3-{1-[1-(diphenylmethyl)azetidin-3-yl]-2-fluoro-2-methylpropyl}-5-fluorophenyl)-2-methyl-2H-tetrazole, 3-[(4-chlorophenyl)(3-{-fluoro-1-[3-fluoro-5-(2-methyl-2H-tetrazol-5-yl)phenyl]-2-methylpropyl}azetidin-1-yl)methyl]benzonitrile, 3-[(4-chlorophenyl)(3-{2-fluoro-1-[3-fluoro-5-(1-methyl-1*H*-tetrazol-5-yl)phenyl]-2-methylpropyl}azetidin-1-yl)methyl]benzonitrile, 3-[(4-cyanophenyl)(3-{2-fluoro-1-[3-fluoro-5-(1-methyl-1*H*-tetrazol-5-yl)phenyl]-2-methylpropyl}azetidin-1-yl)methyl]benzonitrile, 3-[(4-cyanophenyl)(3-{2-fluoro-1-[3-fluoro-5-(2-methylm-2*H*-tetrazol-5-yl)phenyl]-2-methylpropyl}azetidin-1-yl)methyl]benzonitrile, 5-{3-[(*S*)-{3-[(1*S*)-1-(3-bromo-5-fluorophenyl)-2-fluoro-2-methylpropyl]azetidin-1-yl}(4-chlorophenyl)methyl]phenyl} -1,3,4-oxadiazol-2(3*H*)-one, 3-[(1*S*)-1-(1-{(*S*)-(4-chlorophenyl)[3-(5-oxo-4,5-dihydro-1,3,4-oxadiazol-2-yl)phenyl]methyl} azetidin-3-yl)-2-fluoro-2-methylpropyl]-5-fluorobenzonitrile, 3-[(1*S*)-1-(1-{(*S*)-(4-cyanophenyl)[3-(5-oxo-4,5-dihydro-1,3,4-oxadiazol-2-yl)phenyl]methyl} azetidin-3-yl)-2-fluoro-2-methylpropyl]-5-fluorobenzonitrile, 3-[(1*S*)-1-(1-{(*S*)-(4-cyanophenyl)[3-(1,3,4-oxadiazol-2-yl)phenyl]methyl} azetidin-3-yl)-2-fluoro-2-methylpropyl]-5-fluorobenzonitrile, 3-[(1*S*)-1-(1-{(*S*)-(4-chlorophenyl)[3-(1,3,4-oxadiazol-2-yl)phenyl]methyl}azetidin-3-yl)-2-fluoro-2-methylpropyl]-5-fluorobenzonitrile, 3-((1*S*)-1-{1-[(*S*)-[3-(5-amino-1,3,4-oxadiol-2-yl)phenyl](4-chlorophenyl)methyl]azetidin-3-yl}-2-fluoro-2-methylpropyl)-5-fluorobenzonitrile, 3-((*S*)-1-{1-[(*S*)-[3-(5-amino-1,3,4-oxadiazol-2-yl)phenyl](4-cyanophenyl)methyl]azetidin-3-yl}-2-fluoro-2-methylpropyl)-5-fluorobenzonitrile, 3-[(1*S*)-1-(1-{(*S*)-(4-cyanophenyl)[3-(1,2,4-oxadiazol-3-yl)phenyl]methyl} azetidin-3-yl)-2-fluoro-2-methylpropyl]-5-fluorobenzonitrile, 3-[(1*S*)-1-(1-{(*S*)-(4-chlorophenyl)[3-(1,2,4-oxadiazol-3-yl)phenyl]methyl}azetidin-3-yl)-2-fluoro-2-methylpropyl]-5-fluorobenzonitrile,5-[3-((*S*)-(4-chlorophenyl){3-[(1*S*)-1-(3,5-difluorophenyl)-2-fluoro-2-methylpropyl] azetidin-1-yl}methyl)phenyl]-1,3,4-oxadiazol-2(3*H*)-one, 5-[3-((*S*)-(4-chlorophenyl){3-[(1*S*)-1-(3,5-difluorophenyl)-2-fluoro-2-methylpropyl]azetidin-1-yl}methyl)phenyl]-1,3,4-oxadiazol-2(3*H*)-one, 4-{(*S*)-{3-[(1*S*)-1-(3,5-difluorophenyl)-2-fluoro-2-methylpropyl]azetidin-1-yl} [3-(5-oxo-4,5-dihydro-1,3,4-oxadiazol-2-yl)phenyl]methyl}-benzonitrile, ACOMPLIA (rimonabant, *N-*(1-piperidinyl)-5-(4-chlorophenyl)-1-(2,4-dichlorophenyl)-4-methylpyrazole-3-carboxamide, SR141716A), 3-(4-chlorophenyl-N'-(4-chlorophenyl)sulfonyl-N-methyl4-phenyl-4,5-dihydro-1H-pyrazole-1-carboxamide(SLV-319), taranabant, *N*-[(1S,2S)-3-(4-Chlorophenyl)-2-(3-cyanophenyl)-1-methylpropyl]-2-methyl-2-[[5-(trifluoromethyl)-2-pyridinyl]oxy]propanamide, and pharmaceutically acceptable salts thereof.

Specific NPY5 antagonists that can be used in combination with the neuromedin U receptor agonists include: 3-oxo-N-(5-phenyl-2-pyrazinyl)-spiro[isobenzofuran-1(3H),4'-piperidine]-1'-carboxamide, 3-oxo-N-(7-trifluoromethylpyrido[3,2-b]pyridin-2-yl)spiro-[isobenzofuran-1(3H),4'-piperidine]-1'-carboxamide,N-[5-(3-fluorophenyl)-2-pyrimidinyl]-3-oxospiro-[isobenzofuran-1(3H),4'-piperidine]-1'-carboxamide, trans-3'-oxo-N-(5-phenyl-2-pyrimidinyl)spiro[cyclohexane-1,1'(3'H)-isobenzofuran]-4-carboxamide, trans-3'-oxo-N-[1-(3-quinolyl)-4-imidazolyl]spiro[cyclohexane-1,1'(3'H)-isobenzofuran]-4-carboxamide, trans-3-oxoN-(5-phenyl-2-pyrazinyl)spiro[4-azaiso-benzofuran- 1(3H),1'-cyclohexane]-4'-carboxamide, trans-N-[5-(3-fluorophenyl)-2-pyrimidinyl]-3-oxospiro[5-azaisobenzofuran-1(3H),1'-cyclohexane]-4'-carboxamide, trans-N-[5-(2-fluorophenyl)-2-pyrimidinyl]-3-oxospiro[5-azaisobenzofuran-1(3H),1'-cyclohexane]-4'-carboxamide, trans-N-[1-(3,5-difluorophenyl)-4-imidazolyl]-3-oxospiro[7-azaisobenzofuran-1(3H),1'-cyclohexane]-4'-carboxamide, trans-3-oxoN-(1-phenyl-4-pyrazolyl)spiro[4-azaisobenzofuran-1(3H),1'-cyclohexane]-4'-carboxamide, trans-N-[1-(2-fluoropbenyl)-3-pyrazolyl]-3-oxospiro[6-azaisobenzofuran-1(3H),1'-cyclohexane]-4'-carboxamide, trans-3-oxo-N-(1-phenyl-3-pyrazolyl)spiro[6-azaisobenzofuran-1-(3H),1'-cyclohexane]-4'-carboxamide, trans-3-oxo-N-(2-phenyl-1,2,3-triazol-4-yl)spiro[6-azaisobenzofuran-1(3H),1'-cyclohexane]-4'-carboxamide, and pharmaceutically acceptable salts and esters thereof.

Specific ACC-1/2 inhibitors that can be used in combination with the neuromedin U receptor agonists include: 1'-[(4,8-dimethoxyquinolin-2-yl)carbonyl]-6-(1*H*-tetrazol-5-yl)spiro[chroman-2,4'-piperidin]-4-one; (5-{1'-[(4,8-dimethoxyquinolin-2-yl)carbonyl]-4-oxospiro[chroman-2,4'-piperidin]-6-yl}-2*H*-tetrazol-2-yl)methyl pivalate; 5-{1'-[(8-cyclopropyl-4-methoxyquinolin-2-yl)carbonyl]-4-oxospiro[chroman-2,4'-piperidin]-6-yl}nicotinic acid; 1'-(8-methoxy-4-morpholin-4-yl-2-naphthoyl)-6-(1*H*-tetrazol-5-yl)spiro[chroman-2,4'-piperidin]-4-one; and 1'-[(4-ethoxy-8-ethylquinolin-2-yl)carbonyl]-6-(1*H*-tetrazol-5-yl)spiro[chroman-2,4'-piperidin]-4-one; and pharmaceutically acceptable salts and esters thereof. MK-3887, L-001738791.

Specific MCH1R antagonist compounds that can be used in combination with the neuromedin U receptor agonists include: 1-{4-[(1-ethylazetidin-3-yl)oxy]phenyl}-4-[(4-fluorobenzyl)oxy]pyridin-2(1*H*)-one, 4[(4-fluorobenzyl)oxy]-1-{4-[(1-isopropylazetidin-3-yl)oxy]phenyl}pyridin-2(1*H*)-one, 1-[4-(azetidin-3-yloxy)phenyl]-4-[(5-chloropyridin-2-yl)methoxy]pyridin-2(1*H*)-one, 4-[(5-chloropyzidin-2-yl)methoxy]-1-{4-[(1-ethylazetidin-3-yl)oxy]phenyl}pyridin-2(1*H*)-one, 4-[(5-chloropyridin-2-yl)methoxy]-1-{4-[(1-propylazetidin-3-yl)oxy]phenyl}pyridin-2(1*H*)-one, and 4[(5-chloropyridin-2-yl)methoxy]-1-(4-{[(2*S*)-1-ethylazetidin-2-yl]methoxy}phenyl)pyridin-2-(1*H*)-one, or a pharmaceutically acceptable salt thereof.

A specific DP-IV inhibitor that can be used in combination with the neuromedin U receptor agonists is 7-[(3R)-3-amino-4-(2,4,5-trifluorophenyl)butanoyl]-3-(trifluoromethyl)-5,6,7,8-tetrahydro-1,2,4-triazolo[4,3-a]pyrazine, or a pharmaceutically acceptable salt thereof.

Specific H3 (histamine H3) antagonists/inverse agonists that can be used in combination with the neuromedin U receptor agonists include: those described in WO05/077905 including:3-{4-[(1-cyclobutyl-4-piperidinyl)oxy]phenyl}-2-ethylpyrido[2,3-d]-pyrimidin-4(3H)-one, 3-{4-[(1-cyclobutyl-4-piperidinyl)oxy]phenyl}-2-methylpyrido[4,3-d]pyrimidin-4(3H)-one, 2-ethyl-3-(4-{3-[(3S)-3-methylpiperidin-1-yl]propoxy}phenyl)pyrido[2,3-d]pyrimidin-4(3H)-one 2-methyl-3-(4-{3-[(3S)-3-methylpiperidin-1-yl]propoxy}phenyl)pyrido[4,3-d]pyrimidin-4(3H)-one, 3-{4-[(1-cyclobutyl-4-piperidinyl)oxy]phenyl}-2,5-dimethyl-4(3H)-quinazolinone, 3-{4-[(1-cyclobutyl-4-piperidinyl)oxy]phenyl}-2-methyl-5-trifluoromethyl-4(3H)-quinazolinone, 3-{4-[(1-cyclobutyl-4-piperidinyl)oxy]phenyl}-5-methoxy-2-methyl-4(3H)-quinazolinone, 3-{4-[(1-cyclobutylpiperidin-4-yl)oxy]phenyl}-5-fluoro-2-methyl-4(3H)-quinazolinone, 3-{4-[(1-cyclobutylpiperidin-4-yl)oxy]phenyl}-7-fluoro-2-methyl-4(3H)-quinazolinone, 3-{4-[(1-cyclobutylpiperidin-4-yl)oxy]phenyl}-6-methoxy-2-methyl-4(3H)-quinazolinone, 3-{4-[(1-cyclobutylpiperidin-4-yl)oxy]phenyl}-6-fluoro-2-methyl-4(3H)-quinazolinone, 3-{4-[(1-cyclobutylpiperidin-4-yl)oxy]phenyl}-8-fluoro-2-methyl-4(3H)-quinazolinone, 3-{4-[(1-cyclopentyl-4-piperidinyl)oxy]phenyl}-2-methylpyrido[4,3-d]pyrimidin-4(3H)-one, 3-{4-[(1-cyclobutylpiperidin-4-yl)oxy]phenyl}-6-fluoro-2-methylpyrido[3,4-d]pyrimidin-4(3H)-one, 3-{4-[(1-cyclobutyl-4-piperidinyl)oxy]phenyl}-2-ethylpyrido[4,3-d]pyrimidin-4(3H)-one, 6-methoxy-2-methyl-3-{4-[3-(1-piperidinyl)propoxy]phenyl}pyrido[3,4-d]pyrimidin-4(3H)-one, 6-methoxy-2-methyl-3-{4-[3-(1-pyrrolidinyl)propoxy]phenyl}pyrido[3,4-d]pyrimidin-4(3H)-one,2,5-dimethyl-3- {4-[3-(1-pyrrolidinyl)propoxy]phenyl} -4(3H)-quinazolinone, 2-methyl-3-{4-[3-(1-pyrrolidinyl)propoxy]phenyl}-5-trifluoromethyl-4(3H)-quinazolinone, 5-fluoro-2-methyl-3-{4-[3-(1-piperidinyl)propoxy]phenyl}-4(3H)-quinazolinone, 6-methoxy-2-methyl-3-{4-[3-(1-piperidinyl)propoxy]phenyl}-4(3H)-quinazolinone, 5-methoxy-2-methyl-3-(4-{3-[(3S)-3-methylpiperidin-1-yl]propoxy}phenyl)-4(3H)-quinazolinone, 7-methoxy-2-methyl-3-(4-{3-[(3S)-3-methylpiperidin-1-yl]propoxy}phenyl)-4(3H)-quinazolinone, 2-methyl-3-(4-{3-[(3S)-3-methylpiperidin-1-yl]propoxy}phenyl)pyrido[2,3-d]pyrimidin-4(3H)-one, 5-fluoro-2-methyl-3-(4-{3-[(2R)-2-methylpyrrolidin-1-yl]propoxy}phenyl)-4(3H)-quinazolinone, 2-methyl-3-(4-{3-[(2R)-2-methylpyrrolidin-1-yl]propoxy}phenyl)pyrido[4,3-d]pyrimidin-4(3H)-one, 6-methoxy-2-methyl-3-(4-{3-[(2R)-2-methylpyrrolidin-1-yl]propoxy}phenyl)-4(3H)-quinazominone, 6-methoxy-2-methyl-3-(4-{3-[(2S)-2-methylpyrrolidin-1-yl]propoxy}phenyl)-4(3H)-quinazolinone, and pharmaceutically acceptable salts thereof.

Specific CCK1R agonists of use in combination with the neuromedin U receptor agonists include: 3-(4-{[1-(3-ethoxyphenyl)-2-(4-methylphenyl)-1*H*-imidazol-4-yl]carbonyl}-1-piperazinyl)-1-naphthoic acid; 3-(4-{[1-(3-ethoxyphenyl)-2-(2-fluoro-4-methylphenyl)-1*H-*imidazol-4-yl]carbonyl}-1-piperazinyl)-1-naphthoic acid; 3-(4-{[1-(3-ethoxyphenyl)-2-(4-fluorophenyl)-1*H*-imidazol-4-yl]carbonyl}-1-piperazinyl)-1-naphthoic acid; 3-(4-{[1-(3-ethoxyphenyl)-2-(2,4-difluorophenyl)-1*H*-imidazol-4-yl]carbonyl}-1-piperazinyl)-1-naphthoic acid; and 3-(4-{[1-(2,3-dihydro-1,4-benzodioxin-6-yl)-2-(4-fluorophenyl)-1-imidazol-4-yl]carbonyl}-1-piperazinyl)-1-naphthoic acid; and pharmaceutically acceptable salts thereof. MK-8406

Specific MC4R agonists of use in combination with the neuromedin U receptor agonists include: 1) (5,*S*)-1'-{[(3*R*,4*R*)-1-*tert*-butyl-3-(2,3,4-trifluorophenyl)piperidin-4-yl]carbonyl}-3-chloro-2-methyl-5-[1-methyl-1-(1-methyl-1*H*-1,2,4-triazol-5-yl)ethyl]-5*H-*spiro[furo[3,4-*b*]pyridine-7,4'-piperidine]; 2) (5*R*)-1'-{[(3*R*,4*R*)-1-*tert*-butyl-3-(2,3,4-trifluorophenyl)-piperidin-4-yl]carbonyl}-3-chloro-2-methyl-5-[1-methyl-1-(1-methyl-1*H*-1,2,4-triazol-5-yl)ethyl]-5*H*-spiro[furo[3,4*-b*]pyridine-7,4'-piperidine]; 3) 2-(1'-{[(3*S*,4*R*)-1-*tert*-butyl-4-(2,4-difluorophenyl)pyrrolidin-3-yl]carbonyl}-3-chloro-2-methyl-5*H*-spiro[furo[3,4-b]pyridine-7,4'-piperidin]-5-yl)-2-methylpropanenitrile; 4) 1'-{[(3*S*,4*R*)-1-*tert*-butyl-4-(2,4-difluorophenyl)pyrrolidin-3-yl]carbonyl}-3-chloro-2-methyl-5-[1-methyl-1-(1-methyl-1*H*-1,2,4-triazol-5-yl)ethyl]-5*H*-spiro[furo[3,4*-b*]pyridine-7,4'-piperidine] ; 5) *N-*[(3*R*,4*R*)-3-({3-chloro-2-methyl-5-[1-methyl-1-(1-methyl-1*H*-1,2,4-triazol-5-yl)ethyl]-1'*H*,5*H*-spiro[furo-[3,4*-b*]pyridine-7,4'-piperidin]-1'-yl} carbonyl)-4-(2,4-difluorophenyl)-cyclopentyl]-*N-*methyltetrahydro-2*H-*pyran-4-amine; 6) 2-[3-chloro-1'-({(1*R*,2*R*)-2-(2,4-difluorophenyl)-4-[methyl(tetrahydro-2*H* pyran-4-yl)amino]-cyclopentyl}-carbonyl)-2-methyl-5*H*-spiro[furo[3,4*-b*]pyridine-7,4'-piperidin]-5-yl]-2-methyl-propane-nitrile; and pharmaceutically acceptable salts thereof.

Additionally, other peptide analogs and mimetics of the incretin hormone glucagon-like peptide 1 (GLP-1), may also be of use in combination with the neuromedin U receptor agonists.

Methods of administrating the pharmacological compositions comprising the one or more neuromedin U receptor agonists to an individual include, but are not limited to, intradermal, intramuscular, intraperitoneal, intravenous, subcutaneous, intranasal, epidural, and oral routes. The compositions can be administered by any convenient route, for example by infusion or bolus injection, by absorption through epithelial or mucocutaneous linings (for example, oral mucosa, rectal and intestinal mucosa, and the like), ocular, and the like and-can be administered together with other biologically-active agents. Administration can be systemic or local. In addition, it may be advantageous to administer the composition into the central nervous system by any suitable route, including intraventricular and intrathecal injection. Intraventricular injection may be facilitated by an intraventricular catheter attached to a reservoir (for example, an Ommaya reservoir). Pulmonary administration may also be employed by use of an inhaler or nebulizer, and formulation with an aerosolizing agent. It may also be desirable to administer the one or more neuromedin U receptor agonists locally to the area in need of treatment; this may be achieved by, for example, and not by way of limitation, local infusion during surgery, topical application, by injection, by means of a catheter, by means of a suppository, or by means of an implant.

Various delivery systems are known and can be used to administer the neuromedin U receptor agonists including, but not limited to, encapsulation in liposomes, microparticles, microcapsules; minicells; polymers; capsules; tablets; and the like. In one embodiment, the neuromedin U receptor agonist may be delivered in a vesicle, in particular a liposome. In a liposome, the neuromedin U receptor agonist is combined, in addition to other pharmaceutically acceptable carriers, with amphipathic agents such as lipids which exist in aggregated form as micelles, insoluble monolayers, liquid crystals, or lamellar layers in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Patent No. 4,837,028 and U.S. Patent No. 4,737,323. In yet another embodiment, the neuromedin U receptor agonist can be delivered in a controlled release system including, but not limited to: a delivery pump (*See,* for example, Saudek, et al., New Engl. J.Med. 321: 574(1989) and a semi-permeable polymeric material (*See*, for example, Howard, et al., J. Neurosurg. 71: 105 (1989)). Additionally, the controlled release system can be placed in proximity of the therapeutic target (for example, the brain), thus requiring only a fraction of the systemic dose. *See,* for example, Goodson, In: Medical Applications of Controlled Release, 1984. (CRC Press, Bocca Raton, Fla.).

The amount of the compositions comprising the neuromedin U receptor agonist which will be effective in the treatment of a particular disorder or condition will depend on the nature of the disorder or condition, and may be determined by standard clinical techniques by those of average skill within the art. In addition, *in vitro* assays may optionally be employed to help identify optimal dosage ranges. The precise dose to be employed in the formulation will also depend on the route of administration, and the overall seriousness of the disease or disorder, and should be decided according to the judgment of the practitioner and each patient's circumstances. Ultimately, the attending physician will decide the amount of the composition with which to treat each individual patient. Initially, the attending physician will administer low doses of the composition and observe the patient's response. Larger doses of the composition may be administered until the optimal therapeutic effect is obtained for the patient, and at that point the dosage is not increased further. In general, the daily dose range lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases. However, suitable dosage ranges for intravenous administration of the compositions comprising the neuromedin U receptor agonist are generally about 5-500 micrograms (µg) of active compound per kilogram (Kg) body weight. Suitable dosage ranges for intranasal administration are generally about 0.01 pg/kg body weight to 1 mg/kg body weight. Effective doses may be extrapolated from dose-response curves derived from in vitro or animal model test systems. Suppositories generally contain active ingredient in the range of 0.5% to 10% by weight; oral formulations preferably contain 10% to 95% active ingredient. Ultimately the attending physician will decide on the appropriate duration of therapy using compositions comprising the neuromedin U receptor agonist of the present invention. Dosage will also vary according to the age, weight and response of the individual patient.

Further provided is a pharmaceutical pack or kit, comprising one or more containers filled with one or more of the ingredients of the pharmaceutical compositions and neuromedin U receptor agonists. Optionally associated with such container(s) may be a notice in the form prescribed by a governmental agency regulating the manufacture, use or sale of pharmaceuticals or biological products, which notice reflects approval by the agency of manufacture, use or sale for human administration.

The following examples are intended to promote a further understanding of the present invention.

### EXAMPLE 1

### Generation of human or rodent NMUR1- or NMUR2-expressing cell lines

Human, mouse or rat cDNAs encoding NMUR1 or NMUR2 (as described in Howard et al. Nature 406: 70-74 (2000) were subcloned in pcDNA5 (Invitrogen) and transfected into FLP-In CHO cells and HEK-293 FLP-In cells purchased from Invitrogen (Carlsbad,CA) using lipofectamine (Invitrogen). The Flp-In system allows integration and expression of a particular gene of interest at a specific genomic location utilizing the Flp recombinase from yeast. The transfected cells were selected by growth in medium containing 200 µg/mL hygromycin (Invitrogen). Populations were frozen at early passage numbers, and these stocks were used for further studies. Stable clones that expressed the mRNAs were identified functionally by FLIPR as well as by RT-PCR. Based on public genomic databases, the rodent NMUR1 receptors do not appear to have the traditional methionine (ATG) as the start codon for translation but contain an alternate start codon (TTG for rat and CTG for mouse). Two different rodent NMUR1 cell lines were thus generated: one with the codon as predicted from the genomic databases and another cell line with an engineered methionine (ATG) as the start codon. Additionally, stable cell lines expressing the human receptors were generated in HEK-293/aeq17 cells which stably express the aequorin gene under the CMV promoter. The human NMUR2 cDNA was cloned into pCDNA3.1 and human NMUR1 was subcloned into pIRES-puro (Clontech, Mountain View, CA), after transfection cells were selected in media containing G418 and either hygromycin (NMUR2) or puromycin (NMUR1).

### EXAMPLE 2

### In vitro functional assays

The NMU receptors signal primarily through Gαq/11 proteins; therefore calcium mobilization assays can be utilized for functional activity.

### FLIPR assay

Stable cell lines expressing human and or rodent NMUR1 or human NMUR2 receptors were plated at a density of 12,000 cells per well overnight on poly-lysine coated 384-well black-walled plates. The following day, the media was removed from the plates and the cells were subsequently loaded with Fluo-3 (Molecular Probes), a calcium sensitive dye, diluted in FLIPR buffer (1X Hank's buffered saline containing 20 mM HEPES, 0.1 % BSA, 2.5 mM probenecid (Sigma) and 1.6 mM TR40). All reagents are from Invitrogen unless otherwise noted. Peptide stocks were resuspended in DMSO at a stock concentration of 2 mM and diluted in FLIPR buffer on the day of the experiment to a 4 µM working stock solution.

After a 90 minute incubation at room-temperature, cell plates were loaded onto a FLIPR (Molecular Devices) to monitor cellular fluorescence (excitation = 488 nM; emission = 540 nM) before and after compound/peptide addition. Eight to twelve point dose responses were tested on NMUR-expressing cell lines using FLIPR with 1 µM peptide as the highest dose. The response after peptide addition was taken as the maximum fluorescence units minus the fluorescence immediately prior to stimulation for each well. EC₅₀ values were calculated using GraphPad Prism (San Diego, CA) software.

*In vitro* responses of Neuromedin U receptor agonists (NMU1 to NMU25) in the FLIPR assay for human (Table 3), mouse (Table 4) and rat (Table 5) NMUR1 and NMUR2. EC₅₀s are reported in nM values. Percent activity refers to the maximum responses at 1 µM compared to the hNMU-25 response at the same concentration. NT = not tested. The tables show that the majority of the neuromedin U receptor agonists are bisepcific and will bind both the NMUR1 and NMU2 receptors.

| Table 3 | | | | |
|---|---|---|---|---|
| Agonist | human NMUR1 | | human NMUR2 | |
| | EC₅₀ | % Activity | EC₅₀ | % Activity |
| NMU1 | 81.4 | 83.7 | 64.8 | 85.0 |
| NMU2 | 5.5 | 99.0 | 2.6 | 97.4 |
| NMU3 | 5.6 | 98.9 | 1.9 | 100 |
| NMU4 | 7.2 | 97.0 | 2.6 | 100 |
| NMU5 | 6.1 | 98.1 | 2.5 | 98.1 |
| NMU6 | 3.5 | 96.7 | 1.5 | 100 |
| NMU7 | 79.6 | 91.4 | 36.1 | 91.8 |
| NMU8 | 10.7 | 99.1 | 5.2 | 100 |
| NMU9 | 50.6 | 94.4 | 91.1 | 94.3 |
| NMU10 | 1000 | 26.6 | 1000 | 31.3 |
| NMU11, | 1000 | 20.6 | 1000 | 37.3 |
| NMU12 | 58.6 | 92.9 | 119.0 | 89.0 |
| NMU13 | 4.8 | 100 | >1000 | 0.0 |
| NMU14 | 103.9 | 95.7 | >1000 | 0.0 |
| NMU15 | 7.7 | 91.5 | 4.2 | 101 |
| NMU16 | 1000 | 74.0 | 1000 | 75.0 |
| NMU17, | 5.0 | 100 | 1.5 | 99.8 |
| NMU18 | 68.1 | 97.1 | 65.695 | 101 |
| NMU19 | 38.9 | 95.9 | 11.5 | 105 |
| NMU20 | 16.4 | 100 | 13.001 | 100 |
| NMU21 | 14.5 | 93.2 | 24.2 | 97.9 |
| NMU22 | 10.2 | 95.6 | 6.5 | 105 |
| NMU23 | 300.2 | 66.9 | 1000 | 71.7 |
| NMU24 | 1000 | 34.4 | 1000 | 63.9 |
| NMU25 | 167.7 | 83.0 | 44.1 | 98.8 |

| Table 4 | | | | | | |
|---|---|---|---|---|---|---|
| | mouse NHUR1-ATG | | Mouse NMUR1-CTG | | mouse NMUR2 | |
| Agonist | EC₅₀ | % Activity | EC₅₀ | % Activity | EC₅₀ | % Activity |
| NMU1 | NT | | NT | | 31.0 | 95.7 |
| NMU2 | 2.1 | 100 | 8.3 | 100 | 1.2 | 100 |
| NMU3 | 2.9 | 100 | 4.7 | 100 | 1.0 | 100 |
| NMU4 | 3.6 | 100 | 11.6 | 100 | 1.2 | 100 |
| NMU5 | 3.3 | 100 | 11.9 | 100 | 1.0 | 100 |
| NMU6 | 1.0 | 100 | 2.0 | 100 | 0.7 | 100 |
| NMU8 | 6.4 | 100 | 15.5 | 100 | 1.8 | 100 |
| NMU9 | 45.7 | 95.0 | 1000 | 71.7 | 19.0 | 100 |
| NMU10 | 172.6 | 76.8 | 1000 | 69.2 | 1000 | 70.5 |
| NMU11 | 1000 | 81.3 | 600.5 | 65.0 | 1000 | 70.5 |
| NMU12 | 72.0 | 98.4 | 407.2 | 68.5 | 19.1 | 94.1 |
| NMU13 | 35.8 | 98.6 | 141.5 | 73.6 | >1000 | 0.0 |
| NMU14 | 584.3 | 60.8 | >1000 | 24.8 | >1000 | 0.0 |
| NMU15 | 1.5 | 100 | 4.0 | 100 | 0.6 | 100 |
| NMU16 | 24.1 | 100 | 651.2. | 57.2 | 137.5 | 97.4 |
| NMU17 | 1.2 | 100 | 2.9 | 116.6 | 0.5 | 100 |
| NMU18 | 15.2 | 100 | 692.9 | 57.7 | 50.2 | 100 |
| NMU19 | 7.9 | 100 | 22.5 | 110.8 | 5.6 | 100 |
| NMU20 | 12.7 | 100 | 480.4 | 65.4 | 14.8 | 100 |
| NMU21 | 9.1 | 100 | 56.3 | 90.8 | 1.6 | 100 |
| NMU22 | 6.2 | 100 | 43.5 | 100 | 6.9 | 100 |
| NMU23 | 14.0 | 100 | 770.5 | 50.9 | 21.3 | 100 |
| NMU24 | 1000 | 81.4 | 591.3 | 56.1 | 1000 | 82.4 |
| NMU25 | 10.2 | 100 | 60.3 | 100.0 | 18.8 | 100 |

| Table 5 | | | | | | |
|---|---|---|---|---|---|---|
| | rat NMUR1-ATG | | rat NMUR1-TTG | | rat NMUR2 | |
| Agonist | EC50 | % Activity | EC₅₀ | % Activity | EC₅₀ | % Activity |
| NMU1 | 718.0 | 59.3 | NT | | 71.7 | 87.1 |
| NMU2 | 3.3 | 89.2 | NT | | 2.2 | 100 |
| NMU3 | 0.7 | 100 | NT | | 1.8 | 100 |
| NMU4 | 3.8 | 100 | NT | | 2.9 | 100 |
| NMU5 | 3.6 | 100 | NT | | 2.3 | 100 |
| NMU6 | 1.0 | 100 | NT | | 1.5 | 100 |
| NMU8 | 36.7 | 97.2 | NT | | 45.1 | 94.7 |
| NMU9 | 9.7 | 100 | NT | | 4.5 | 100 |
| NMU10 | 201.4 | 80.1 | NT | | 41.5 | 97.4 |
| NMU11 | 315.6 | 50.9 | NT | | 1000 | 33.2 |
| NMU12 | 1000 | 52.7 | NT | | 1000 | 43.4 |
| NMU14 | 239.5 | 75.7 | NT | | 54.3 | 97.0 |
| NMU13 | 9.5 | 100 | 114.7 | 94.6 | >1000 | 0.0 |
| NMU14 | 132.9 | 87.7 | >1000 | 36.7 | >1000 | 0.0 |
| NMU15 | 4.2 | 100 | 24.6 | 107.5 | 2.2 | 100 |
| NMU16 | 417.6 | 75.4 | 1000 | 34.6 | 314.7 | 86.9 |
| NMU17 | 0.9 | 100 | 4.9 | 105.3 | 1.2 | 100 |
| NMU18 | 28.6 | 95.9 | 105 | 90.7 | 63.0 | 96.7 |
| NMU19 | 6.8 | 100 | 12.8 | 100 | 8.7 | 100 |
| NM20 | 7.2 | 100 | 24.4 | 93.8 | 16.9 | 100 |
| NMU21 | 19.5 | 99.9 | 220.4 | 77.5 | 7.0 | 99.2 |
| NMU22 | 14.0 | 100 | 39.1 | 96.4 | 17.1 | 98.8 |
| NMU23 | 127.4 | 76.4 | 1000 | 39.8 | 47.4 | 90.2 |
| NMU24 | 236.4 | 80.8 | 221.8 | 67.7 | 986.4 | 52.2 |
| NMU25 | 20.1 | 99.6 | 81.0 | 93.5 | 37.8 | 96.4 |

### Aequorin

In addition to FLIPR, NMU receptor function can also be evaluated using an aequorin assay. Stable cell lines expressing the aequorin jelly fish gene can be used to report the activation of GPCRs by monitoring intracellular calcium mobilization. The objective is to identify compounds which specifically stimulate aequorin bioluminescence. Calcium-dependent luminescence is generated by the treatment of cells with the coelenterate luciferin, coelenterazine. Briefly, confluent monolayers of HEK-293/aeq 17 cells expressing hNMUR1 or hNMUR2 are "charged" with coelenterazine (Molecular Probes, Carlsbad, CA). Confluent T75 flasks are rinsed with media containing 300 µM glutathione and 0.1 % FBS. Cells are incubated at 37°C for one hour in 8 mL media, 0.1% FBS, 300 µM glutathione, and 20 µM coelenterazine. T75 flasks are subsequently rinsed with 6 mL ECB buffer (140 mM NaCl, 20 mM KCl, 20 mM HEPES, 5 mM glucose, 1 mM MgCl, 1 mM CaCl₂, 0.1 mg/mL BSA, PH 7.3-7.4). Cells are removed from the flask in ECB buffer, pelleted, and resuspended at a density of 2X10⁵ cells/mL. Agonists are added to the cells and activity is determined using a luminometer.

### IP1 Assay

In addition to direct measurements ofcalcium, NMU receptor activity can be determined by measurements of myo-inositol 1 phosphate (IP1), one of the major products of the phosphatidyl inositol cascade, which tightly correlates with Gq-coupled activity. An assay kit (IPOne) from Cisbio (Bedford, MA) is available that uses HTRF (homogeneous time resolved fluorescence) to measure IP1 levels. The assay follows the manufacturer's directions. Briefly, the cells are plated overnight at a density of 30,000 cells per well in 384-well white walled plates. The next day media is removed from the cells, and 10 uL agonist is added which is diluted in stimulation buffer (10mM HEPES, 1 mM CaCl₂, 0.5 mM MgCl₂, 4,2 mM KCL, 146 mM NaCl, 5.5 mM glucose, 50 mM L1Cl, pH 7.4). Cells are incubated for 1 hour at 37°C with agonist. Detection molecules are added, IP1-d2 conjugate and anti-IP1 cryptate (prepared per manufacturer's protocol), and cells are incubated at 1 hour at room temperature. Fluorescence is measured on an Envision machine and the results are calculated from the fluorescence ratios from the instrument readout.

### Alternate measurements of NMU receptor activity

Additional data suggest that NMU receptor signaling can occur via Gαi-coupled activity. Activation of either hNMUR1 or hNMUR2 has shown to result in the inhibition of forskolin (10uM)-stimulated cAMP accumulation. To measure Gi-coupled signaling, the inhibition of forskolin induced cAMP can be measured. Briefly, cells are plated 24 hours prior to running the experiment. Neuromedin U receptor agonist is added to the cells and incubated for 10 minutes, followed by an addition of 10uM forskolin. After a 10 minute incubation, the cAMP is extracted from the cells and measured by a radioreceptor assay. Basal levels of cAMP and forskolin stimulated levels of cAMP are measured with and without agonist treatment.

A summary of functional and binding data for NMUR1 subtype selective neuromedin U receptor agonists specific for NMUR1 across species is shown in Tables 6 and 7. Calculated percent activity is relative to native hNMU responses.

| Table 6 | | | | |
|---|---|---|---|---|
| *In vitro* responses to NMUR1 selective peptide H | | | | |
| Species/ Receptor | IC₅₀ (nM) | % activity | IC₃₀ (nM) | % inhibition of NMU-25 binding |
| human R1 | 1.25 (0.2) | 90% | 1.1 (0.14) | 93% |
| human R2 | >1000 (0.8) | 0 | 782 (0.056) | 7% |
| | | | | |
| mouse R1-ATG | 25 (0.9) | 90% | 66 (0.4) | 77% |
| mouse R2 | >1000 (0.2) | 40% | >1000 (0.2) | 20% |
| mouse R1-CTG | 66 (2.9) | 77 | 25 (0.15) | 83 |
| | | | | |
| rat R1-ATG | 31.1 (1.3) | 95% | 15.9 (1.2) | 100% |
| rat R2 | >1000 (1.6) | 42% | >1000 (0.6) | 0 |
| rat R1-TTG | 152.8 (1.0) | 91% | 11 (0.2) | 100% |

| | | | | |
|---|---|---|---|---|
| ( ) indicates values for human NMU-25 with 100% activity | | | | |

| Table 7 | | | | |
|---|---|---|---|---|
| In vitro responses to NMUR1 selective peptide NMU13 | | | | |
| Species/ Receptor | IC₅₀ (nM) | % activity | IC₅₀ (nM) | % inhibition of NMU-25 binding |
| human R1 | 1.7 (0.2) | 91% | | 100% |
| human R2 | >1000 (0.8) | 0 | >1000 (0.056) | 48% |
| | | | | |
| mouse R1-ATG | 7.8 (0.9) | 96% | 5.4 (0.4) | 100% |
| mouse R2 | >1000 (0.2) | 27% | >1000 (0.2) | 30% |
| mouse R1-CTG | 14 (2.9) | 88% | 3.5 (0.15) | 100% |
| | | | | |
| rat R1-ATG | 15.8 (1.3) | 92% | 1.6 (1.2) | 100% |
| rat R2 | >1000 (1.6) | 24% | >1000 (0.6) | 0 |
| rat R1-TTG | 107.4(1.0) | 94% | 3.24 (0.2) | 100% |

| | | | | |
|---|---|---|---|---|
| ( ) indicates values for human NMU-25 with 100% activity | | | | |

### EXAMPLE 3

### Binding Assays

### Membrane preparation:

Confluent cell monolayers expressing NMU receptors (the HEK cells described above) were harvested with phosphate buffered saline, collected by centrifugation, and resuspended in membrane buffer (50 mM TrisCl pH 7.4, 5 mM MgCl₂, 1X Protease Inhibitor Cocktail, 10 µM phosphoramidon). After the cell pellet was homogenized, the solution was centrifuged at 18,000 rpm for 20 minutes at 4°C. The pellet was resuspended in membrane buffer to yield a final concentration of 0.5-5 µg/µL of membrane and stored at -80°C.

### ¹²⁵I-hNMU-25 binding assay:

Experiments were performed in assay buffer (25 mM TrisCl, pH 7.4, 10 mM MgCl₂, 2mM EDTA, 1X Protease Inhibitor Cocktail, 100 µg/mL Bacitricin, 10 µM phosphoramidon) in 200 µL volumes in a 96-well format using 2-5 µg of membrane and 0.1 nM ¹²⁵I-hNMU-25 (about 12,000 cpm/well). For non-specific binding, 1 µM hNMU-25 was added. About 5 µL ofpeptides were added to measure antagonist activity, and the entire reaction was incubated at room temperature with shaking for 80 minutes. The reaction was terminated by rapid filtration through 0.3% poly-ethylenimine presoaked Millipore 96-well filter plates and washed with ice-cold buffer (5 mM TrisCl pH 7.4, 10 mM MgCl₂, 2.5 mM EDTA, 0.04% Triton X-100). Plates were air-dried overnight at room temperature and recovered radioactivity was determined by standard scintillation counting. IC₅₀ values were determined using GraphPad Prism software.

### Data analysis:

Concentration-response curves and radioligand-binding data were fitted using Prism (GraphPad Software). Results are summarized below in Tables 8 and 9.

| Table 8 | | | | | | | | |
|---|---|---|---|---|---|---|---|---|
| | HEK-hR1 | | | | HEK-hR2 | | | |
| Agonist | IC₅₀ (nM) | % activity | IC₅₀ (nM) | % activity | IC₅₀ (nM) | % activity | IC₅₀ (nM) | % activity |
| A | 2.7 | 92 | 10 | 94 | >1000 | 0 | >1000 | 0 |
| B | 9 | 103 | 14 | 96 | >1000 | 0 | >1000 | 35 |
| C | 1.1 | 87 | 0.42 | 100 | >1000 | 0 | >1000 | 0 |
| D | 1.5 | 96 | 2.6 | 100 | >1000 | 0 | >1000 | 0 |
| E | 1.9 | 100 | 75 | 93 | >1000 | 188 | >1000 | 5 |
| F | 42 | 96 | >1000 | 30 | 870 | 96 | >1000 | 12 |
| G | 8.4 | 91 | 2.1 | 97 | >1000 | 14 | 31 | 33 |
| H | 19 | 88 | 1.1 | 93 | >1000 | 0 | 780 | 7 |

| Table 9 | | | | | | | | |
|---|---|---|---|---|---|---|---|---|
| | HEK-hR1 | | | | HEK-hR2 | | | |
| Agonist | EC50 | % | IC50 | % | EC50 | % | IC50 | % |
| | (nM) | activity | (nM) | activity | (nM) | activity | (nM) | activity |
| I | >1000 | 7 | >1000 | 23 | 10 | 99 | 9.1 | 90 |
| J | >1000 | 0 | >10000 | 34 | 160 | 59 | 150 | 63 |
| K | 400 | 61 | >1000 | 25 | 2 | 100 | 13 | 100 |
| L | >1000 | 74 | >1000 | 0 | 6.2 | 100 | 37 | 100 |
| M | >1000 | 0 | >1000 | 0 | 33 | 93 | 180 | 71 |
| N | 53 | 87 | >1000 | 38.4 | 3.9 | 100 | 22 | 100 |
| O | 420 | 35 | 400 | 64 | 16 | 67 | 3 | 92 |
| P | 46 | 11 | >1000 | 1 | 25 | 100 | 150 | 81 |
| Q | >1000 | 0 | >1000 | 16 | 18.3 | 97 | 12 | 89 |
| NMU-25 | 5.5 | 100 | 0.47 | 100 | 1.5 | 100 | 0.19 | 100 |
| NMU-8 | 1.36 | 100 | 2.4 | 100 | 2.9 | 104 | 0.3 | 100 |

### EXAMPLE 4

### Synthesis of Neuromedin U receptor agonists

Neuromedin U receptor agonists can be produced using techniques well known in the art. For example, a polypeptide region of a truncated NMU analog can be chemically or biochemically synthesized and, if desired, modified to produce a blocked N-terminus and/or blocked C terminus. Techniques for chemical synthesis of polypeptides are well known in the art. (*See* for example., Vincent, in Peptide and Protein Drug Delivery, New York, N.Y., Dekker, 1990) Examples of techniques for biochemical synthesis involving the introduction of a nucleic acid into a cell and expression of nucleic acids are provided in Ausubel, Current Protocols in Molecular Biology, John Wiley,1987-1998, and Sambrook, et al., in Molecular Cloning, A Laboratory Manual, 2nd Ed., Cold Spring Harbor Laboratory Press, 1989.

### 1.1 Amino Acid Substitutions and Modifications

The neuromedin U receptor agonist NMU1 is a PEGylated peptide in which a branched PEG of 40kDa is linked at the N-terminus of the native human neuromedin U peptide. The N-terminal group of the peptide was acylated with a branched (PEG)₂40K N-hydroxysuccinimide analog (for example, mPEG2-NHS-40k; Nektar, San Carlos, CA; Cat# 2Z3Y0T01). This was designed to create a neuromedin U receptor agonist with improved pharmacological profile. The peptide precursor, the wild type sequence of NMU, was reacted with an N-hydroxysuccinimide derivative of a branched PEG of 40kDa. PEGylation with this reagent occurs specifically at the N-terminal amino group of the peptide, as this is the only available amino group in the peptide.

The neuromedin U receptor agonist NMU2 is the native NMU peptide that is acetylated at the N-terminus. It was designed to study the impact of acetylation, or more generally, acylation at the N-terminus on activity. Based on the minimal active sequence spanning residues 19-25, the peptide sequences were modified at these residues 17-25 by adding an additional acetylated cysteine residue at the N-terminus. The cysteine thiolated group was further derivatized with (a) N-ethylmaleimide to obtain NMU6, a control peptide for the conjugation; (b) (PEG)₂ 40kDa to obtain NMU7, a PEGylated analog designed to have an improved *in vivo* pharmacological profile; or (c) a cholesterol group to obtain NMU11, a lipidated analog designed to have an improved *in vivo* pharmacological profile.

Other peptide sequences were designed starting from the native NMU sequence and adding an acetylated cysteine residue at the N-terminus. For example, a cysteine thiolated group of was derivatized with (a) N-ethylinaleimide to obtain NMU8, a control peptide for the conjugation; (b) (PEG)₂40kDa from Nektar (mPEG₂-MAL-40k, Cat# 2D3Y0T01) to obtain NMU9, or (PEG)₂40kDa from NOF Corporation, Japan (SUNBRIGHT 4GL2-400MA) to obtain NMU12; (c) a cholesterol group to obtain NMU10; (d) (PEG)20kDa to obtain NMU21; (e) (PEG)₂40kDa to obtain NMU26; or (f) (PEG)40kDa to obtain NMU27. All these neuromedin U receptor agonists were designed to have an improved *in vivo* pharmacological profile.

In other peptide examples, the native NMU-25 amino acid residue at position 20 was changed to D-alanine and the amino acid residue at position 21 to tryptophan. These mutations confer added selectivity for the NMUR1 receptor and have an additional acetylated cysteine residue at the N-terminus. The cysteine thiolated group was derivatized with (a) N-ethylmaleimide to obtain NMU13, a control peptide; or (b) (PEG)₂40kDa to obtain NMU14, a PEGylated analog designed to have an improved *in vivo* pharmacological profile.

Peptides NMU 15 and 16 were designed to obtain a PEGylated peptide based on the minimal active sequence comprising amino acid residues 19-25. The sequences were modified by introduction at the N-terminus of a Ttds group (1-amino-4,7,10-trioxa-13-tridecanamine succinic acid) as a spacer and an acetylated cysteine residue. The spacer was introduced to minimize the impact on activity on the minimalist sequence due to the addition of the PEG moiety. The cysteine thiolated group was derivatized with (a) N-ethylmaleimide to obtain NMU15, a control peptide for conjugation; or (b) (PEG)₂40kDa to obtain NMU16, a PEGylated analog designed to have an improved *in vivo* pharmacological profile.

Peptides NMU 17 and 18 were designed to obtain PEGylated peptides based on the N-terminally truncated sequence 12-25. The peptides have an additional acetylated cysteine residue at the N-terminus. The cysteine thiolated group was derivatized with (a) N-ethylmaleimide to obtain NMU17, a control peptide for conjugation; or (b) (PEG)₂40kDa to obtain NMU18, a PEGylated analog designed to have an improved *in vivo* pharmacological profile.

Peptides NMU 19 and 20 were designed to obtain a PEGylated peptide based on the N-terminally truncated sequence 7-25. The peptides have an additional acetylated cysteine residue at the N-terminus. The cysteine thiolated group was derivatized with (a) N-ethylmaleimide to obtain NMU19, a control peptide for conjugation; or (b) (PEG)₂40kDa to obtain NMU20, a PEGylated analog designed to have an improved *in vivo* pharmacological profile.

Peptides NMU 22 and 23 were designed starting from the native NMU sequence and adding two cysteine residues at the N-terminus. The N-terminus of the peptides was acetylated. The cysteine thiolated groups were derivatized with (a) N-ethylmaleimide to obtain NMU22, a control peptide for conjugation; or (b) (PEG)20kDa to obtain NMU23, a PEGylated analog designed to have an improved *in vivo* pharmacological profile.

Peptides NMU 24 and NMU 25 are based on the native NMU sequence to which a palmitoylated cysteine residue at the N-terminus was added. They were designed to study the impact on activity of (1) N-terminal palmitoylation, or more generally acylation with a fatty acid chain; and (2) the combined effect of both PEGylation and lipidation. The cysteine thiolated group was derivatized with (a) N-ethylmaleimide to obtain NMU24, a lipidated analog designed to have an improved *in vivo* pharmacological profile and also to act as a control peptide for conjugation; or (b) (PEG)₂40kDa to obtain NMU25, this lipidated and PEGylated analog was designed to have an improved *in vivo* pharmacological profile.

Peptides NMU 28 and 29 span residues 17-25 of the native NMU. An acetylated cysteine residue was added the N-terminus as well as a Ttds group to act as a spacer. The spacer was introduced to minimize the impact on activity on the sequence due to the addition of the PEG moiety. The cysteine thiolated group was derivatized with (a) N-ethylmaleimide to obtain NMU28, a control peptide for the conjugation; or (b) (PEG)₂40kDa to obtain NMU29, a PEGylated analog designed to have an improved *in vivo* pharmacological profile.

### 1.2. PEGylation and/or Cholesteroylation

The sites of PEGylation on the neuromedin U receptor agonists of the present invention were chosen taking into account the structure of NMU and its interactions with the NMU receptors. Hence, the PEGylation is preferably site-specific. PEGylation at the N-terminal amino group of the peptide NMU is possible since this is the only available amino group in the sequence. For instance the N-terminal group of the peptide was acylated with a branched ((PEG)₂40kDa N-hydroxysuccinimide analog (for example, mPEG2-NHS-40k, Nektar, Cat# 2Z3Y0T01).

### 1.3. Synthesis of Neuromedin U receptor Agonists

The neuromedin U receptor agonist (see Table 1) were synthesized by solid phase using Fmoc/tBu chemistry on a peptide synthesizer ABI433A (Applied Biosystems). For each peptide 0.75 g of a resin Fmoc-Linker AM-Champion, 1% cross-linked (Biosearch Technologies, Inc., Novato, CA) and a PEG-PS based resin derivatized with a modified Rink linker p-[(R,S)-α-[9H-Fluoren-9-yl-methoxyformamido]-2,4-dimethoxybenzyl]-phenoxyacetic acid (Rink, Tetrahedron Lett. 28: 3787-3789 (1987); Bernatowicz, et al., Tetrahedron Lett. 30: 4645-4667 (1989)) was used. The acylation reactions were performed for 60 minutes with four-fold excess of activated amino acid over the resin free amino groups. The amino acids were activated with equimolar amounts of HBTU (2-(1H-benzottiazole-1-yl)-1,1,3,3-tetramethyluronium hexafluorophosphate) and a 2-fold molar excess of DIEA (N,N-diisopropylethylamine) in DMF.

Alternatively, the peptides were synthesized by solid phase using Fmoc/t-Bu chemistry on a Pioneer Peptide Synthesizer (Applied Biosystems). In this case, all the acylation reactions were performed for 60 minutes with a four-fold excess of activated amino acid over the resin free amino groups following the end of peptide assembly on the synthesizer. The side chain protecting groups were: tert-butyl for Asp, Glu, Ser and Tyr; trityl for Asn, Cys and Gln; 2,2,4,6,7-pentamethyldihydrobenzofuran-5-sulfonyl for Arg. The N-terminal acetylation reaction was performed at the end of the peptide assembly by reaction with a 10-fold excess of acetic anhydride in DMF. The N-terminal palmitoylation reaction (for NMU24 and NMU25) was performed at the end of the peptide assembly by reaction with a four-fold excess of activated palmitic acid over the resin free amino groups. The palmitic acid was activated with equimolar amounts of DIPC (1,3 Diisopropylcarbodiimide) and HOBt (Hydroxybenzotriazole) in DMF.

At the end of the synthesis, the dry peptide-resins were individually treated with 20 mL of the cleavage mixture, 88% TFA, 5% phenol, 2% triisapropylsilane and 5% water (Sole and Barany, J. Org. Chem. 57: 5399-5403 (1992)) for 2.5 hours at room temperature. Each resin was filtered and the solution was added to cold methyl-t-butyl ether in order to precipitate the peptide. After centrifugation, the peptide pellets were washed with fresh cold methyl-t-butyl ether to remove the organic scavengers. The process was repeated twice. Final pellets were dried, resuspended in H₂O, 20% acetonitrile, and lyophilized.

The crude peptides were purified by reverse-phase HPLC using semi-preparative Waters RCM Delta-Pak™ C₄ or C₁₈ cartridges (40 x 200 mm,15 µm) and using as eluents (A) 0.1 % TFA in water and (B) 0.1% TUFA in acetonitrile, flow rate 80 mL/min. Analytical HPLC was performed on a Phenomenex, Jupiter C₄ column (150 x 4.6 mm, 5 µm) or ReproSil-Pur 300 C₄ column (150 x 4.6 mm, 5 µm) (Dr. Maisch GmbH) or Beckman, Ultrasphere C₁₈ column (250 x 4.6 mm, 5 µm), flow rate one mL/min. The purified peptide was characterized by electrospray mass spectrometry on a Micromass LCZ platform.

The synthesis of peptide NMU6 was performed by dissolving the thiol containing NMU peptide precursor in HEPES 0.1M pH 7.3, EDTA 4mM. A 1.5 molar excess of N-ethylmaleimide was added. After one hour incubation, the peptide was purified by HPLC.

The synthesis of peptide NMU8 was performed by dissolving the thiol containing NMU peptide precursor in HEPES 0.1M pH 7.3, EDTA 4mM. A 1.5 molar excess of N-ethylmaleimide was added. After one hour incubation, the peptide was purified by HPLC.

The synthesis of peptide NMU13 was performed by dissolving the thiol containing NMU peptide precursor in HEPES 0.1M pH 7.3, urea 8M, EDTA 4mM. A 1.5 molar excess of N-ethylmaleimide was added. After one hour incubation, the peptide was purified by HPLC.

The synthesis ofpeptide NMU15 was performed by dissolving the thiol containing NMU peptide precursor in HEPES 0.1M pH 7.3, EDTA 4mM. A 1.5 molar excess of N-ethylmaleimide was added. After one hour incubation, the peptide was purified by HPLC.

The synthesis of peptide NMU17 was performed by dissolving the thiol containing NMU peptide precursor in HEPES 0.1M pH 7.3, EDTA 4mM. A 1.5 molar excess of N-ethylmaleimide was added. After one hour incubation, the peptide was purified by HPLC.

The synthesis of peptide NMU19 was performed by dissolving the thiol containing NMU peptide precursor in sodium phosphate 0.1M pH 6.5, urea 4M, EDTA 4mM. A 1.5 molar excess of N-ethylmaleimide was added. After one hour incubation, the peptide was purified by HPLC.

The synthesis of peptide NMU22 was performed by dissolving the thiol containing NMU peptide precursor in sodium phosphate 0.2M pH 6.5, urea 8M, EDTA 4mM. A three molar excess of N-ethylmaleimide was added. After one hour incubation, the peptide was purified by HPLC.

The synthesis of peptide NMU24 was performed by dissolving the thiol containing NMU peptide precursor in sodium phosphate 0.2M pH 6.5, urea 8M, EDTA 4mM. A three molar excess of N-ethylmaleimide was added. After one hour incubation, the peptide was purified by HPLC.

The synthesis of peptide NMU28 was performed by dissolving the thiol containing NMU peptide precursor in HEPES 0.1M pH 7.3, EDTA 4mM. A 1.5 molar excess of N-ethylmaleimide was added. A fter one hour incubation, the peptide was purified by HPLC.

### 1.4. PEGylation of Neuromedin U (NMU) analogs

PEGylation reactions were run under conditions permitting amide bond (NMU1) or thioether bond formation. The PEGylated NMU peptides were then isolated using cation exchange chromatography (IXC) and size exclusion chromatography (SEC). Cation exchange chromatography (IXC) was carried out on TSK SP-5PW (Tosoh) column (16 x 100 mm) with a linear gradient of NaCl (0-1M) in 3.5 column volumes in formic acid 0.2%, flow rate loading one mL/min, gradient elution 2 mL/min. Size exclusion chromatography (SEC) was carried out on TSK-HW50 (Tosoh) column (21 x 700 mm) in acetic acid 0.1% (w/v), 30% acetonitrile, flow rate one mL/min. PEGylated NMU analogs were characterized using RP-HPLC, HPLC-SEC and MALDI-Tof Mass Spectrometry.

NMU1 peptide was synthesized from the native NMU peptide precursor to produce a derivative with PEG covalently attached via an amide bond.

### Synthesis of NMU1

8.7 mg of peptide precursor (2.8 µmoles) were dissolved in 1.5 mL of 0.2 M HEPES, pH 7.3. Then 360 mg of mPEG2-NHS-4k (NEKTAR, 2Z3Y0t01) (8.6 µmoles) dissolved in 3.5 mL water (1:3 mole/mole ratio of peptide to PEG) was added to this solution. After 18 hours incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

NMU7, NMU9, NMU12, NMU14, NMU16, NMU18, NMU20,NMU21, NMU23, NMU25, NMU26, NMU27 and NMU29 peptides were synthesized from the thiol-containing NMU peptide precursors to produce derivatives with PEG covalently attached via a thioether bond.

### Synthesis of NMU7

10 mg of peptide precursor (7.6 µmoles) were dissolved in 1 mL of 0.2 M HEPES, pH 7.3,4 mM EDTA. Then 340 mg of mPEG2-MAL-40k (NEKTAR, 2D3Y0T01) (8.4 µmoles) dissolved in three mL water (1:1.1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU9

15 mg of peptide precursor (4.6 µmoles) were dissolved in 1 mL of 0.2 M sodium phosphate, pH 6.5, urea 8M, 4 mM EDTA. Then 204 mg of mPEG2-MAL-40k (NEKTAR, 2D3Y0T01) (5.1 µmoles) dissolved in two mL water (1:1.1 mole/mole ratio of peptide to PEG) was added to this solution. After one hour incubation, the PEGylated peptide solution was acidified to 1 % formic acid and purified by cation exchange chromatography (IXC). The IXC purified PEGylated-peptide was further purified by SEC and characterized by RP-HPLC and MALDI-Tof.

### Synthesis of NMU12

8 mg of peptide precursor (2.5 µmoles) were dissolved in 1 mL of 0.1 M HEPES, pH 7.3, urea 8M, 4 mM EDTA. Then 115 mg of SUNBRIGHT GL2-400MA (NOF Corp.) (2.7 µmoles) dissolved in two mL water (1:1.1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU14

10 mg of peptide precursor (3.1 µmoles) were dissolved in 1 mL of 0.1 M HEPES, pH 7.3, urea 8M, 4 mM EDTA. Then 145 mg of SUNBRlGHT GL2-400MA (NOF Corp.) (3.4 µmoles) dissolved in two mL water (1:1.1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU16

8.3 mg of peptide precursor (6.0 µmoles) were dissolved in 1 mL of 0.1 M HEPES, pH 7.3, 4 mM EDTA. Then 280 mg of SUNBRIGHT GL2-400MA (NOF Corp.) (6.6 µmoles) dissolved in two mL water (1:1.1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU18

10 mg of peptide precursor (5:4 µmoles) were dissolved in 1 mL of 0.1 M HEPES, pH 7.3,4 mM EDTA. Then 250 mg of SUNBRIGHT GL2-400MA (NOF Corp.) (5.9 µmoles) dissolved in two mL water (1:1.1 mole/mole ratio ofpeptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU20

10 mg of peptide precursor (4.1 µmoles) were dissolved in 1 mL of 0.1 M sodium phosphate, pH 7.3, urea 4M, 4 mM EDTA. Then 174 mg of SUNBRIGHT GL2-400MA (NOF Corp.) (4.1 µmoles) dissolved in two mL water (1:1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU21

10 mg of peptide precursor (3.1 µmoles) were dissolved in 1 mL of 90 mM sodium phosphate, pH 6.6, urea 4M, 4 M EDTA. Then 65 mg of SUNBRIGHT ME-200MA (NOF Corp.) (3.1 µmoles) dissolved in one mL water (1:1 mole/mole ratio ofpeptide to PEG) was added to this solution. After one hour incubation, the PEGylated peptide-solution was acidified to 1% formic acid and purified by cation exchange chromatography (IXC). The IXC purified PEGylated-peptide was further purified by SEC and characterized by RP-HPLC and MALDI-Tof.

### Synthesis of NMU23

10 mg of peptide precursor (3.0 µmoles) were dissolved in 1 mL of 90 mM sodium phosphate, pH 7.1, urea 8M, 4 mM EDTA. Then, 157 mg of SUNBRIGHT ME-200MA (NOF Corp.) (7.5 µmoles) dissolved in two mL water (1:2.5 mole/mole ratio ofpeptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALTI-Tof.

### Synthesis of NMU25

10 mg of peptide precursor (2.9 µmoles) were dissolved in 1 mL of 90mM sodium phosphate, pH 7.1, urea 8M, 4 mM EDTA. Then, 125 mg of SUNBRIGHT GL2-400MA (NOF Corp.) (2.9 µmoles) dissolved in two mL urea 8M (1:1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU26

10 mg ofpeptide precursor (3.1 µmoles) were dissolved in 1 mL of 90 mM sodium phosphate, pH 7.1, urea 8M, 4 mM EDTA. Then 70 mg of SUNBRIGHT GL2-200MA (NOF Corp.) (3.1 µmoles) dissolved in two mL water (1:1 mole/mole ratio ofpeptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU27

10 mg of peptide precursor (3.1 µmoles) were dissolved in 1 mL of 90 mM sodium phosphate, pH 7.1, urea 8M, 4 mM EDTA. Then 136 mg of mPEG-maleimide-40kDa (DOWpharma, 008-016) (3.4 µmoles) dissolved in two mL water (1:1.1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof.

### Synthesis of NMU29

8 mg of peptide precursor (5.1 µmoles) were dissolved in 1 mL of 0.1 M HEPES, pH 7.3, 4 mM EDTA. Then 217 mg of SUNBRIGHT GL2-400MA (NOF Corp.) (5.1 µmoles) dissolved in two mL water (1:1 mole/mole ratio of peptide to PEG) was added to this solution. After one hour incubation, the PEGylated peptide solution was acidified to 1% formic acid and purified by cation exchange chromatography (IXC). The IXC purified PEGylated-peptide was further purified by SEC and characterized by RP-HPLC and MALDI-Tof

### 1.5. Cholesteroylation of Neuromedin U (NMU) analogs

Derivatizations with cholesterol were run under conditions permitting thioether bond formation. The cholesteroylated neuromedin U receptor agonists were then purified by RP-HPLC and characterized by electrospray mass spectrometry.

### Synthesis of NMU10

18 mg of peptide precursor (5.6 µmoles) were dissolved in 1 mL DMF. Then three mg of cholesteryl bromoacetate (14 µmoles), dissolved in 70 µL THF (1:1.1 mole/mole ratio of peptide to cholesteryl bromoacetate), and 5 µL of DIEA (N,N-diisopropylethylamine) (2.5-fold molar excess over the peptide) were added to this solution. After one hour incubation, the cholesteroylated peptide was purified by RP-HPLC and characterized by electrospray mass spectrometry.

### Synthesis of NMU11

17 mg of peptide precursor (12.9 µmoles) were dissolved in 1 mL DMF. Then 7.2 mg of cholesteryl bromoacetate (14 µmoles), dissolved in 0.17 mL THF (1:1.1 mole/mole ratio of peptide to cholesteryl bromoacetate), and 11.7 µL of DIEA (5-fold molar excess over the peptide) were added to this solution. After one hour incubation, the cholesteroylated peptide was purified by RP-HPLC and characterized by electrospray mass spectrometry.

### EXAMPLE 5

### Feeding study with NMU and analogs thereof

NMUR1 knockout *(Nmur1-*/*-)* mice were generated using standard homologous recombination techniques. Nmur1 mice were subsequently transferred to Taconic Farms where they were either maintained on a 75% C57BL/6 x 25% 129S6/SvEv mixed genetic background or backcrossed six generations to C57BL/6. NMUR2 knockout (Nmur2-/-) mice were licensed from Deltagen Inc., San Mateo, CA and subsequently transferred to Taconic Farms where they were either maintained on a 75% C57BL/6 x 25% 129/O1aHsd mixed genetic background or backcrossed for seven generations to C57BL/6. NMUR1 and NMUR2 double knockout (Nmur1&2-/-) mice were generated by crossing N6 Nmur1-/- mice to N7 Nmur2-/- mice. Mice were individually housed in Tecniplast cages in a conventional SPF facility. Mice were initially maintained on a regular chow diet and then early in their life were switched to a high fat diet (D12492: 60 % kcal from fat; Research Diets, Inc., New Brunswick, NJ) with *ad libitum* access to water in a 12-hour light/12-hour dark cycle.

*Ad libitum* fed male diet-induced obese mice were weighed and dosed either i.p. or s.c. about 30 minutes prior to the onset of the dark phase of the light cycle and provided with a preweighed aliquot of high fat diet D12492 which was then weighed 2 hours and 18 hours (day . 1), 42 hours (day 2), 66 hours (day 3), and 90 hours (day 4) after the onset of the initial dark phase. Mice were weighed at the 18, 42, 66 and 90 hour time points. Data showed the outcome of the feeding study (all values are reported as mean ± SEM and data was analyzed using a two-tailed unpaired Student's t test; p values ≤ 0.05 were reported as significant and are denoted with an asterisk).

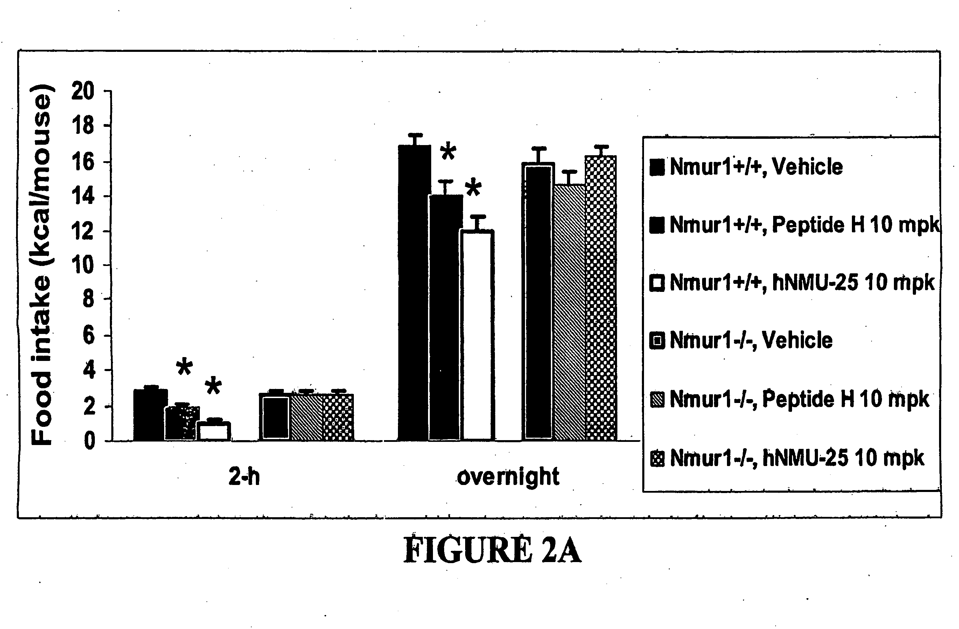

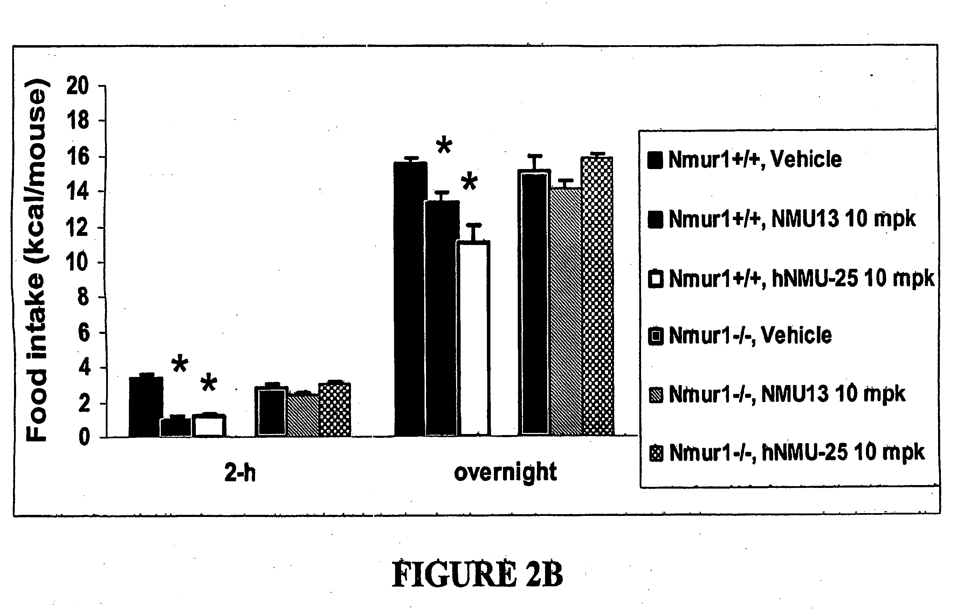

As shown in Figures 2A and 2B, acute peripheral administration of the NMUR1-selective peptides, neuromedin U receptor agonist H and NMU13, significantly reduced food intake in wild-type mice, but not in *Nmur1* knockout mice, demonstrating that NMUR1 is required for the anorectic actions of these analogs. Additionally, these data demonstrate that NMUR1-selective agonism is sufficient to recapitulate the anorectic actions of the pan NMU1/2 agonist NMU.

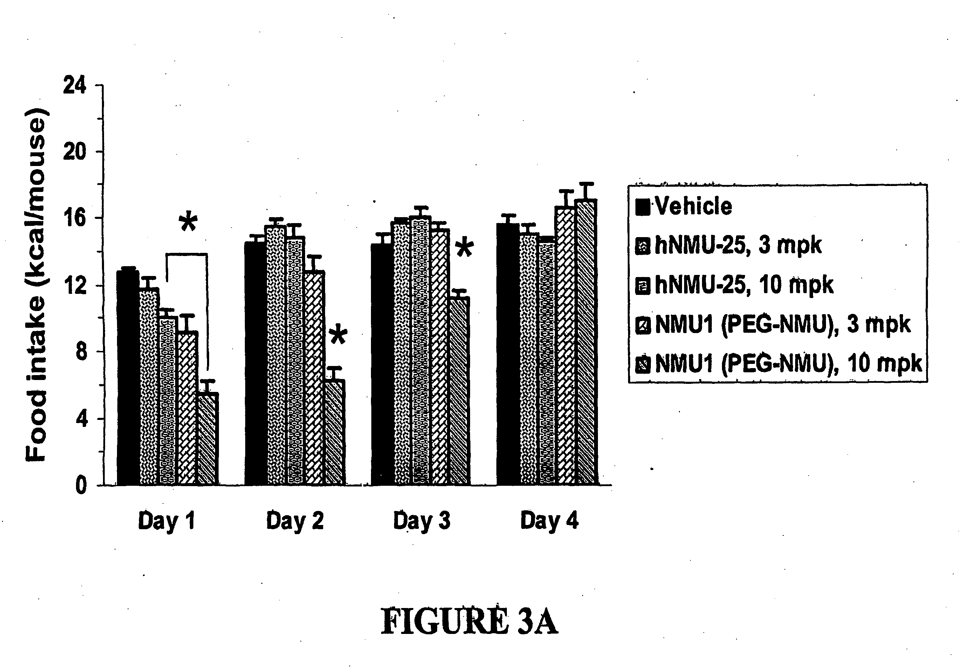

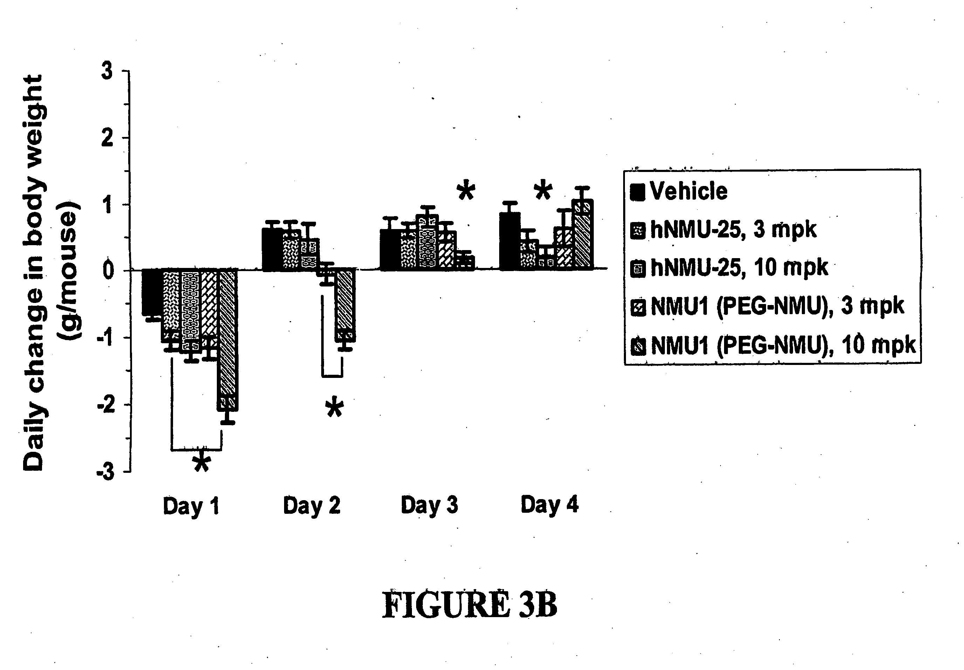

Figures 3A and 3B illustrate the finding that acute subcutaneous administration of PEGylated NMU reduces food intake for three days post-dose. Consistent with the *in vitro* and *in vivo* metabolic profile of the PEGylated analogs, NMU1 exhibits greater efficacy at reducing overnight food intake when compared to hNMU-25 and reductions in food intake are observed for three days post-dose. Significant reductions in body weight were also observed.

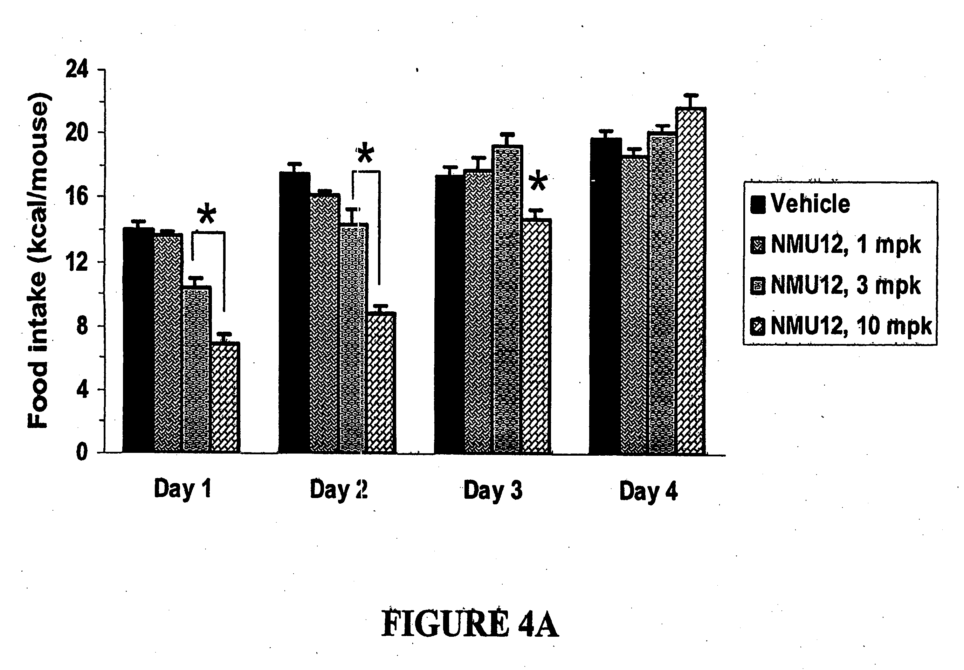

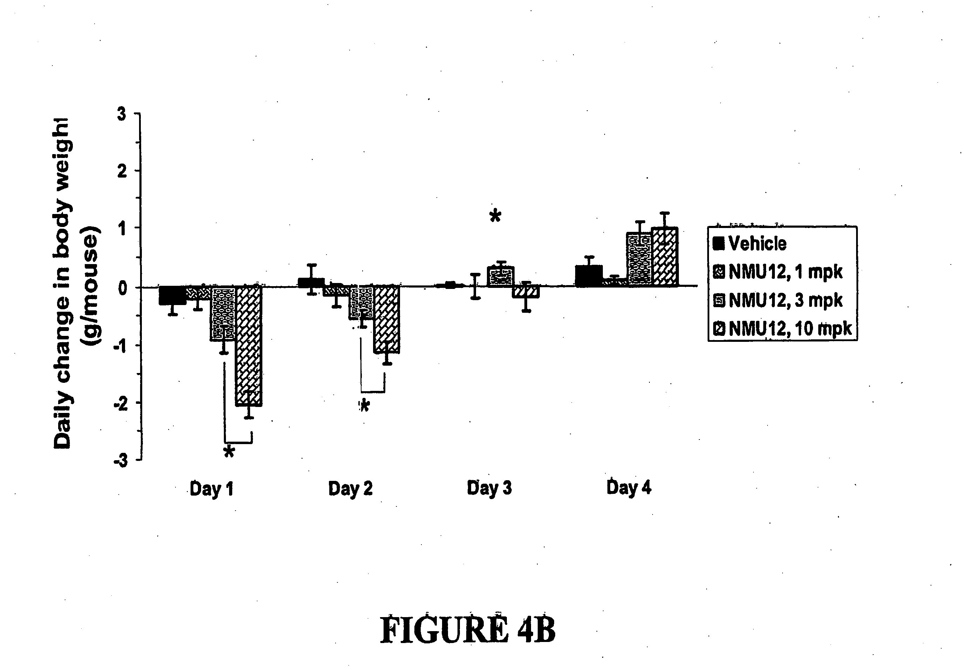

Figures 4A and 4B demonstrate that NMU12 is also an effective anorectic peptide. Similar to NMU1, a significant reduction in food intake and body weight are observed for three days following a single subcutaneous administration of peptide.

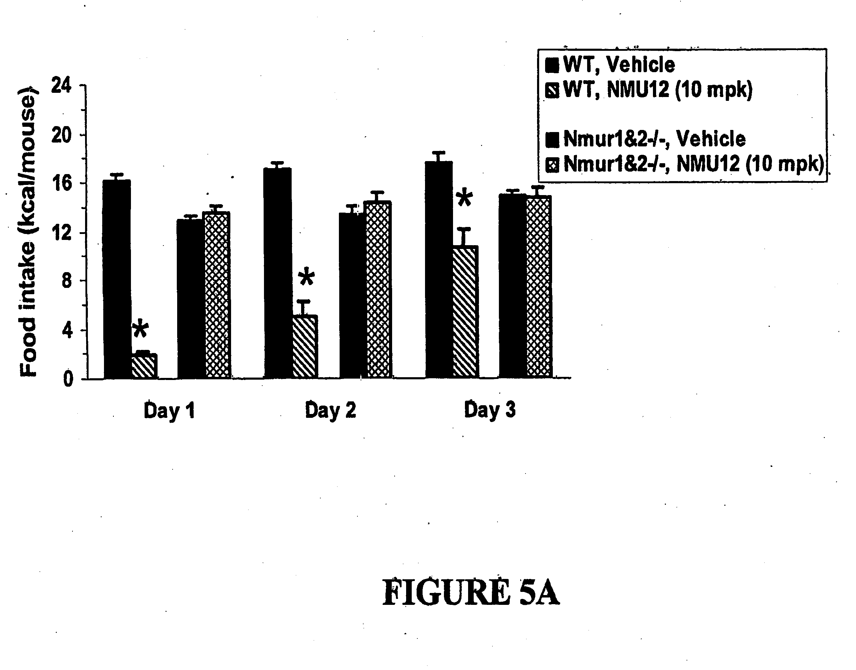

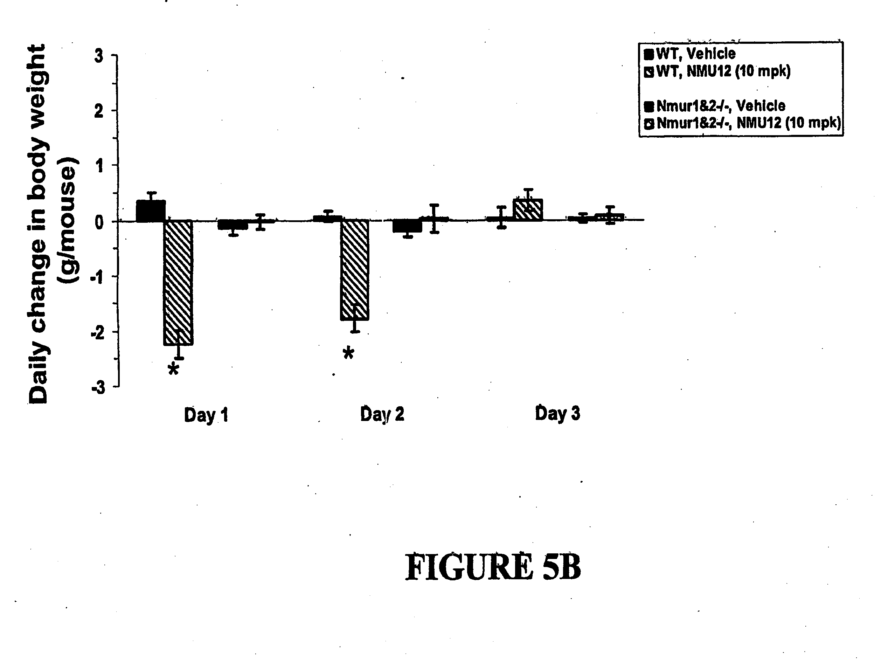

Further, Figures 5A and 5B illustrate that the anorectic effects of NMU12 are mediated by the NMUR1 and NMUR2 receptors. Acute administration of NMU12 was highly efficacious in wild-type animals but no effect was observed in the NMUR1/NMUR2 double knockout animals. Since the effect at the NMUR2 receptor occurs primarily in the brain, the results indicate that NMU12 is capable of crossing the blood-brain barrier. The effects of NMUR2 to reduce food intake and body weight require central exposure, whereas those of NMUR1 require peripheral exposure.

Figures 6A and 6B show that longer term anorectic effects of PEGylated NMU12 are mediated by both the NMUR1 and NMUR2 receptors. Reductions in food intake and body weight are observed for two days post dose in the NMU1 knockout animals. However, only overnight effects are observed in the NMUR2 knockout animals. This is in contrast to the anorectic effects of hNMU-25 which are mediated solely by NMUR1, demonstrating that hNMU-25 and NMU12 have distinct mechanisms of action.

Figures 7A-7C demonstrate that chronic administration of NMU12 can reduce food intake and body weight. NMU12 was dosed every day (QD), every other day (Q2D) or every three days (Q3D). Panel A shows the cumulative change in body weight for nine days after the beginning of treatment. The last dose was administered on day four of the study and measurements were taken to day nine. Cumulative food intake (B) and body weight (C) was significantly reduced in all dosing paradigms for NMU12. Food intake was reduced 12-27% at these doses relative to the vehicle treated group. Likewise, the cumulative change in body weight ranged from a loss of 3.3% to as much as a 7.3% relative to the vehicle control group.

A summary of percent reductions in food intake and body weight change from in vivo experiments with hNMU-25 and NMU analogs is shown in Table 10. Calculations are based on vehicle responses.

| Table 10 | | | | | | | |
|---|---|---|---|---|---|---|---|
| Agonist | Dose | % Food intake (FI) reductions | | | Body weight (BW) change (in grams) {relative to vehicle} | | |
| | | Day 1 | Day 2 | Day 3 | Day 1 | Day 2 | Day 3 |
| hNMU-25 | 10 mg/kg | 21 | | | -0.6 | | |
| NMU1 | 10mg/kg | 57 | 56 | 22 | -1.4 | -1.7 | -0.4 |
| NMU2 | 3mg/kg | 25 | | | -0.7 | | |
| NMU4 | 3 mg/kg | 12 | | | -0.3 | | |
| NMU5 | 3 mg/kg | 26 | | | -0.5 | | |
| NMU7 | 10 mg/kg | 44 | | | -1:7 | | |
| NMU9 | 10 mg/kg | 56 | 57 | 18 | -2.0 | -1.2 | -0.2 |
| NMU10 | 10 mg/kg | 24 | | | -1.2 | | |
| NMU11 | 10 mg/kg | 54 | 30 | | -1.8 | -0.7 | |
| NMU12 | 10 mg/kg | 51 | 50 | 15 | -1.8 | -1.3 | -0.2 |
| NMU14 | 30 mg/kg | | | | | | |
| | 50mg/kg | 14 | | | -0.1 | | |
| NMU16 | 10 mg/kg | 11 | | | -0.3 | | |
| | 30 mg/kg | 25 | | | -0.9 | | |
| NMU18 | 10 mg/kg | 71 | 40 | | -2.6 | -0.2 | |
| NMU20 | 10 mg/kg | 67 | 65 | 23 | -2.2 | -1.1 | +0.3 |

### EXAMPLE 6

### Plasma stability experiments

To determine stability of NMU12 and NMU1 in plasma from different species, in vitro spike-in experiments were performed. NMU12, NMU1, or hNMU-25 was added to plasma (purchased from Bioreclamation) at a final concentration of 1 µM and incubated at various temperatures (room temperature, 4°C and 37°C). Aliquots of these plasma samples were taken at various time points, frozen at -80°C, and subsequently run in the FLIPR assay with the cell line expression human NMUR1 to determine the percent of active peptide that remained after various incubation time points. The percent activity of peptide remaining in a sample was calculated based on the FLIPR response for the sample at the starting timepoint (T = 0) before any temperature challenge had been done with the peptide sample. At T = 0 the expected recovery is 100%.

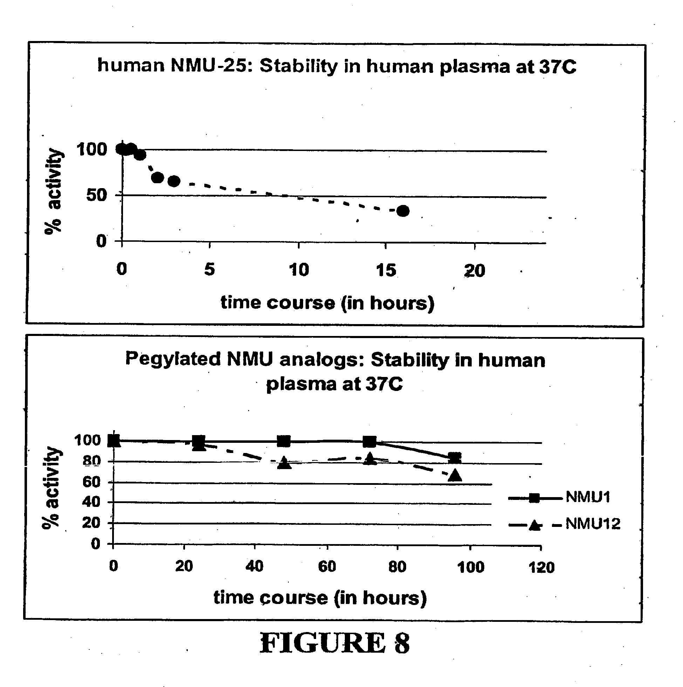

Figure 8 illustrates the *in vitro* stability of hNMU-25 and PEGylated neuromedin U receptor agonists NMU1 and NMU12 in human plasma with spike-in experiments, indicating that PEGylation of NMU provides greater stability in human plasma. The half-life of hNMU-25 in human plasma is less than 16 hours whereas the PEGylated analogs NMU1 (PEGylated hNMU-25) and NMU12 exhibited a half-life greater than three days in human plasma incubated at 37°C.

### EXAMPLE 7

### Pharmacokinetic analysis of peptides using the bioassay

Neuromedin U receptor agonist NMU12 exposure levels in dosed animals were measured using the bioassay and compared with hNMU-25 exposure levels. Animals were dosed subcutaneously with 10 mg/kg NMU12 or hNMU-25 and plasma was collected at various time points post-dose. The bioassay was a FLIPR-based assay, which was performed essentially as described above with the following modifications. Sample preparation prior to the assay was performed on ice to minimize degradation of plasma samples. The assay was run with 4% plasma as the final concentration. A three-point titration was done with the dosed plasma and tested on human NMUR1-expressing cell lines using FLIPR. The response after sample addition was taken as the maximum fluorescence units minus the fluorescence immediately prior to stimulation for each well. In addition to a three-point titration of dosed plasma samples, a 16-point titration of the peptide (hNMU-25 or NMU12) was run in 4% plasma (naive plasma) to serve as the standard. The concentration of the peptide in plasma was calculated based on extrapolations from the appropriate standard using the GraphPad Prism software.

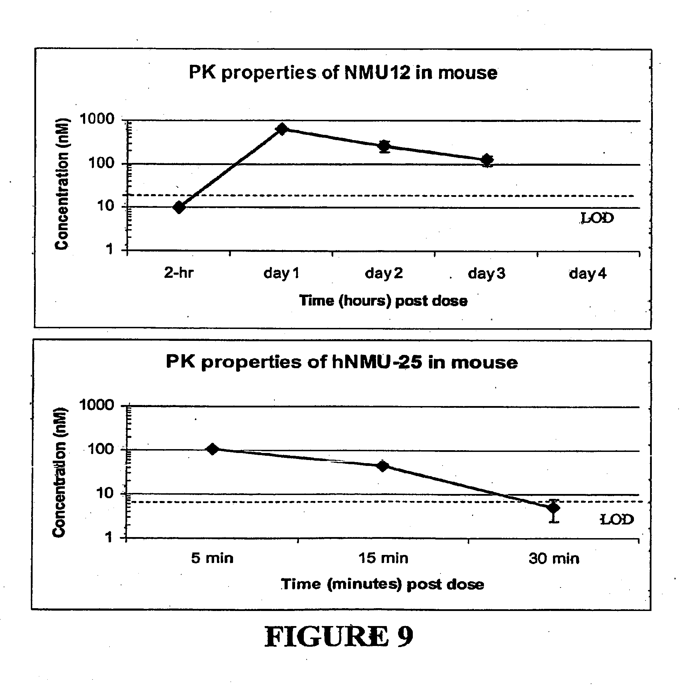

Figure 9 shows the pharmacokinetic properties of hNMU-25 and PEGylated NMU12 in mice, indicating that PEGylation of NMU provides greater metabolic stability *in vivo.* The dashed line indicates the limits of detection for the assay (LOD). These experiments demonstrate that PEGylation of NMU enhances both its *in vitro* and *in vivo* metabolic stability.

While the present invention is described herein with reference to illustrated embodiments, it should be understood that the invention is not limited hereto. Those having ordinary skill in the art and access to the teachings herein will recognize additional modifications and embodiments within the scope thereof. Therefore, the present invention is limited only by the claims attached herein.

## Claims

1. A neuromedin U receptor agonist, which has the formula
Z¹-peptide-Z²
wherein the peptide has the amino acid sequence set forth in SEQ ID NO:2 and further includes a cysteine residue at the N-terminus of the peptide in which the thiol group of the cysteine residue is covalently joined to a polyethylene glycol (PEG) molecule;
Z¹ is an optionally present protecting group that, if present, is joined to the N-terminal amino group of the cysteine residue;
and Z² is NH₂ or an optionally present protecting group that, if present, is joined to the C-terminal carboxy group,
or a pharmaceutically acceptable salt thereof.

2. The neuromedin U receptor agonist of claim 1, wherein a protecting group is joined to the C-terminal carboxy group.

3. The neuromedin U receptor agonist of claim 1, wherein a protecting group is joined to the N-terminal amino group of the cysteine residue.

4. The neuromedin U receptor agonist of claim 1, wherein the agonist has the formula Ac-C₂-peptide-CONH₂ wherein Ac is an acetyl group, C₂ is Cys(PEG)₂40kDa and the peptide has the amino acid sequence shown in SEQ ID NO:2.

5. A neuromedin U receptor agonist according to any previous claim for use in treating a metabolic disorder.

6. A pharmaceutical composition comprising the neuromedin U receptor agonist of any one of Claims 1-4 and a pharmaceutically acceptable carrier.

## Patentansprüche

1. Ein Neuromedin-U-Rezeptor-Agonist, der die Formel
Z¹-Peptid-Z²
besitzt,
wobei das Peptid die Aminosäuresequenz besitzt, die in SEQ ID NR.: 2 angegeben ist, und ferner einen Cysteinrest am N-Terminus des Peptids umfasst, wobei die Thiolgruppe des Cysteinrestes kovalent an ein Polyethylenglycol(PEG)-Molekül gebunden ist,
Z¹ eine gegebenenfalls vorhandene Schutzgruppe ist, die, falls vorhanden, an die N-terminale Aminogruppe des Cysteinrestes gebunden ist,
und Z² NH₂ oder eine gegebenenfalls vorhandene Schutzgruppe ist, die, falls vorhanden, an die C-terminale Carboxygruppe gebunden ist,
oder ein pharmazeutisch annehmbares Salz davon.

2. Der Neuromedin-U-Rezeptor-Agonist nach Anspruch 1, bei dem eine Schutzgruppe an die C-terminale Carboxygruppe gebunden ist.

3. Der Neuromedin-U-Rezeptor-Agonist nach Anspruch 1, bei dem eine Schutzgruppe an die N-terminale Aminogruppe des Cysteinrestes gebunden ist.

4. Der Neuromedin-U-Rezeptor-Agonist nach Anspruch 1, wobei der Agonist die Formel Ac-C₂-Peptid-CONH₂ besitzt, wobei Ac eine Acetylgruppe ist, C₂ Cys(PEG)₂40kDa ist und das Peptid die Aminosäuresequenz besitzt, die in SEQ ID NR.: 2 gezeigt ist.

5. Ein Neuromedin-U-Rezeptor-Agonist gemäß einem vorhergehenden Anspruch zur Verwendung bei der Behandlung einer Stoffwechselstörung.

6. Eine pharmazeutische Zusammensetzung, die den Neuromedin-U-Rezeptor-Agonist nach einem der Ansprüche 1-4 und einen pharmazeutisch annehmbaren Träger umfasst.

## Revendications

1. Agoniste du récepteur de neuromédine U, qui présente la formule:
Z¹-peptide-Z²
dans lequel le peptide possède la séquence d'acides aminés présentée dans la SEQ ID NO:2 et inclut en outre un résidu de cystéine à l'extrémité N du peptide dans lequel le groupe thiol du résidu de cystéine est joint par covalence à une molécule de polyéthylèneglycol (PEG);
Z¹ est un groupe protecteur éventuellement présent qui, s'il est présent, est joint au groupe amino de l'extrémité N du résidu de cystéine;
et Z² est NH₂ ou un groupe protecteur éventuellement présent qui, s'il est présent, est joint au groupe carboxy de l'extrémité C,
ou un sel pharmaceutiquement acceptable de celui-ci.

2. Agoniste du récepteur de neuromédine U selon la revendication 1, dans lequel un groupe protecteur est joint au groupe carboxy de l'extrémité C.

3. Agoniste du récepteur de neuromédine U selon la revendication 1, dans lequel un groupe protecteur est joint au groupe amino de l'extrémité N du résidu de cystéine.

4. Agoniste du récepteur de neuromédine U selon la revendication 1, où l'agoniste présente la formule Ac-C₂-peptide-CONH₂, où Ac est un groupe acétyle, C₂ est Cys(PEG)₂ 40 kDa et le peptide possède la séquence d'acides aminés montrée dans la SEQ ID NO:2.

5. Agoniste du récepteur de neuromédine U selon l'une quelconque des revendications précédentes pour une utilisation dans le traitement d'un trouble métabolique.

6. Composition pharmaceutique comprenant l'agoniste du récepteur de neuromédine U selon l'une quelconque des revendications 1-4 et un support pharmaceutiquement acceptable.


### SEQUENCE LISTING

<110> Merck & Co., Inc.
   Marsh, Donald J.
   Pessi, Antonello
   Bednarek, Maria A.
   Bianchi, Elisabetta
   Ingallinella, Paolo
   Peier, Andera M.
<120> NEUROMEDIN U RECEPTOR AGONISTS AND USES THEREOF
<130> PCT-21984Y
<150> 60/783,933
   <151> 2006-03-20
<160> 28
<170> FastSEQ for Windows version 4.0
<210> 1
   <211> 25
   <212> PRT
   <213> Artificial sequence
<220>
<223> NMU Agonist Peptide
   <223> Xaa Can be any amino acid or absent
<400> 1
   Xaa Xaa Xaa Xaa Xaa Xaa Xaa Xaa Xaa Xaa Xaa Xaa Xaa Xaa Xaa Xaa
    1               5                  10                  15
   Xaa Xaa Phe Leu Phe Arg Pro Arg Asn
               20                  25
<210> 2
   <211> 25
   <212> PRT
   <213> Artificial Sequence
<220>
   <223> NMU-25 Agonist
<400> 2
   Phe Arg Val Asp Glu Glu Phe Gln Ser Pro Phe Ala Ser Gln Ser Arg
    1               5                  10                  15
   Gly Tyr Phe Leu Phe Arg Pro Arg Asn
               20                  25
<210> 3
   <211> 14
   <212> PRT
   <213> Artificial Sequence
<220>
   <223> NMU (12-25) Agonist
<400> 3
   Ala Ser Gln Ser Arg Gly Tyr Phe Leu Phe Arg Pro Arg Asn
   1               5                   10
<210> 4
   <211> 9
   <212> PRT
   <213> Artificial sequence
<220>
   <223> NMU (17-25) Agonist
<400> 4
   Gly Tyr Phe Leu Phe Arg Pro Arg Asn
   1               5
<210> 5
   <211> 7
   <212> PRT
   <213> Artificial sequence
<220>
   <223> NMU (19-25) Agonist
<400> 5
   Phe Leu Phe Arg Pro Arg Asn
   1               5
<210> 6
   <211> 19
   <212> PRT
   <213> Artificial Sequence
<220>
   <223> NMU (7-25) Agonist
<400> 6
   Phe Gln Ser Pro Phe Ala Ser Gln Ser Arg Gly Tyr Phe Leu Phe Arg
   1               5                   10                  15
   Pro Arg Asn
<210> 7
   <211> 25
   <212> PRT
   <213> Artificial Sequence
<220>
   <223> NMU Agonist
<221> VARIANT
   <222> 18
   <223> Xaa is Y, W, F, a des-amino acid or an acyl group, or absent
<221> VARIANT
   <222> 19
   <223> Xaa is A, W, Y, F, or an aliphatic amino acid
<221> VARIANT
   <222> 20
   <223> Xaa is G, sarcosine (Sar), D-leu, NMe-Leu, D-Ala, A, or absent
<221> VARIANT
   <222> 21
   <223> Xaa is NMe-Phe, an aliphatic amino acid, an aromatic amino acid, A, or W
<221> VARIANT
   <222> 22
   <223> Xaa is K, A, or L
<221> VARIANT
   <222> (23)...(23)
   <223> Xaa is Sar, A, or L
<221> VARIANT
   <222> (24)...(24)
   <223> Xaa is Harg or K
<221> VARIANT
   <222> (25)...(25)
   <223> Xaa is any D- or L-amino acid, Nle or D-Nle, or A
<400> 7
   Phe Arg Val Asp Glu Glu Phe Gln Ser Pro Phe Ala Ser Gln Ser Arg
    1               5                  10                  15
   Gly Xaa Xaa Xaa Xaa Xaa Xaa Xaa Xaa
               20                  25
<210> 8
   <211> 8
   <212> PRT
   <213> Artificial Sequence
<220>
   <223> NMU Agonist
<221> VARIANT
   <222> 1
   <223> Xaa is A, W, Y, F, an aliphatic amino acid, or absent
<221> VARIANT
   <222> 2
   <223> Xaa is A, W, Y, F, or an aliphatic amino acid
<221> VARIANT
   <222> 3
   <223> Xaa is L, F, G, Sar, D-Leu, NMe-Leu, D-Ala, A, or absent
<221> VARIANT
   <222> 4
   <223> Xaa is NMe-Phe, an aliphatic amino acid, an aromatic amino acid, A, or W
<221> VARIANT
   <222> 5
   <223> Xaa is R, F, K, A, or L
<221> VARIANT
   <222> (6)...(6)
   <223> Xaa is P, Sar, A, or L
<221> VARIANT
   <222> (7)...(7)
   <223> Xaa is R, Harg, or K
<221> VARIANT
   <222> (8)...(8)
   <223> Xaa is any D- or L-amino acid, Nle or D-Nle, or A
<400> 8
   Xaa Xaa Xaa Xaa Xaa Xaa Xaa Xaa
    1               5
<210> 9
   <211> 7
   <212> PRT
   <213> Artificial sequence
<220>
   <223> NMU Agonist
   <400> 9
   Tyr Phe Trp Arg Phe Arg Asn
    1               5
<210> 10
   <211> 8
   <212> PRT
   <213> Artificial Sequence
<220>
   <223> NMU Agonist
<221> VARIANT
   <222> 3
   <223> Xaa is D-Leu
<400> 10
   Tyr Phe Xaa Trp Arg Pro Arg Asn
   1 5
<210> 11
   <211> 8
   <212> PRT
   <213> Artificial Sequence
<220>
   <223> NMU Agonist
<400> 11
   Tyr Phe Gly Trp Arg Pro Arg Asn
   1 5
<210> 12
   <211> 8
   <212> PRT
   <213> Artificial sequence
<220>
   <223> NMU Agonist
<221> VARIANT
   <222> (0)...(0)
   <223> Xaa is D-Ala
<400> 12
   Tyr Phe Xaa Trp Arg Pro Arg Asn
   1 5
<210> 13
   <211> 7
   <212> PRT
   <213> Artificial Sequence
<220>
   <223> NMU Agonist
   <400> 13
   Phe Phe Trp Arg Pro Arg Asn
   1 5
<210> 14
   <211> 24
   <212> PRT
   <213> Artificial sequence
<220>
   <223> NMU Agonist
<400> 14
   Phe Arg Val Asp Glu Glu Phe Gln Ser Pro Phe Ala Ser Gln Ser Arg
   1 5 10 15
   Gly Tyr Phe Trp Arg Pro Arg Asn
   20
<210> 15
   <211> 24
   <212> PRT
   <213> Artificial Sequence
<220>
   <223> NMU Agonist
<221> VARIANT
   <222> 20
   <223> Xaa is D-Leu
<400> 15
   Phe Arg Val Asp Glu Glu Phe Gln Ser Pro Phe Ala Ser Gln Ser Arg
   1 5 10 15
   Gly Tyr Phe Xaa Leu Trp Arg Pro
   20
<210> 16
   <211> 8
   <212> PRT
   <213> Artificial Sequence
<220>
   <223> NMU Agonist
<221> VARIANT
   <222> 7
   <223> Xaa is Harg
<400> 16
   Phe Trp Leu Phe Arg Pro Xaa Asn
   1 5
<210> 17
   <211> 8
   <212> PRT
   <213> Artificial Sequence
<220>
   <223> NMU Agonist
<221> VARIANT
   <222> 7
   <223> Xaa is Harg
<400> 17
   Phe Trp Leu Phe Arg Ala Xaa Asn
   1 5
<210> 18
   <211> 8
   <212> PRT
   <213> Artificial sequence
<220>
   <223> NMU Agonist
<221> VARIANT
   <222> 8
   <223> Xaa is D-Nle
<400> 18
   Trp Phe Leu Phe Arg Pro Arg Xaa
   1 5
<210> 19
   <211> 8
   <212> PRT
   <213> Artificial Sequence
<220>
   <223> NMU Agonist
<400> 19
   Phe Trp Leu Phe Arg Ala Arg Asn
   1 5
<210> 20
   <211> 8
   <212> PRT
   <213> Artificial sequence
<220>
   <223> NMU Agonist
<400> 20
   Tyr Ala Leu Phe Arg Ala Arg Asn
   1 5
<210> 21
   <211> 8
   <212> PRT
   <213> Artificial sequence
<220>
   <223> NMU Agonist
<400> 21
   Phe Ala Leu Phe Arg Pro Arg Asn
   1 5
<210> 22
   <211> 24
   <212> PRT
   <213> Artificial sequence
<220>
   <223> NMU Agonist
<221> VARIANT
   <222> 24
   <223> Xaa is Harg
<400> 22
   Phe Arg Val Asp Glu Glu Phe Gln Pro Phe Ala Ser Gln Ser Arg Gly
   1 5 10 15
   Phe Trp Leu Phe Arg Pro Xaa Asn
   20
<210> 23
   <211> 25
   <212> PRT
   <213> Artificial sequence
<220>
   <223> NMU Agonist
<221> VARIANT
   <222> 24
   <223> Xaa is Harg
<400> 23
   Phe Arg Val Asp Glu Glu Phe Gln Ser Pro Phe Ala Ser Gln Ser Arg
   1 5 10 15
   Gly Phe Trp Leu Phe Arg Ala Xaa Asn
   20 25
<210> 24
   <211> 25
   <212> PRT
   <213> Artificial sequence
<220>
   <223> NMU Agonist
<221> VARIANT
   <222> 25
   <223> Xaa is D-Nle
<400> 24
   Phe Arg Val Asp Glu Glu Phe Gln Ser Pro Phe Ala Ser Gln Ser Arg
   1               5                   10                  15
   Gly Phe Trp Leu Phe Arg Pro Arg Xaa
               20                  25
<210> 25
   <211> 25
   <212> PRT
   <213> Artificial Sequence
<220>
   <223> NMU Agonist
<221> VARIANT
   <222> 20
   <223> Xaa is D-Ala
<400> 25
   Phe Arg Val Asp Glu Glu Phe Gln Ser Pro Phe Ala Ser Gln Ser Arg
   1               5                   10                  15
   Gly Tyr Phe Xaa Trp Arg Pro Arg Asn
               20                  25
<210> 26
   <211> 7
   <212> PRT
   <213> Artificial Sequence
<220>
   <223> NMU Agonist
<221> VARIANT
   <222> 2
   <223> Xaa is D-Leu
<400> 26
   Phe Xaa Trp Arg Pro Arg Asn
   1               5
<210> 27
   <211> 25
   <212> PRT
   <213> Artificial Sequence
<220>
   <223> NMU Agonist
<221> VARIANT
   <222> (1)...(17)
   <223> Xaa is any amino acid or absent
<221> VARIANT
   <222> 18
   <223> Xaa is Y, W, F, a des-amino acid or an acyl
<221> VARIANT
   <222> 19
   <223> Xaa is F, A, W, or an aliphatic amino acid
<221> VARIANT
   <222> 20
   <223> Xaa is L, G Sar, D-Leu, NMe-Leu, D-Ala, D, or absent
<221> VARIANT
   <222> 21
   <223> Xaa is F, NMe-Phe, an aliphatic amino acid, an aromatic amino acid, A, or W
<221> VARIANT
   <222> (22)...(22)
   <223> Xaa is R, K, A, or L
<221> VARIANT
   <222> (23)...(23)
   <223> Xaa is P, Sar, A, or L
<221> VARIANT
   <222> (24)...(24)
   <223> Xaa is R, Harg, or K
<221> VARIANT
   <222> (25)...(25) D- or L-amino acid, Nle or Dle, or A
   <223> Xaa is N, any D- or L-amino acid, Nle or Dle, or A
<400> 27
   Xaa Xaa Xaa Xaa Xaa Xaa Xaa Xaa Xaa Xaa Xaa Xaa Xaa Xaa Xaa Xaa
    1               5                   10                  15
   Xaa Xaa Xaa Xaa Xaa Xaa Xaa Xaa Xaa
               20                  25
<210> 28
   <211> 25
   <212> PRT
   <213> Artificial Sequence
<220>
   <223> NMU agonist
<400> 28
   Phe Arg Val Asp Glu Glu Phe Gln Ser Pro Phe Ala Ser Gln Ser Arg
    1               5                   10                  15
   Pro Tyr Phe Leu Phe Arg Pro Arg Asn
               20                  25